# EUROPEAN PATENT APPLICATION

(11) **EP 0 319 316 A2**
(43) Date of publication of application: **07.06.1989**
(21) Application number: 88311441.5
(22) Date of filing: 02.12.1988
(51) Int. Cl.: C07H 19/16, A61K 31/70

(54) **Griseolic acid monoesters, their preparation and use**

(30) Priority: 02.12.1987 JP 306200/87; 30.06.1988 JP 162882/88
(71) Applicant: SANKYO COMPANY LIMITED, Tokyo (JP)
(72) Inventor: Kaneko, Masakatsu, Shinagawa-ku Tokyo 140 (JP); Kimura, Misako, Shinagawa-ku Tokyo 140 (JP); Kamokari, Makoto, Shinagawa-ku Tokyo 140 (JP); Yokoyama, Tomihisa, Shinagawa-ku Tokyo 140 (JP); Yamazaki, Mitsuo, Shinagawa-ku Tokyo 140 (JP); Hirai, Koichi, Shinagawa-ku Tokyo 140 (JP); Sato, Susumu, Shinagawa-ku Tokyo 140 (JP); Yasumoto, Takashi, Shinagawa-ku Tokyo 140 (JP)
(74) Representative: Gibson, Christian John Robert

(57) **Abstract**

Monoester griseolic acid derivatives of formula (I): 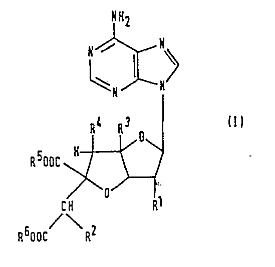 (in which: R¹ and R² are the same or different and each represents a hydrogen atom, a hydroxy group or a protected hydroxy group; R³ and R⁴ each represents a hydrogen atom, or together represent an extra carbon-carbon bond between the two carbon atoms to which they are attached; one of R^{S} and R⁶ represents a hydrogen atom and the other represents a carboxy-protecting group removable in the biochemical environment of the human eye); and pharmaceutically acceptable salts thereof have been found to have valuable properties in the treatment of ophthalmic disorders. They may be prepared by the selective esterification of the appropriate free acid.

## Description

The present invention relates to a series of novel griseolic acid monoesters, which have been surprisingly found to have the valuable ability to lower intraocular pressure.

Griseolic acid is a nucleoside-type compound having an adenine base and two carboxylic acid groups. It and salts thereof were first disclosed in, inter alia, European Patent Specification No. 29 329A, but its structure was not, at that stage, known. Its structure was first disclosed in U.S. Patent Specification No. 4 460 765. In both of the above-mentioned specifications, griseolic acid and its salts were said to inhibit the activity of cyclic adenosine 3',5'-monophosphate (cAMP) phosphodiesterase (PDE) and thus have a variety of potential activities, for example as an angiocardiokinetic agent, an antiasthmatic agent, a smooth muscle relaxant, an antiinflammatory agent, a treatment for diabetes and as a treatment for various kinds of psoriasis.

The structure of griseolic acid may be represented by the formula (A):

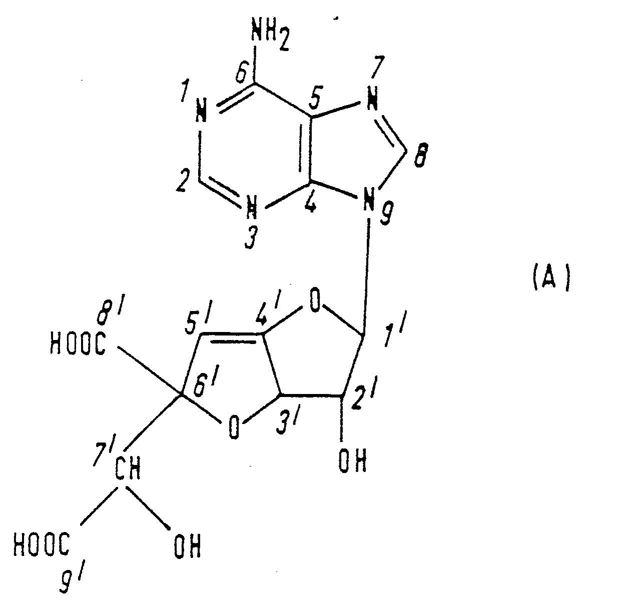

It should be noted that natural griseolic acid, being a product of natural biosynthesis, is obtained stereospecifically. In fact, at both the 2' and the 7' positions, it is in the R-configuration. The numbering system shown on the above formula (A) is that used herein for the nomenclature of griseolic acid derivatives.

Subsequently, a variety of griseolic acid derivatives were discovered, and these are disclosed, inter alia, as follows:

European Patent Publication No. 143 557A discloses a number of derivatives in which there are substituents at one or more of the 1, 6, 8, 2', 4', 5' and 7' positions and in which the double bond at the 4'-5' position is optionally hydrogenated. This discloses in general terms mono- and di- esters of the compounds the subject of that specification, but in fact only specifically discloses the preparation and use of the diesters, which are said to be easiest to prepare.

Japanese Patent Application Kokai (i.e. as laid open to public inspection) No. 60-149394 discloses 7'-deoxygriseolic acid derivatives and does not specifically discuss the esters.

European Patent Publication No. 162 715A discloses a compound which, in that specification, is referred to as "dihydrodesoxygriseolic acid", but which here, for consistency with other names, is referred to as "7'-deoxy-4',5'-dihydrogriseolic acid". Salts and esters of this compound are also disclosed, but there is no specific disclosure of the mono esters.

European Patent Publication No. 200 522A discloses a process for preparing certain griseolic acid derivatives, and does not specifically discuss the esters.

European Patent Publication No. 214 708A discloses a series of griseolic acid and 7'-deoxy-4',5'-dihydro- griseolic acid derivatives in which primarily the adenine base portion of the principal compound has been modified. Again, esters are disclosed and it is clear that diesters are preferred.

We have now discovered a series of new compounds which are the monoesters of certain of the known griseolic acid derivatives and which surprisingly have a potentially valuable ophthalmic activity, which has not previously been reported in compounds of this class. Although we have found that grieolic acid itself and certain diesters (but not all diesters) have this type of activity, griseolic acid is unable to cross the ophthalmic membrane and cannot therefore be administered by instillation. If it were to be administered, it would have to be by injection, which is clearly undesirable for this form of treatment. The compounds of the present invention can be administered by instillation, have effectively no toxicity and are very effective in the treatment of ophthalmic disorders. Those diesters which have this activity have, unlike the monoesters, limited solubility in the type of media used for the administration of ophthalmic preparations.

The compounds of the present invention are those compounds having the formula (I): 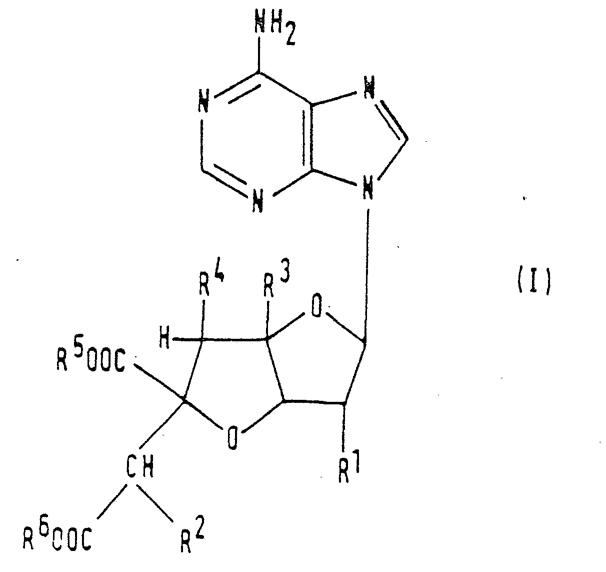 in which:
R¹ and R² are the same or different and each represents a hydrogen atom, a hydroxy group or a protected hydroxy group;
R³ and R⁴ each represents a hydrogen atom, or together represent an extra carbon-carbon bond between the two carbon atoms to which they are attached (i.e., together with the bond shown, they form a double bond); one of R⁵ and R⁶ represents a hydrogen atom and the other represents a carboxy-protecting group removable in the biochemical environment of the human eye;
and pharmaceutically acceptable salts thereof.

The invention also provides a pharmaceutical composition for application to the eyes, and comprising at least one ophthalmically active compound in admixture with an ophthalmically acceptable carrier or diluent, wherein said active compound is at least one compound of formula (I), defined above, or a pharmaceutically acceptable salt thereof.

The invention still further provides the use for the manufacture of a medicament for the treatment of optical disorders, including inflammation and glaucoma, in an animal, especially mammal, e.g. human, of at least one ophthalmically active compound of formula (I), defined above, or a pharmaceutically acceptable salt thereof. The administration is preferably by instillation.

In the accompanying drawings:
Figure 1 shows the change of intraocular pressure with time for a compound of the invention and a prior art compound in Experiment 2 hereafter; and
Figure 2 shows the change of intraocular pressure with time for a compound of the invention and a prior art compound in Experiment 3 hereafter.

In the compounds of the present invention, R and R² may be the same or different and each represents a hydrogen atom, a hydroxy group or a protected hydroxy group. Where the compounds of the invention are to be used merely as intermediates in the preparation of other, and perhaps more active, compounds, the nature of the protecting group employed will not be critical, and any hydroxy protecting group appropriate to the particular preparation step(s) may be employed. However, where the compound is itself intended for therapeutic use, the protecting group should be pharmaceutically acceptable. Preferably, the protecting group is such that it can be hydrolyzed and thus removed in the animal body, especially in the eye.

Examples of such protecting groups include:
aliphatic acyl groups, such as: the alkanoyl groups, particularly Ci - C₂₀, more preferably Ci - C₁₈, alkanoyl groups, e.g. the formyl, acetyl, propionyl, butyryl, isobutyryl, pentanoyl, pivaloyl, valeryl, isovaleryl, octanoyl, lauroyl, myristoyl, tridecanoyl, palmitoyl and stearoyl groups; halogenated alkanoyl groups, especially C₂ - C₆, more preferably C₂ - C₄, haloalkanoyl groups (in which there may be one or more halogen atoms, e.g. from 1 to 3 halogen atoms), and most preferably haloacetyl groups, e.g. the chloroacetyl, dichloroacetyl, trichloroacetyl and trifluoroacetyl groups; lower alkoxyalkanoyl groups in which the alkoxy part is preferably Ci - C₄ and the alkanoyl part is preferably C₂ - C₇, more preferably C₂ - Cs, e.g. the methoxyacetyl group; and alkenoyl or alkynoyl groups, especially C₃ - C₁₈, preferably C₃ - C₇, and more preferably Cs - Cs, alkenoyl and alkynoyl groups, e.g. the acryloyl, propioloyl, methacryloyl, crotonoyl, isocrotonoyl, oleoyl, elaidoyl, maleoyl, fumaroyl, citraconoyl, mesaconoyl and (E)-2-methyl-2-butenoyl groups;
aromatic acyl groups, such as arylcarbonyl groups, in which the aryl part is preferably a phenyl or naphthyl group which is unsubstituted or has at least one of substituents (a), as defined hereafter, for example: unsubstituted groups, such as the benzoyl, a-naphthoyl and β-naphthoyl groups; halogen-substituted arylcarbonyl groups, such as the 2-bromobenzoyl and 4-chlorobenzoyl groups; lower alkyl-substituted arylcarbonyl groups, such as the 2,4,6-trimethylbenzoyl and 4-toluoyl groups; lower alkoxy-substituted arylcarbonyl groups, such as the 4-anisoyl group; nitrated arylcarbonyl groups, such as the 4-nitrobenzoyl and 2-nitrobenzoyl groups; lower alkoxycarbonylated arylcarbonyl groups, in which the alkoxy group is preferably C₁-C₆, more preferably C₁-C₄, such as the 2-(methoxycarbonyl)benzoyl group; and aryl-substituted arylcarbonyl groups, such as the 4-phenylbenzoyl group;
tetrahydropyranyl or tetrahydrothiopyranyl groups, which may be unsubstituted or have at least one of substituents (b), defined below, for example the tetrahydropyran-2-yl, 3-bromotetrahydropyran-2-yl, 4-methoxytetrahydropyran-4-yl, tetrahydrothiopyran-2-yl and 4-methoxytetrahydrothiopyran-4-yl groups; tetrahydrofuranyl or tetrahydrothienyl groups, which may be unsubstituted or have at least one of substituents (b), defined below, for example the tetrahydrofuran-2-yl and tetrahydrothien-2-yl groups, which may be substituted or unsubstituted;
silyl groups, such as: the tri-lower-alkylsilyl groups in which each alkyl part is the same or different and each represents a Ci - Cs, preferably C₁ - C₄, alkyl group, e.g. the trimethylsilyl, triethylsilyl, isopropyldimethylsilyl, t-butyldimethylsilyl, methyldiisopropylsilyl, methyl-di-t-butylsilyl and triisopropylsilyl groups, and tri-lower-alkylsilyl groups in which 1 or 2 of the alkyl groups have been replaced by an aryl group (preferably a phenyl or naphthyl group, more preferably phenyl, which may be unsubstituted or have at least one of substituents (a), defined below) e.g. the diphenylmethylsilyl, diphenylbutylsilyl, diphenylisopropylsilyl and phenyldiisopropylsilyl groups;
alkoxymethyl groups, in which the alkoxy part is preferably C₁ - C₆, more preferably C₁ - C₄, and in which the alkoxy part is unsubstituted or has at least one of substituents (c), defined below, for example: the lower unsubstituted alkoxymethyl groups, such as the methoxymethyl, 1,1-dimethyl-1-methoxymethyl, ethoxymethyl, propoxymethyl, isopropoxymethyl, butoxymethyl and t-butoxymethyl groups; lower alkoxy-substituted lower alkoxymethyl groups, such as the 2-methoxyethoxymethyl group; and halogenated lower alkoxymethyl groups, such as the 2,2,2-trichloroethoxymethyl and bis(2-chloroethoxy)methyl groups;
substituted ethyl groups such as: the lower (preferably C₁ - C₆, more preferably Ci - C₄) alkoxylated ethyl groups, such as the 1-ethoxyethyl, 1-methyl-1-methoxyethyl and 1-isopropoxyethyl groups; halogen-substituted ethyl groups, such as the 2,2,2-trichloroethyl group; and arylselenylated ethyl groups, such as the 2-(phenylselenyl)ethyl group;
aralkyl groups, particularly those in which a lower alkyl group (preferably C₁ - Cs, more preferably Ci - C₄, most preferably C₁ - C₃) is substituted with from 1 to 3 Cs - C₁₄ aryl groups (preferably a phenyl, naphthyl or anthryl group which may be unsubstituted or have at least one of substituents (a), defined below), for example: unsubstituted groups, e.g. the benzyl, phenethyl, 3-phenylpropyl, a-naphthylmethyl, β-naphthylmethyl, diphenylmethyl, triphenylmethyl, a-naphthyldiphenylmethyl and 9-anthrylmethyl groups; lower (preferably Ci - C₆, more preferably C₁ - C₄) alkyl-substituted groups, e.g. the 4-methylbenzyl, 2,4,6-trimethylbenzyl and 3,4,5-trimethylbenzyl groups; lower (preferably C₁ - C₆, more preferably Ci - C₄) alkoxy-substituted groups, e.g. the 4-methoxybenzyl and (4-methoxyphenyl)diphenylmethyl groups; nitro-substituted groups, e.g. the 2-nitrobenzyl, 4-nitrobenzyl and bis(2-nitrophenyl)methyl groups; halogen-substituted groups, e.g. the 4-chlorobenzyl and 4-bromobenzyl groups; cyano-substituted groups, e.g. the 4-cyanobenzyl and 4-cyanobenzyldiphenylmethyl groups; and Ci - C3 alkylenedioxy-substituted groups, such as the piperonyl group;
alkoxycarbonyl groups, such as the unsubstituted lower alkoxycarbonyl groups (in which the alkoxy group is preferably Ci - C₆, more preferably C₁ - C₄), e.g. the methoxycarbonyl, ethoxycarbonyl, t-butoxycarbonyl and isobutoxycarbonyl groups; and such lower alkoxycarbonyl groups which are substituted with one or more halogen atoms, tri-lower alkylsilyl groups, arylalkylsilyl groups or alkanoyloxy groups, such as the 2,2.2-trichloroethoxycarbonyl, 2-trimethylsilylethoxycarbonyl and pivaloyloxymethoxycarbonyl groups; alkenyloxycarbonyl groups, in which the alkenyl part is preferably C₂ - C₇, more preferably C₂ - Cs, such as the vinyloxycarbonyl and allyloxycarbonyl groups; and
aralkyloxycarbonyl groups, in which the aralkyl part is preferably as defined above, and more preferably the aryl ring is unsubstituted or is substituted with 1 or 2 of substituents (a), defined below, preferably lower alkoxy or nitro groups, such as the benzyloxycarbonyl, 4-methoxybenzyloxycarbonyl, 3,4-dimethoxybenzyloxycarbonyl, 2-nitrobenzyloxycarbonyl and 4-nitrobenzyloxycarbonyl groups.

Of these protecting groups, we particularly prefer those which are readily hydrolized in a living body, i.e. which make the compound into a prodrug to be administered to the living body; examples of these include the pivaloyloxymethoxycarbonyl group and the aliphatic acyl groups and aromatic acyl groups exemplified above.

In the above examples, substituents (a) are: lower (preferably Ci - Cs, more preferably Ci - C4) alkyl groups, e.g. the methyl, ethyl, propyl, isopropyl, butyl, isobutyl, sec-butyl and t-butyl groups; lower (preferably C₁ - C₆, more preferably Ci - C₄) alkoxy groups, e.g. the methoxy, ethoxy, propoxy, isopropoxy, butoxy, isobutoxy, sec-butoxy, t-butoxy, pentyloxy, isopentyloxy, 2-methylbutoxy, neopentyloxy, hexyloxy, 4-methylpentyloxy, 3-methylpentyloxy, 2-methylpentyloxy, 3,3-dimethylbutoxy, 2,2-dimethylbutoxy, 1,1-dimethylbutoxy, 1,2-dimethyibutoxy, 1,3-dimethylbutoxy and 2,3-dimethylbutoxy groups; the nitro group; halogen atoms, such as the fluorine, chlorine, bromine and iodine atoms; the cyano group; aryl groups, e.g. as exemplified above, provided that, where any such aryl group is a substituent on an aryl group, it is not itself further substituted by another aryl group; C₁ - Cs, preferably C₁ or C₂ alkylenedioxy groups, e.g. the methylenedioxy group; the hydroxy group; the amino group; mono- and di- alkylamino groups, in which the or each alkyl group is preferably Cᵢ - C₆, more preferably Cᵢ - C₄- and examples are as given for the alkyl substituents above; the carbamoyl group; mono- and di- alkylcarbamoyl groups, in which the or each alkyl group is preferably C₁ - Cₛ, more preferably Ci - C₄, and examples are as given for the alkyl substituents above; C₁ - C₁₈ aliphatic acyl groups, such as those defined above as protecting groups; halogenated lower (e.g. C₁- C₆- especially C₁ - C₄) alkyl groups, such as the trifluoromethyl, trichloromethyl, difluoromethyl, dichloromethyl, dibromomethyl, fluoromethyl, 2,2,2-trichloroethyl, 2,2,2-trifluoroethyl, 2-bromoethyl, 2-chloroethyl, 2-fluoroethyl and 2,2-dibromoethyl groups; the carboxy group; and esterified derivatives of the carboxy group, e.g. alkoxycarbonyl groups, in which the alkoxy group is preferably C₁ - Cs more preferably Ci- C₄ (such as the methoxycarbonyl, ethoxycarbonyl, propoxycarbonyl, isopropoxycarbonyl, butoxycarbonyl, isobutoxycarbonyl, sec-butoxycarbonyl or t-butoxycarbonyl groups), halogenated alkoxycarbonyl groups in which the halogenated alkyl part is as defined above (such as the trifluoromethoxycarbonyl group) or aralkyloxycarbonyl groups, in which the aryl part is preferably as defined above, e.g. the benzyloxycarbonyl group and substituted derivatives thereof.

Substituents (b), which are optional substituents on heterocyclic groups are as exemplified above for substituents (a), but additionally include the oxygen atom, to form an oxo group.

Substituents (c) include those groups and atoms exemplified above for substituents (a), other than alkyl groups and substituted alkyl groups.

The nature of the groups represented by R⁵ and R⁶ in the compounds of the present invention is important to the invention. One of these must be a hydrogen atom, and the other an ester group. If both are ester groups, to form a diester, then the resulting compound may, depending on the nature of the ester groups, have ophthalmic activity, but the resulting compounds are not soluble in pharmacologically acceptable buffer solutions, and cannot, therefore be applied in practice to the eyes. On the other hand, if neither is an ester group, the resulting griseolic acid or derivative thereof may also have the desired ophthalmic activity, but will not be able to transfer across the ophthalmic membrane and will therefore be equally ineffective.

Accordingly, it is important that one of R⁵ and R⁶ should be a hydrogen atom and the other should be an ester group. The nature of the ester group is not critical to the invention, provided that it is capable of being removed by the biochemical environment of the mammalian eye and that the group does not result in any adverse effect on the patient. Examples of such groups are well known to those skilled in the art. For example, such groups include: those groups of formula (II): 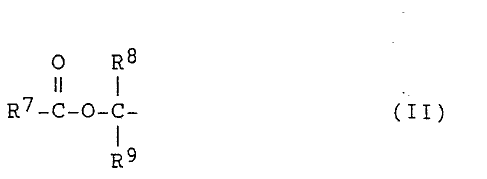 in which:
R⁷ represents a straight or branched chain alkyl group having from 1 to 10 carbon atoms, an aryl group having from 6 to 14 ring carbon atoms or a cycloalkyl group having from 3 to 10 carbon atoms, the aryl group being unsubstituted or having at least one of substituents (a);
R⁸ represents a hydrogen atom, a straight or branched chain alkyl group having from 1 to 10 carbon atoms or a cycloalkyl group having from 3 to 10 carbon atoms; and
R⁹ represents a hydrogen atom or a straight or branched chain alkyl group having from 1 to 10 carbon atoms; those groups of formula (111): 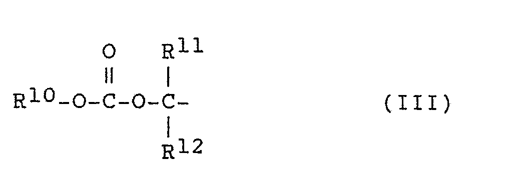 in which:
R¹⁰ represents a straight or branched chain alkyl group having from 1 to 10 carbon atoms, an aryl group having from 6 to 14 ring carbon atoms or a cycloalkyl group having from 3 to 10 carbon atoms, the aryl group being unsubstituted or having at least one of substituents (a);
R¹¹ represents a hydrogen atom, a straight or branched chain alkyl group having from 1 to 10 carbon atoms or a cycloalkyl group having from 3 to 10 carbon atoms; and
R¹² represents a hydrogen atom or a straight or branched chain alkyl group having from 1 to 10 carbon atoms; those groups of formula (IV): 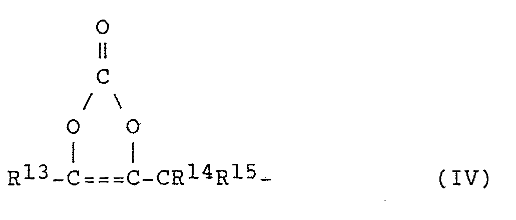 in which:
R¹³ represents a straight or branched chain alkyl group having from 1 to 10 carbon atoms, a cycloalkyl group having from 3 to 10 carbon atoms or an aryl group having from 6 to 10 carbon atoms, which aryl group is unsubstituted or has at least one of substituents (a), defined above;
R¹⁴ and R¹⁵ are the same or different and each represents a hydrogen atoms and straight or branched chain alkyl groups having from 1 to 10 carbon atoms; those groups of formula (V): 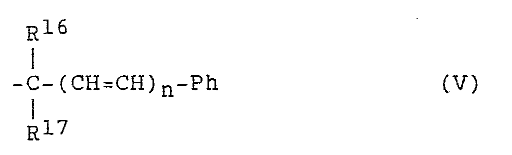 in which
R¹⁶ and R¹⁷ are the same or different and each represents a hydrogen atoms and C₁ - Cs alkyl groups;
Ph represents a phenyl group which is unsubstituted or has at least one substituent selected from Ci - C₁₈ aliphatic acyloxy groups, C₁ - C₁₈ aliphatic acylthio groups, substituted C₁ - C₁₈ aliphatic acyloxy and Ci - C₁₈ aliphatic acylthio groups, aromatic acyloxy groups, aromatic acylthio groups, Ci - C₁₀ alkyl groups, Ci - C₆ alkoxy groups, halogen atoms, hydroxy groups, mercapto groups, carboxy groups, amino groups and nitro groups, in which the substituent(s) on said substituted aliphatic acyloxy and acylthio groups are selected from halogen atoms and Cs - C₁₀ cycloalkyl groups, said aromatic acyloxy and acylthio groups being unsubstituted or having at least one of substituents (a); and n represents 0, 1 or 2; those groups of formula (Vl): 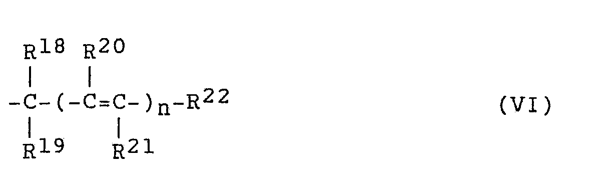 in which
R¹8, R¹⁹, R20 and R²¹, are the same or different and each represents a hydrogen atoms and C₁ - C₆ alkyl groups;
R²² represents a carboxylic acyloxy group; and
n is as defined above; those groups of formula (VII): 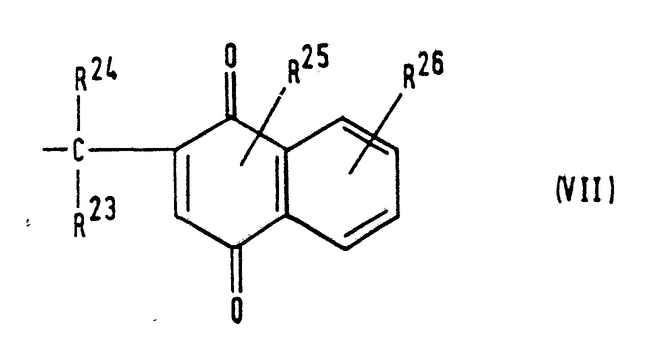 in which R²³ and R²⁴ are the same or different and each represents a hydrogen atom or a C₁ - C₆ alkyl group; and
R²⁵ and R²⁶ are the same or different and each represents a C₁ - C₆ alkyl group, a Ci - C₆ alkoxy group or a halogen atom;
and those groups of formula (VIII): 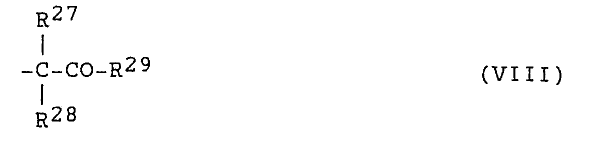 in which:
R²⁷ and R²⁸ are the same or different and each represents a hydrogen atom or a Ci - C₆ alkyl group; and R²⁹ represents a hydrogen atom, a Cᵢ - C₆ alkyl group or an aryl group having from 6 to 14 ring atoms and which is unsubstituted or has at least one of substituents (a), defined above.

In the compounds of the present invention, where R⁷, R⁸, R⁹, R¹⁰, R¹¹, R¹², R¹³, R¹⁴, R¹⁵ or the substituent on the phenyl group represented by Ph represents a straight or branched chain alkyl group having from 1 to 10 carbon atoms, examples include the methyl, ethyl, propyl, isopropyl, butyl, isobutyl, sec-butyl, t-butyl, pentyl, isopentyl, 2-methylbutyl, neopentyl, 1-ethylpropyl, hexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, heptyl, 1-methylhexyl, 2-methylhexyl, 3-methylhexyl, 4-methylhexyl, 5-methylhexyl, 1-propylbutyl, 1,1-diethylpropyl, 4,4-dimethylpentyl, octyl, 1-methylheptyl, 2-methylheptyl, 3-methylheptyl, 4-methylheptyl, 5-methylheptyl, 6-methylheptyl, 1-propylpentyl, 2-ethylhexyl, 5,5-dimethylhexyl, nonyl, 3-methyloctyl, 4-methyloctyl, 5-methyloctyl, 6-methyloctyl, 1-propylhexyl, 2-ethylheptyl, 6,6-dimethylheptyl, decyl, 1-methylnonyl, 3-methylnonyl, 8-methylnonyl, 3-ethyloctyl, 3,7-dimethyloctyl and 7,7-dimethyloctyl, groups, of which we prefer the straight and branched chain alkyl groups having from 1 to 6 carbon atoms.

In the compounds of the present invention, where R⁷, R⁸, R^{iO}, R¹¹, R¹³ or the substituent on an acyloxy or acylthio-substituted phenyl group represented by Ph represents a cycloalkyl group having from 3 to 10 carbon atoms, this may be, for example, a saturated cyclic hydrocarbon group having from 3 to 10 ring carbon atoms (including the saturated terpenic hydrocarbons), such as a cyclopropyl, cyclobutyl, cyclopentyl, cyclohexyl, cycloheptyl, cyclooctyl, cyclononyl, cyclodecyl, pinanyl, bornanyl or norbornanyl group, of which we prefer those saturated cyclic hydrocarbon groups having from 5 to 7 ring carbon atoms.

Where R¹³ represents an aryl group having from 6 to 10 carbon atoms, or R⁷, R¹⁰ or R²⁹ represents an aryl group having from 6 to 14 carbon atoms, this is preferably any one of the aryl groups exemplified above. Preferred such groups include the phenyl and naphthyl groups which may be unsubstituted or have one or more of substituents (a), defined above; examples include the phenyl and naphthyl groups and such groups which are optionally substituted.

Where R¹⁶, R¹⁷, R18, R19, R20, R21, R23, R24, R25, R26, R27, R28 or R29 represents an alkyl group, this may be a straight or branched chain group having from 1 to 6 carbon atoms and examples of such groups include the methyl, ethyl, propyl, isopropyl, butyl, isobutyl, sec-butyl, t-butyl, pentyl, isopentyl, 2-methylbutyl, neopentyl, 1-ethylpropyl, hexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl and 2-ethylbutyl groups.

Where R²² represents a carboxylic acyloxy group, this is preferably an aliphatic acyloxy group having from 1 to 18 carbon atoms, and is more preferably: an alkanoyloxy group, for example a Ci - C₁₈ alkanoyloxy group, e.g. a formyloxy, acetoxy, propionyloxy, butyryloxy, 2-pentenoyloxy, isobutyryloxy, 2-hexenoyloxy, pivaloyloxy, valeryloxy, isovaleryloxy, octanoyloxy, decanoyloxy, 4-methyldecanoyloxy, 9-methyldecanoyloxy, 4-ethylnona- noyloxy, 4,8-dimethylnonanoyloxy, dodecanoyloxy, tetradecanoyloxy, pentadecanoyloxy, hexadecanoyloxy, hexadecenoyloxy, heptadecanoyloxy, 2-methylhexadecanoyloxy, 15-methylhexadecanoyloxy, 14,14-dimethyl- pentadecanoyloxy, 16-methylheptadecanoyloxy, lauroyloxy, myristoyloxy, tridecanoyloxy, palmitoyloxy or stearoyloxy group; a halogenated alkanoyloxy group, especially a C₂ - C₆, more preferably C₂ - C₄, haloalkanoyloxy group (in which there may be one or more halogen atoms, e.g. from 1 to 3 halogen atoms), and most preferably a haloacetoxy group, e.g. a chloroacetoxy, dichloroacetoxy, trichloroacetoxy or trifluoroacetoxy group; or an alkanoyloxy group having from 1 to 3, more preferably 1, C₄ - C₁₀ cycloalkyl substituents; examples of such groups include the cyclopropylacetoxy, cyclobutylacetoxy, cyclopentylacetoxy, cyclohexy- lacetoxy, cycloheptylacetoxy, cyclooctylacetoxy, tetrahydronaphthylacetoxy, bornanylacetoxy, 8,9,10-norbor- nanylacetoxy, 3-cyclopropylpropionyloxy, 3-cyclobutylpropionyloxy, 3-cyclopentylpropionyloxy, 3-cyclohe- xylpropionyloxy, 3-cycloheptylpropionyloxy, 3-cyclooctylpropionyloxy, 3-tetrahydronaphthylpropionyloxy, 3-bornanylpropionyloxy, 3-(8,9,10-norbornanyl)propionyloxy, 4-cyclopropylbutyryloxy, 4-cyclobutylbutyryloxy, 4-cyclopentylbutyryloxy, 4-cyclohexylbutyryloxy, 4-cycloheptylbutyryloxy, 4-cyclooctylbutyryloxy, 4-tetrahy- dronaphthylbutyryloxy, 4-bornanylbutyryloxy and 4-(8,9,10-norbornanyl)butyryloxy groups.

Alternatively, R²² may represent an aromatic acyloxy group, such as an arylcarbonyloxy group, in which the aryl part is preferably a phenyl or naphthyl group which is unsubstituted or has at least one of substituents (a), as defined above, for example: an unsubstituted group, such as the benzoyloxy, a-naphthoyloxy and β-naphthoyloxy groups; a halogen-substituted arylcarbonyloxy group, such as the 2-bromobenzoyloxy and 4-chlorobenzoyloxy groups; a lower alkyl-substituted arylcarbonyloxy group, such as the 2,4,6-trimethylbenzoyloxy and 4-toluoyloxy groups; a lower alkoxy-substituted arylcarbonyloxy group, such as the 4-anisoyloxy group; a nitrated arylcarbonyloxy group, such as the 4-nitrobenzoyloxy and 2-nitrobenzoyloxy groups; a lower alkoxycarbonylated arylcarbonyloxy group, in which the alkoxy group is preferably Ci - C₆, more preferably Ci - C4, such as the 2-(methoxycarbonyl)benzoyloxy group; or an aryl-substituted arylcarbonyloxy group, such as the 4-phenylbenzoyloxy group.

R²² may also represent an acylthio group, which is preferably an aliphatic acylthio group having from 1 to 18 carbon atoms, and is more preferably an alkanoylthio group, for example: a C₁ - C₁₈ alkanoylthio group, e.g. a formylthio, acetylthio, propionylthio, butyrylthio, isobutyrylthio, pentanoylthio, pivaloylthio, valerylthio, isovalerylthio, octanoylthio, lauroylthio, myristoylthio, tridecanoylthio, palmitoylthio or stearoylthio group; a halogenated alkanoylthio group, especially a C₂ - Cₛ, more preferably C₂ - C₄, haloalkanoylthio group (in which there may be one or more halogen atoms, e.g. from 1 to 3 halogen atoms), and most preferably a haloacetylthio group, e.g. a chloroacetylthio, dichloroacetylthio, trichloroacetylthio or trifluoroacetylthio group; or an alkanoylthio group having from 1 to 3, more preferably 1, C₄ - C₁₀ cycloalkyfthio substituent; examples of such groups include the cyclopropylacetylthio, cyclobutylacetylthio, cyclopentylacetylthio, cyclohexylacetylthio, cycloheptylacetylthio, cyclooctylacetylthio, tetrahydronaphthylacetylthio, bornanylacetylthio, 8,9,10-norborna- nylthioacetylthio, 3-cyclopropylpropionylthio, 3-cyclobutylpropionylthio, 3-cyclo pentylpropionylthio, 3-cy- clohexylpropionylthio, 3-cycloheptylpropionylthio 3-cyclooctylpropionylthio, 3-tetrahydronaphthylpropio- nylthio, 3-bornanylpropionylthio, 3-(8,9,10-norbornanyl)propionylthio, 4-cyclopropylbutyrylthio, 4-cyclobu- tylbutyrylthio, 4-cyclopentylbutyrylthio, 4-cyclohexylbutyrylthio, 4-cycloheptylbutyrylthio, 4-cyclooctylbuty- rylthio, 4-tetrahydronaphthylbutyrylthio, 4-bornanylbutyrylthio and 4-(8,9,10-norbornanyl)butyrylthio groups.

Alternatively, it may be an aromatic acylthio group, such as an arylcarbonylthio group, in which the aryl part is preferably a phenyl or naphthyl group which is unsubstituted or has at least one of substituents (a), as defined above, for example: an unsubstituted group, such as the benzoylthio, a-naphthoylthio and R-naphthoylthio groups; a halogen-substituted arylcarbonylthio group, such as the 2-bromobenzoylthio and 4-chlorobenzoylthio groups; a lower alkyl-substituted arylcarbonylthio group, such as the 2,4,6-trimethylben- zoylthio and 4-toluoylthio groups; a lower alkoxy-substituted arylcarbonylthio group, such as the 4-anisoylthio group; a nitrated arylcarbonylthio group, such as the 4-nitrobenzoylthio and 2-nitrobenzoylthio groups; a lower alkoxycarbonylated arylcarbonylthio group, in which the alkoxy group is preferably C₁ - C₆, more preferably Ci - C₄, such as the 2-(methoxycarbonyl)benzoylthio group; or an aryl-substituted arylcarbonylthio group, such as the 4-phenylbenzoylthio group.

Where the substituent on the phenyl group represented by Ph is an aliphatic acyloxy group, an aliphatic acylthio group, an aromatic acyloxy group or an aromatic acylthio group, then this may be any one of those groups exemplified above for R22.

Where R²⁵ or R²⁶ represents an alkoxy group, this may be a straight or branched chain group having from 1 to 6 carbon atoms, preferably from 1 to 4 carbon atoms, and examples include the methoxy, ethoxy, propoxy, isopropoxy, butoxy, isobutoxy, sec-butoxy, t-butoxy, pentyloxy, isopentyloxy, 2-methylbutoxy, neopentyloxy, 1-ethylpropoxy, hexyloxy, 4-methylpentyloxy, 3-methylpentyloxy, 2-methylpentyloxy, 1-methylpentyloxy, 3,3-dimethylbutoxy, 2,2-dimethylbutoxy, 1,1-dimethylbutoxy, 1,2-dimethylbutoxy, 1,3-dimethylbutoxy, 2,3-dimethylbutoxy and 2-ethylbutoxy groups.

Where halogen atoms are referred to herein, e.g. in connection with substituents on the phenyl group represented by Ph, or in connection with R²⁵ or R²⁶, these may be the fluorine, chlorine, bromine or iodine atoms.

n represents the cypher 0 or the integer 1 or 2, preferably 0 or 1.

Most preferably, one of R⁵ and R⁶ represents an ester group of formula (Ila): 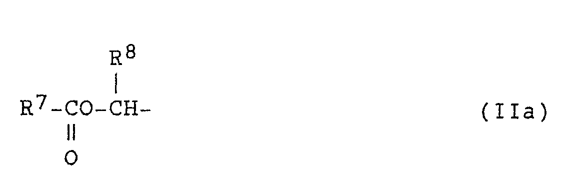
in which R⁷ and R⁸ are as defined above; or a group of formula (Illa): 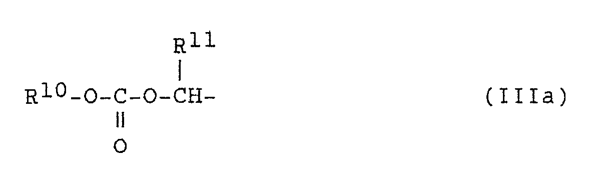
in which R¹⁰ and R11 are as defined above; or a group of formula (IVa): 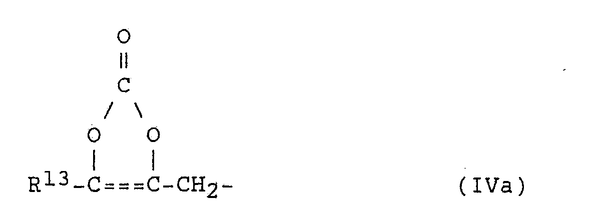
in which R¹³ is as defined above; or a group of formula (Va): 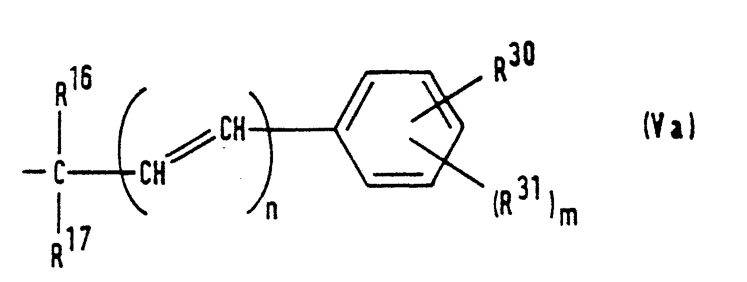 in which:
   R¹⁶, R¹⁷ and n are as defined above;
   m represents the cypher 0 or an integer from 1 to 3, preferably 0, 1 or 2.
   R̅³⁰ represents a C₁ - C₁₈ aliphatic acyloxy group, a Ci - C₁₈ aliphatic acylthio group, a substituted Ci - C₁₈ aliphatic acyloxy or C₁- C₁₈ aliphatic acylthio group, an aromatic acyloxy group or an aromatic acylthio group, said aromatic acyloxy and acylthio groups being unsubstituted or having at least one of substituents (a); R³¹ represents a C₁- C₁₈ aliphatic acyloxy group, a Ci - C₁₈ aliphatic acylthio group, a substituted Ci - C₁₈ aliphatic acyloxy or Ci - C₁₈ aliphatic acylthio group, an aromatic acyloxy group, an aromatic acylthio group, a Ci - C₁₀ alkyl group, a Ci - C₆ alkoxy group, a halogen atom, a hydroxy group, a mercapto group, a carboxy group, an amino group or a nitro group, said aromatic acyloxy and acylthio groups being unsubstituted or having at least one of substituents (a);
   the substituent(s) on said substituted acyloxy and acylthio groups represented by R³⁰ and R³¹ being selected from halogen atoms and C₃ - C₁₀ cycloalkyl groups.

In the above formulae, _{R}7, _{R}8, R¹⁰, R¹¹, R¹³, R¹⁶, R17 and n are preferably as exemplified above, and R³⁰ and R³¹ are preferably as exemplified above in relation to substituents on phenyl groups.

Preferred classes of compounds of the present invention are those compounds of formula (I), in which:
(1) R¹ and R² are the same or different and each represents a hydrogen atom, a hydroxy group, a Ci - C₁₈ aliphatic carboxylic acyloxy group or a aromatic carboxylic acyloxy group.
(2) R¹ and R² are the same or different and each represents a hydrogen atom or a hydroxy group.
(3) R³ and R⁴ together form a single bond.
(4) one of R⁵ and R⁶ represents a group of formula: 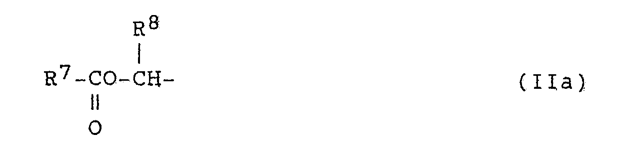 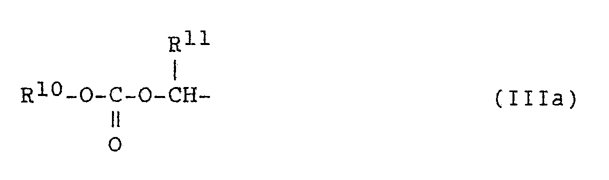 or 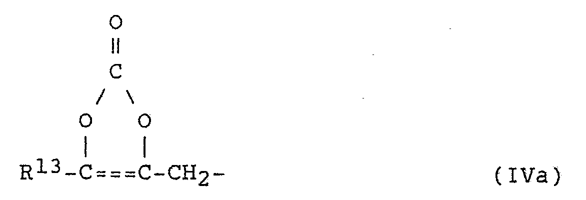 in which _{R}7, R8, R¹⁰, R¹¹ and R¹³ are as defined above.
(5) one of R⁵ and R⁶ represents a group of formula (Ila), as shown in (4) above, in which R⁷ represents a straight or branched chain alkyl group having from 1 to 6 carbon atoms or a cycloalkyl group having from 5 to 7 carbon atoms.
(6) one of R⁵ and R⁶ represents a group of formula (Ila), as shown in (4) above, in which R⁸ represents a hydrogen atom or a straight or branched chain alkyl group having from 1 to 6 carbon atoms.
(7) one of R⁵ and R⁶ represents a group of formula (Ila), as shown in (4) above, in which R⁸ represents a hydrogen atom or a straight or branched alkyl group having 1 to 3 carbon atoms.
(8) one of R⁵ and R⁶ represents a group of formula (Illa), as shown in (4) above, in which R¹⁰ represents a straight or branched chain alkyl group having from 1 to 6 carbon atoms.
(9) one of R⁵ and R⁶ represents a group of formula (illa), as shown in (4) above, in which R¹¹ represents a hydrogen atom or a straight or branched chain alkyl group having from 1 to 6 carbon atoms.
(10) one of R⁵ and R⁶ represents a group of formula (Illa), as shown in (4) above, in which R¹¹ represents a hydrogen atom or a straight or branched chain alkyl group having from 1 to 3 carbon atoms.
(11) one of R⁵ and R⁶ represents a group of formula (IVa), as shown in (4) above, in which R¹³ represents a phenyl group, a straight or branched chain alkyl group having from 1 to 6 carbon atoms, or a cycloalkyl group having from 5 to 7 carbon atoms.
(12) one of R⁵ and R⁶ represents a group of formula (IVa), as shown in (4) above, in which R¹³ represents a straight or branched chain alkyl group having from 1 to 6 carbon atoms.
(13) one of R⁵ and R⁶ represents a group of formula (IVa), as shown in (4) above, in which R¹³ represents a straight or branched chain alkyl group having from 1 to 3 carbon atoms.
(14) R¹ and R² are the same or different and each represents a hydrogen atom, a hydroxy group, a Ci - C₁₈ aliphatic carboxylic acyloxy group or an aromatic carboxylic acyloxy group; and one of R⁵ and R⁶ represents a group of formula (Ila), (Illa) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 6 carbon atoms;
   R¹⁰ represents a straight or branched chain alkyl group having from 1 to 6 carbon atoms;
   R¹¹ represents a hydrogen atom or a straight or branched chain alkyl group having from 1 to 6 carbon atoms; and
   R¹³ represents a phenyl group, a straight or branched chain alkyl group having from 1 to 6 carbon atoms or a cycloalkyl group having from 5 to 7 carbon atoms.
(15) R¹ and R² are the same or different and each represents a hydrogen atom, a hydroxy group, a Ci - C₁₈ aliphatic carboxylic acyloxy group or an aromatic carboxylic acyloxy group;
   R³ and R⁴ each represents a hydrogen atom, or together represent an extra carbon-carbon bond between the two carbon atoms to which they are attached; and one of R⁵ and R⁶ represents a group of formula (Ila), (Illa) or (lVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 6 carbon atoms;
   R¹⁰ represents a straight or branched chain alkyl group having from 1 to 6 carbon atoms;
   R¹¹ represents a hydrogen atom or a straight or branched chain alkyl group having from 1 to 6 carbon atoms; and
   R¹³ represents a straight or branched chain alkyl group having from 1 to 6 carbon atoms.
(16) R¹ and R² are the same or different and each represents a hydrogen atom, a hydroxy group, a Ci - C₁₈ aliphatic carboxylic acyloxy group or an aromatic carboxylic acyloxy group; and one of R⁵ and R⁶ represents a group of formula (Ila), (ilia) or (lVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 6 carbon atoms;
   R¹ represents a straight or branched chain alkyl group having from 1 to 6 carbon atoms;
   R11 represents a hydrogen atom or a straight or branched chain alkyl group having from 1 to 6 carbon atoms; and
   R¹³ represents a straight or branched chain alkyl group having from 1 to 3 carbon atoms.
(17) R¹ and R² are the same or different and each represents a hydrogen atom, a hydroxy group, a Ci - C₁₈ aliphatic carboxylic acyloxy group or an aromatic carboxylic acyloxy groups; and
   one of R⁵ and R⁶ represents a group of formula (Ila), (IIIa) or (lVa), as defined in (4), above, in which: wherein R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 6 carbon atoms;
   R¹⁰ represents a straight or branched chain alkyl group having from 1 to 6 carbon atoms;
   R¹¹ represents a hydrogen atom or a straight or branched chain alkyl group having from 1 to 3 carbon atoms; and
   R¹³ represents a phenyl group, a straight or branched chain alkyl group having from 1 to 6 carbon atoms or a cycloalkyl group having from 5 to 7 carbon atoms.
(18) R¹ and R² are the same or different and each represents a hydrogen atom, a hydroxy group, a Ci - C₁₈ aliphatic carboxylic acyloxy group or an aromatic carboxylic acyloxy groups; and one of R⁵ and R⁶ represents a group of formula (Ila), (Illa) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 6 carbon atoms;
   R¹⁰ represents a straight or branched chain alkyl group having from 1 to 6 carbon atoms;
   R¹¹ represents a hydrogen atom or a straight or branched chain alkyl group having from 1 to 3 carbon atoms; and
   R¹³ represents a straight or branched chain alkyl group having from 1 to 6 carbon atoms.
(19) R¹ and R² are the same or different and each represents a hydrogen atom, a hydroxy group, a C₁ - C₁₈ aliphatic carboxylic acyloxy group or an aromatic carboxylic acyloxy groups; and one of R⁵ and R⁶ represents a group of formula (Ila), (Illa) or (lVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 6 carbon atoms;
   R¹⁰ represents a straight or branched chain alkyl group having from 1 to 6 carbon atoms;
   R¹¹ represents a hydrogen atom or a straight or branched chain alkyl group having from 1 to 3 carbon atoms; and
   R¹³ represents a straight or branched chain alkyl group having from 1 to 3 carbon atoms.
(20) R¹ and R² are the same or different and each represents a hydrogen atom, a hydroxy group, a C₁ - C₁₈ aliphatic carboxylic acyloxy group or an aromatic carboxylic acyloxy groups; and one of R⁵ and R⁶ represents a group of formula (Ila), (Illa) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 3 carbon atoms;
   R¹⁰ represents a straight or branched chain alkyl group having from 1 to 6 carbon atoms;
   R¹¹ represents a hydrogen atom or a straight or branched chain alkyl group having from 1 to 6 carbon atoms; and
   R¹³ represents a phenyl group, a straight or branched chain alkyl group having from 1 to 6 carbon atoms or a cycloalkyl group having from 5 to 7 carbon atoms.
(21) R¹ and R² are the same or different and each represents a hydrogen atom, a hydroxy group, a C₁-C₁₈ aliphatic carboxylic acyloxy group or an aromatic carboxylic acyloxy groups; and one of R⁵ and R⁶ represents a group of formula (Ila), (Illa) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 3 carbon atoms;
   R¹⁰ represents a straight or branched chain alkyl group having from 1 to 6 carbon atoms;
   R¹¹ represents a hydrogen atom or a straight or branched chain alkyl group having from 1 to 6 carbon atoms; and
   R¹³ represents a straight or branched chain alkyl group having from 1 to 6 carbon atoms.
(22) R¹ and R² are the same or different and each represents a hydrogen atom, a hydroxy group, a Cᵢ - C₁₈ aliphatic carboxylic acyloxy group or an aromatic carboxylic acyloxy groups; and one of R⁵ and R⁶ represents a group of formula (Ila), (Illa) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 3 carbon atoms;
   R¹⁰ represents a straight or branched chain alkyl group having from 1 to 6 carbon atoms;
   R¹¹ represents a hydrogen atom or a straight or branched chain alkyl group having from 1 to 6 carbon atoms; and
   R¹³ represents a straight or branched chain alkyl group having from 1 to 3 carbon atoms.
(23) R¹ and R² are the same or different and each represents a hydrogen atom, a hydroxy group, a Ci - C₁₈ aliphatic carboxylic acyloxy group or an aromatic carboxylic acyloxy groups; and one of R⁵ and R⁶ represents a group of formula (Ila), (Ilia) or (lVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 3 carbon atoms;
   R¹⁰ represents a straight or branched chain alkyl group having from 1 to 6 carbon atoms;
   R¹¹ represents a hydrogen atom or a straight or branched chain alkyl group having from 1 to 3 carbon atoms; and
   R¹³ represents a phenyl group, a straight or branched chain alkyl group having from 1 to 6 carbon atoms or a cycloalkyl group having from 5 to 7 carbon atoms.
(24) R¹ and R² are the same or different and each represents a hydrogen atom, a hydroxy group, a C₁ - C₁₈ aliphatic carboxylic acyloxy group or an aromatic carboxylic acyloxy groups; and one of R⁵ and R⁶ represents a group of formula (Ila), (Illa) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 3 carbon atoms;
   R¹⁰ represents a straight or branched chain alkyl group having from 1 to 6 carbon atoms;
   R¹¹ represents a hydrogen atom or a straight or branched chain alkyl group having from 1 to 3 carbon atoms; and
   R¹³ represents a straight or branched chain alkyl group having from 1 to 6 carbon atoms.
(25) R¹ and R² are the same or different and each represents a hydrogen atom, a hydroxy group, a Ci - C₁₈ aliphatic carboxylic acyloxy group or an aromatic carboxylic acyloxy groups; and one of R⁵ and R⁶ represents a group of formula (Ila), (Illa) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 3 carbon atoms;
   R¹⁰ represents a straight or branched chain alkyl group having from 1 to 6 carbon atoms;
   R¹¹ represents a hydrogen atom or a straight or branched chain alkyl group having from 1 to 3 carbon atoms; and
   R¹³ represents a straight or branched chain alkyl group having from 1 to 3 carbon atoms.
(26) R¹ and R² are the same or different and each represents a hydrogen atom, a hydroxy group, a Ci - C₁₈ aliphatic carboxylic acyloxy group or an aromatic carboxylic acyloxy groups;
   R³ and R⁴ together form a single bond; and one of R⁵ and R⁶ represents a group of formula (Ila), (Illa) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 6 carbon atoms;
   R¹⁰ represents a straight or branched chain alkyl group having from 1 to 6 carbon atoms;
   R¹¹ represents a hydrogen atom or a straight or branched chain alkyl group having from 1 to 6 carbon atoms; and
   R¹³ represents a phenyl group, a straight or branched chain alkyl group having from 1 to 6 carbon atoms or a cycloalkyl group having from 5 to 7 carbon atoms.
(27) R¹ and R² are the same or different and each represents a hydrogen atom, a hydroxy group, a Ci - C¹⁸ aliphatic carboxylic acyloxy group or an aromatic carboxylic acyloxy groups;
   R³ and R⁴ together form a single bond; and one of R⁵ and R⁶ represents a group of formula (Ila), (Illa) or (lVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 6 carbon atoms;
   R¹⁰ represents a straight or branched chain alkyl group having from 1 to 6 carbon atoms;
   R¹¹ represents a hydrogen atom or a straight or branched chain alkyl group having from 1 to 6 carbon atoms; and
   R¹³ represents a straight or branched chain alkyl group having from 1 to 6 carbon atoms.
(28) R¹ and R² are the same or different and each represents a hydrogen atom, a hydroxy group, a C₁ - C₁₈ aliphatic carboxylic acyloxy group or an aromatic carboxylic acyloxy groups;
   R³ and R⁴ together form a single bond; and one of R⁵ and R⁶ represents a group of formula (Ila), (Illa) or (lVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 6 carbon atoms;
   R¹⁰ represents a straight or branched chain alkyl group having from 1 to 6 carbon atoms;
   R¹¹ represents a hydrogen atom or a straight or branched chain alkyl group having from 1 to 6 carbon atoms; and
   R¹³ represents a straight or branched chain alkyl group having from 1 to 3 carbon atoms.
(29) R¹ and R² are the same or different and each represents a hydrogen atom, a hydroxy group, a Ci - C₁₈ aliphatic carboxylic acyloxy group or an aromatic carboxylic acyloxy groups;
   R³ and R⁴ together form a single bond; and one of R⁵ and R⁶ represents a group of formula (Ila), (Illa) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 6 carbon atoms;
   R¹⁰ represents a straight or branched chain alkyl group having from 1 to 6 carbon atoms;
   R¹¹ represents a hydrogen atom or a straight or branched chain alkyl group having from 1 to 3 carbon atoms; and
   R¹³ represents a phenyl group, a straight or branched chain alkyl group having from 1 to 6 carbon atoms or a cycloalkyl group having from 5 to 7 carbon atoms.
(30) R¹ and R² are the same or different and each represents a hydrogen atom, a hydroxy group, a C₁ - Ci aliphatic carboxylic acyloxy group or an aromatic carboxylic acyloxy groups;
   R³ and R⁴ together form a single bond; and one of R⁵ and R⁶ represents a group offormula (Ila), (Illa) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 6 carbon atoms;
   R¹⁰ represents a straight or branched chain alkyl group having from 1 to 6 carbon atoms;
   R¹¹ represents a hydrogen atom or a straight or branched chain alkyl group having from 1 to 3 carbon atoms; and
   R¹³ represents a straight or branched chain alkyl group having from 1 to 6 carbon atoms.
(31) R¹ and R² are the same or different and each represents a hydrogen atom, a hydroxy group, a C₁ - C₁₈ aliphatic carboxylic acyloxy group or an aromatic carboxylic acyloxy groups;
   R³ and R⁴ together form a single bond; and one of R⁵ and R⁶ represents a group of formula (Ila), (Illa) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 6 carbon atoms;
   R¹⁰ represents a straight or branched chain alkyl group having from 1 to 6 carbon atoms;
   R¹¹ represents a hydrogen atom or a straight or branched chain alkyl group having from 1 to 3 carbon atoms; and
   R¹³ represents a straight or branched chain alkyl group having from 1 to 3 carbon atoms.
(32) R¹ and R2 are the same or different and each represents a hydrogen atom, a hydroxy group, a Cᵢ - C₁₈ aliphatic carboxylic acyloxy group or an aromatic carboxylic acyloxy groups;
   R3 and R⁴ together form a single bond; and one of R⁵ and R⁶ represents a group of formula (Ila), (Ilia) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 3 carbon atoms;
   R¹⁰ represents a straight or branched chain alkyl group having from 1 to 6 carbon atoms;
   R¹¹ represents a hydrogen atom or a straight or branched chain alkyl group having from 1 to 6 carbon atoms; and
   R¹³ represents a phenyl group, a straight or branched chain alkyl group having from 1 to 6 carbon atoms or a cycloalkyl group having from 5 to 7 carbon atoms.
(33) R¹ and R² are the same or different and each represents a hydrogen atom, a hydroxy group, a Cᵢ - C₁ aliphatic carboxylic acyloxy group or an aromatic carboxylic acyloxy groups;
   R3 and R⁴ together form a single bond; and one of R⁵ and R⁶ represents a group of formula (Ila), (ilia) or (lVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 3 carbon atoms;
   R¹⁰ represents a straight or branched chain alkyl group having from 1 to 6 carbon atoms;
   R¹¹ represents a hydrogen atom or a straight or branched chain alkyl group having from 1 to 6 carbon atoms; and
   R¹³ represents a straight or branched chain alkyl group having from 1 to 6 carbon atoms.
(34) R¹ and R2 are the same or different and each represents a hydrogen atom, a hydroxy group, a C₁ - C₁₈ aliphatic carboxylic acyloxy group or an aromatic carboxylic acyloxy groups;
   R³ and R⁴ together form a single bond; and one of R⁵ and R⁶ represents a group of formula (Ila), (Illa) or (lVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 3 carbon atoms;
   R¹⁰ represents a straight or branched chain alkyl group having from 1 to 6 carbon atoms;
   R¹¹ represents a hydrogen atom or a straight or branched chain alkyl group having from 1 to 6 carbon atoms;
   and R¹³ represents a straight or branched chain alkyl group having from 1 to 3 carbon atoms.
(35) R¹ and R² are the same or different and each represents a hydrogen atom, a hydroxy group, a Ci - C₁₈ aliphatic carboxylic acyloxy group or an aromatic carboxylic acyloxy groups;
   R³ and R⁴ together form a single bond; and one of R⁵ and R⁶ represents a group of formula (Ila), (Illa) or (lVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 3 carbon atoms;
   R¹⁰ represents a straight or branched chain alkyl group having from 1 to 6 carbon atoms;
   R¹¹ represents a hydrogen atom or a straight or branched chain alkyl group having from 1 to 3 carbon atoms; and
   R¹³ represents a phenyl group, a straight or branched chain alkyl group having from 1 to 6 carbon atoms or a cycloalkyl group having from 5 to 7 carbon atoms.
(36) R¹ and R² are the same or different and each represents a hydrogen atom, a hydroxy group, a Ci - C₁₈ aliphatic carboxylic acyloxy group or an aromatic carboxylic acyloxy groups;
   R³ and R⁴ together form a single bond; and one of R⁵ and R⁶ represents a group of formula (Ila), (Illa) or (lVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 3 carbon atoms;
   R¹⁰ represents a straight or branched chain alkyl group having from 1 to 6 carbon atoms;
   R¹¹ represents a hydrogen atom or a straight or branched chain alkyl group having from 1 to 3 carbon atoms; and
   R¹³ represents a straight or branched chain alkyl group having from 1 to 6 carbon atoms.
(37) R¹ and R² are the same or different and each represents a hydrogen atom, a hydroxy group, a Ci - C₁₈ aliphatic carboxylic acyloxy group or an aromatic carboxylic acyloxy groups;
   R³ and R⁴ together form a single bond; and one of R⁵ and R⁶ represents a group of formula (Ila), (Illa) or (lVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 3 carbon atoms;
   R¹⁰ represents a straight or branched chain alkyl group having from 1 to 6 carbon atoms;
   R¹¹ represents a hydrogen atom or a straight or branched chain alkyl group having from 1 to 3 carbon atoms; and
   R¹³ represents a straight or branched chain alkyl group having from 1 to 3 carbon atoms.
(38) R and R² are the same or different and each represents a hydrogen atom or a hydroxy group; and one of R⁵ and R⁶ represents a group of formula (Ila), (Illa) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 6 carbon atoms;
   R¹⁰ represents a straight or branched chain alkyl group having from 1 to 6 carbon atoms;
   R¹¹ represents a hydrogen atom or a straight or branched chain alkyl group having from 1 to 6 carbon atoms;
   and R¹³ represents a phenyl group, a straight or branched chain alkyl group having from 1 to 6 carbon atoms or a cycloalkyl group having from 5 to 7 carbon atoms.
(39) R¹ and R² are the same or different and each represents a hydrogen atom or a hydroxy group; and one of R⁵ and R⁶ represents a group of formula (Ila), (Illa) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 6 carbon atoms;
   R¹⁰ represents a straight or branched chain alkyl group having from 1 to 6 carbon atoms;
   R¹¹ represents a hydrogen atom or a straight or branched chain alkyl group having from 1 to 6 carbon atoms; and
   R¹³ represents a straight or branched chain alkyl group having from 1 to 6 carbon atoms.
(40) R¹ and R² are the same or different and each represents a hydrogen atom or a hydroxy group; and one of R⁵ and R⁶ represents a group of formula (Ila), (Illa) or (lVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 6 carbon atoms;
   R¹⁰ represents a straight or branched chain alkyl group having from 1 to 6 carbon atoms;
   R¹¹ represents a hydrogen atom or a straight or branched chain alkyl group having from 1 to 6 carbon atoms; and
   R¹³ represents a straight or branched chain alkyl group having from 1 to 3 carbon atoms.
(41) R¹ and R² are the same or different and each represents a hydrogen atom or a hydroxy group; and one of R⁵ and R⁶ represents a group of formula (Ila), (Illa) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 6 carbon atoms;
   R¹⁰ represents a straight or branched chain alkyl group having from 1 to 6 carbon atoms;
   R¹¹ represents a hydrogen atom or a straight or branched chain alkyl group having from 1 to 3 carbon atoms; and
   R¹³ represents a phenyl group, a straight or branched chain alkyl group having from 1 to 6 carbon atoms or a cycloalkyl group having from 5 to 7 carbon atoms.
(42) R¹ and R² are the same or different and each represents a hydrogen atom or a hydroxy group; and one of R⁵ and R⁶ represents a group of formula (Ila), (Illa) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 6 carbon atoms;
   R¹⁰ represents a straight or branched chain alkyl group having from 1 to 6 carbon atoms;
   R¹¹ represents a hydrogen atom or a straight or branched chain alkyl group having from 1 to 3 carbon atoms; and
   R¹³ represents a straight or branched chain alkyl group having from 1 to 6 carbon atoms.
(43) R and R² are the same or different and each represents a hydrogen atom or a hydroxy group; and one of R⁵ and R⁶ represents a group of formula (Ila), (ilia) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 6 carbon atoms;
   R¹⁰ represents a straight or branched chain alkyl group having from 1 to 6 carbon atoms;
   R¹¹ represents a hydrogen atom or a straight or branched chain alkyl group having from 1 to 3 carbon atoms; and
   R¹³ represents a straight or branched chain alkyl group having from 1 to 3 carbon atoms.
(44) R¹ and R² are the same or different and each represents a hydrogen atom or a hydroxy group; and one of R⁵ and R⁶ represents a group of formula (Ila), (Illa) or (lVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 3 carbon atoms;
   R¹⁰ represents a straight or branched chain alkyl group having from 1 to 6 carbon atoms;
   R¹¹ represents a hydrogen atom or a straight or branched chain alkyl group having from 1 to 6 carbon atoms; and
   R¹³ represents a phenyl group, a straight or branched chain alkyl group having from 1 to 6 carbon atoms or a cycloalkyl group having from 5 to 7 carbon atoms.
(45) R¹ and R² are the same or different and each represents a hydrogen atom or a hydroxy group; and one of R⁵ and R⁶ represents a group of formula (Ila), (Ilia) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 3 carbon atoms;
   R¹⁰ represents a straight or branched chain alkyl group having from 1 to 6 carbon atoms;
   R¹¹ represents a hydrogen atom or a straight or branched chain alkyl group having from 1 to 6 carbon atoms; and
   R¹³ represents a straight or branched chain alkyl group having from 1 to 6 carbon atoms.
(46) R and R² are the same or different and each represents a hydrogen atom or a hydroxy group; and one of R⁵ and R⁶ represents a group offormula (Ila), (ilia) or (lVa), as defined in (4), above, in which:
   R⁷ represents a straight or branched chain alkyl group having from 1 to 6 carbon atoms or a cycloalkyl group having from 5 to 7 carbon atoms; R⁸ represents a hydrogen atom or a straight or branched chain alkyl group having from 1 to 3 carbon atoms;
   R¹⁰ represents a straight or branched chain alkyl group having from 1 to 6 carbon atoms;
   R¹¹ represents a hydrogen atom or a straight or branched chain alkyl group having from 1 to 6 carbon atoms; and
   R¹³ represents a straight or branched chain alkyl group having from 1 to 3 carbon atoms.
(47) R and R² are the same or different and each represents a hydrogen atom or a hydroxy group; and one of R⁵ and R⁶ represents a group of formula (Ila), (Illa) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 3 carbon atoms;
   R¹⁰ represents a straight or branched chain alkyl group having from 1 to 6 carbon atoms;
   R¹¹ represents a hydrogen atom or a straight or branched chain alkyl group having from 1 to 3 carbon atoms; and
   R¹³ represents a phenyl group, a straight or branched chain alkyl group having from 1 to 6 carbon atoms or a cycloalkyl group having from 5 to 7 carbon atoms.
(48) R¹ and R² are the same or different and each represents a hydrogen atom or a hydroxy group; and one of R⁵ and R⁶ represents a group of formula (Ila), (Illa) or (lVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 3 carbon atoms;
   R¹⁰ represents a straight or branched chain alkyl group having from 1 to 6 carbon atoms;
   R¹¹ represents a hydrogen atom or a straight or branched chain alkyl group having from 1 to 3 carbon atoms; and
   R¹³ represents a straight or branched chain alkyl group having from 1 to 6 carbon atoms.
(49) R¹ and R² are the same or different and each represents a hydrogen atom or a hydroxy group; and one of R⁵ and R⁶ represents a group of formula (Ila), (Illa) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 3 carbon atoms;
   R¹⁰ represents a straight or branched chain alkyl group having from 1 to 6 carbon atoms;
   R¹¹ represents a hydrogen atom or a straight or branched chain alkyl group having from 1 to 3 carbon atoms; and
   R¹³ represents a straight or branched chain alkyl group having from 1 to 3 carbon atoms.
(50) R¹ and R² are the same or different and each represents a hydrogen atom or a hydroxy group; and R³ and R⁴ together form a single bond;
   one of R⁵ and R⁶ represents a group of formula (Ila), (IIIa) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 6 carbon atoms;
   R¹⁰ represents a straight or branched chain alkyl group having from 1 to 6 carbon atoms;
   R¹¹ represents a hydrogen atom or a straight or branched chain alkyl group having from 1 to 6 carbon atoms; and
   R¹³ represents a phenyl group, a straight or branched chain alkyl group having from 1 to 6 carbon atoms or a cycloalkyl group having from 5 to 7 carbon atoms.
(51) R and R² are the same or different and each represents a hydrogen atom or a hydroxy group; and R³ and R⁴ together form a single bond;
   one of R⁵ and R⁶ represents a group of formula (lIa), (Illa) or (lVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 6 carbon atoms;
   R¹⁰ represents a straight or branched chain alkyl group having from 1 to 6 carbon atoms;
   R¹¹ represents a hydrogen atom or a straight or branched chain alkyl group having from 1 to 6 carbon atoms; and
   R¹³ represents a straight or branched chain alkyl group having from 1 to 6 carbon atoms.
(52) R¹ and R² are the same or different and each represents a hydrogen atom or a hydroxy group; and R³ and R⁴ together form a single bond;
   one of R⁵ and R⁶ represents a group of formula (Ila), (Illa) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 6 carbon atoms;
   R¹⁰ represents a straight or branched chain alkyl group having from 1 to 6 carbon atoms;
   R¹¹ represents a hydrogen atom or a straight or branched chain alkyl group having from 1 to 6 carbon atoms; and
   R¹³ represents a straight or branched chain alkyl group having from 1 to 3 carbon atoms.
(53) R¹ and R² are the same or different and each represents a hydrogen atom or a hydroxy group; and R³ and R4 together form a single bond;
   one of R⁵ and R⁶ represents a group of formula (Ila), (Illa) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 6 carbon atoms;
   R¹⁰ represents a straight or branched chain alkyl group having from 1 to 6 carbon atoms;
   R¹¹ represents a hydrogen atom or a straight or branched chain alkyl group having from 1 to 3 carbon atoms; and
   R¹³ represents a phenyl group, a straight or branched chain alkyl group having from 1 to 6 carbon atoms or a cycloalkyl group having from 5 to 7 carbon atoms.
(54) R¹ and R² are the same or different and each represents a hydrogen atom or a hydroxy group; and R³ and R⁴ togetherform a single bond;,
   one of R⁵ and R⁶ represents a group of formula (Ila), (Illa) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 6 carbon atoms;
   R¹⁰ represents a straight or branched chain alkyl group having from 1 to 6 carbon atoms;
   R¹¹ represents a hydrogen atom or a straight or branched chain alkyl group having from 1 to 3 carbon atoms; and
   R¹³ represents a straight or branched chain alkyl group having from 1 to 6 carbon atoms.
(55) R¹ and R² are the same or different and each represents a hydrogen atom or a hydroxy group; and R³ and R⁴ together form a single bond;
   one of R⁵ and R⁶ represents a group of formula (Ila), (Illa) or (IVa), as defined in (4), above, in which:
   _{R}7 represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 6 carbon atoms;
   R¹⁰ represents a straight or branched chain alkyl group having from 1 to 6 carbon atoms;
   R¹¹ represents a hydrogen atom or a straight or branched chain alkyl group having from 1 to 3 carbon atoms; and
   R¹³ represents a straight or branched chain alkyl group having from 1 to 3 carbon atoms.
(56) R¹ and R² are the same or different and each represents a hydrogen atom or a hydroxy group; and R³ and R⁴ together form a single bond;
   one of R⁵ and R⁶ represents a group of formula (Ila), (Illa) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 3 carbon atoms;
   R¹⁰ represents a straight or branched chain alkyl group having from 1 to 6 carbon atoms;
   R¹¹ represents a hydrogen atom or a straight or branched chain alkyl group having from 1 to 6 carbon atoms; and
   R¹³ represents a phenyl group, a straight or branched chain alkyl group having from 1 to 6 carbon atoms or a cycloalkyl group having from 5 to 7 carbon atoms.
(57) R¹ and R² are the same or different and each represents a hydrogen atom or a hydroxy group; and R³ and R⁴ together form a single bond;
   one of R⁵ and R⁶ represents a group of formula (Ila), (Illa) or (IVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 3 carbon atoms;
   R¹⁰ represents a straight or branched chain alkyl group having from 1 to 6 carbon atoms;
   R¹¹ represents a hydrogen atom or a straight or branched chain alkyl group having from 1 to 6 carbon atoms; and
   R¹³ represents a straight or branched chain alkyl group having from 1 to 6 carbon atoms.
(58) R¹ and R² are the same or different and each represents a hydrogen atom or a hydroxy group; and R³ and R⁴ together form a single bond;
   one of R⁵ and R⁶ represents a group of formula (Ila), (Illa) or (lVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 3 carbon atoms;
   R¹⁰ represents a straight or branched chain alkyl group having from 1 to 6 carbon atoms;
   R¹¹ represents a hydrogen atom or a straight or branched chain alkyl group having from 1 to 6 carbon atoms; and
   R¹³ represents a straight or branched chain alkyl group having from 1 to 3 carbon atoms.
(59) R¹ and R² are the same or different and each represents a hydrogen atom or a hydroxy group; and R3 and R⁴ together form a single bond;
   one of R⁵ and R⁶ represents a group of formula (Ila), (Illa) or (lVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 3 carbon atoms;
   R¹⁰ represents a straight or branched chain alkyl group having from 1 to 6 carbon atoms;
   R¹¹ represents a hydrogen atom or a straight or branched chain alkyl group having from 1 to 3 carbon atoms; and
   R¹³ represents a phenyl group, a straight or branched chain alkyl group having from 1 to 6 carbon atoms or a cycloalkyl group having from 5 to 7 carbon atoms.
(60) R¹ and R² are the same or different and each represents a hydrogen atom or a hydroxy group; and R3 and R⁴ together form a single bond;
   one of R⁵ and R⁶ represents a group of formula (Ila), (Illa) or (lVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 3 carbon atoms;
   R¹⁰ represents a straight or branched chain alkyl group having from 1 to 6 carbon atoms;
   R¹¹ represents a hydrogen atom or a straight or branched chain alkyl group having from 1 to 3 carbon atoms; and
   R¹³ represents a straight or branched chain alkyl group having from 1 to 6 carbon atoms.
(61) R¹ and R² are the same or different and each represents a hydrogen atom or a hydroxy group; and R³ and R⁴ together form a single bond;
   one of R⁵ and R⁶ represents a group of formula (Ila), (Illa) or (lVa), as defined in (4), above, in which:
   R⁷ represents a straight or branched chain alkyl group having from 1 to 6 carbon atoms or a cycloalkyl group having from 5 to 7 carbon atoms;
   R⁸ represents a hydrogen atom or a straight or branched chain alkyl group having from 1 to 3 carbon atoms;
   R¹⁰ represents a straight or branched chain alkyl group having from 1 to 6 carbon atoms;
   R¹¹ represents a hydrogen atom or a straight or branched chain alkyl group having from 1 to 3 carbon atoms; and
   R¹³ represents a straight or branched chain alkyl group having from 1 to 3 carbon atoms.
(62) R⁵ represents a hydrogen atom.
(63) R⁶ represents a hydrogen atom.
(64) one of R⁵ and R⁶ represents a group of formula (Va): 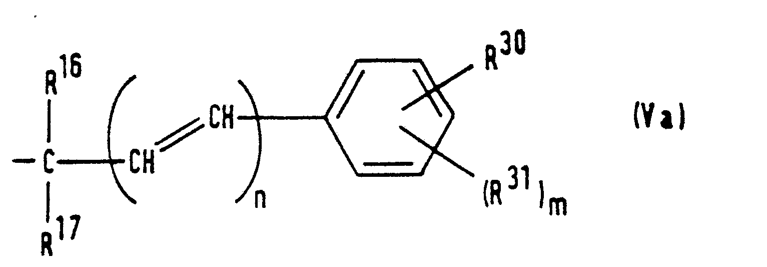 in which: Ris, R¹⁷, _{R}30, R31, m and n are as defined above.
(65) one of R⁵ and R⁶ represents a group of formula (Va), as defined in (64) above, in which R¹⁶ represents a hydrogen atom or a C₁- C₆ alkyl group.
(66) one of R⁵ and R⁶ represents a group of formula (Va), as defined in (64) above, in which R¹⁷ represents a hydrogen atom or a methyl group.
(67) one of R⁵ and R⁶ represents a group of formula (Va), as defined in (64) above, in which R³⁰ represents an aliphatic carboxylic acyloxy group containing from 1 to 18 carbon atoms, said group being unsubstituted or having at least one halogen substituent, or an aromatic acyloxy group which is unsubstituted or has at least one of substituents (a).
(68) one of R⁵ and R⁶ represents a group of formula (Va), as defined in (64) above, in which R³⁰ represents an aliphatic carboxylic acyloxy group containing from 1 to 6 carbon atoms, said group being unsubstituted or having at least one halogen substituent.
(69) one of R⁵ and R⁶ represents a group of formula (Va), as defined in (64) above, in which R³¹ represents an aliphatic carboxylic acyloxy group containing from 1 to 18 carbon atoms, an alkyl group containing from 1 to 6 carbon atoms, an alkoxy group containing from 1 to 6 carbon atoms or a halogen atom.
(70) one of R⁵ and R⁶ represents a group of formula (Va), as defined in (64) above, in which m is 0, 1 or 2.
(71) one of R⁵ and R⁶ represents a group of formula (Va), as defined in (64) above, in which n is 0 or 1.
(72) one of R⁵ and R⁶ represents a hydrogen atom; and the other of R⁵ and R⁶ represents a group of formula (Va), as defined in (64) above, in which:
   R¹⁶ represents a hydrogen atom or a lower alkyl group,
   R¹⁷ represents a hydrogen atom,
   R³⁰ represents an aliphatic carboxylic acyloxy group containing from 1 to 18 carbon atoms, said group being unsubstituted or having at least one halogen substituent, or an aromatic acyloxy group which is unsubstituted or has at least one of substituents (a),
   R³¹ represents an aliphatic carboxylic acyloxy group containing from 1 to 18 carbon atoms, an alkyl group containing from 1 to 6 carbon atoms, an alkoxy group containing from 1 to 6 carbon atoms or a halogen atom,
   m is 0, 1 or 2, and
   n is 0 or 1.
(73) one of R⁵ and R⁶ represents a hydrogen atom; and the other of R⁵ and R⁶ represents a group of formula (Va), as defined in (64) above, in which:
   R¹⁶ represents a hydrogen atom or a C₁- Cs alkyl group,
   R¹⁷ represents a hydrogen atom or a methyl group,
   _{R}30 represents an aliphatic carboxylic acyloxy group containing from 1 to 6 carbon atoms, said group being unsubstituted or having at least one halogen substituent,
   R³1 represents an aliphatic carboxylic acyloxy group containing from 1 to 6 carbon atoms, an alkyl group containing from 1 to 6 carbon atoms, an alkoxy group containing from 1 to 6 carbon atoms or a halogen atom,
   m is 0, 1 or 2, and
   nis0or1.
(74) one of R⁵ and R⁶ represents a hydrogen atom; and the other of R⁵ and R⁶ represents a group of formula (Va), as defined in (64). above, in which:
   R¹⁶ and R¹⁷ each represents a hydrogen atom,
   R³⁰ represents an aliphatic carboxylic acyloxy group containing from 1 to 6 carbon atoms, said group being unsubstituted or having at least one halogen substituent,
   m is 0, and
   n is 0 or 1.
(75) one of R⁵ and R⁶ represents a hydrogen atom; and the other of R⁵ and R⁶ represents a group of formula (Va), as defined in (64) above, in which:
   R¹⁶ and R¹⁷ each represents a hydrogen atom,
   R³⁰ and R³¹ each represents an aliphatic carboxylic acyloxy group containing from 1 to 6 carbon atoms, said group being unsubstituted or having at least one halogen substituent,
   m is 1, and
   n is 0 or 1.
(76) one of R⁵ and R⁶ represents a hydrogen atom; and the other of R⁵ and R⁶ represents a group of formula (Va), as defined in (64) above, in which:
   R¹⁶ and R¹⁷ each represents a hydrogen atom,
   R³⁰ represents an aliphatic carboxylic acyloxy group containing from 1 to 6 carbon atoms, said group being unsubstituted or having at least one halogen substituent,
   R³¹ represents an alkyl group containing from 1 to 6 carbon atoms an alkoxy group containing from 1 to 6 carbon atoms,
   m is 1 or 2, and
   n is 0 or 1.
(77) compounds as defined in (62) to (76) above, in which:
   R¹ and R² are the same or different and each represents a hydrogen atom, a hydroxy group, a Ci - C₁₈ aliphatic carboxylic acyloxy group or an aromatic carboxylic acyloxy groups.
(78) compounds as defined in (62) to (76) above, in which:
   R¹ and R² are the same or different and each represents a hydrogen atom or a hydroxy group.
(79) compounds as defined in (62) to (76) above, in which:
   R³ and R⁴ together form a single bond.
(80) compounds as defined in (62) to (76)'above, in which:
   R³ and R⁴ each represents a hydrogen atom.
(81) compounds as defined in (62) to (76) above, in which:
   R¹ and R² are the same or different and each represents a hydrogen atom, a hydroxy group, a Ci - C₁₈ aliphatic carboxylic acyloxy group or an aromatic carboxylic acyloxy groups; and
   R³ and R⁴ together form a single bond.
(82) compounds as defined in (62) to (76) above, in which:
   R and R² are the same or different and each represents a hydrogen atom or a hydroxy group; and
   R3 and R⁴ together form a single bond.
(83) compounds as defined in (62) to (76) above, in which:
   R¹ and R² are the same or different and each represents a hydrogen atom, a hydroxy group, a C₁-C₁₈ aliphatic carboxylic acyloxy group or an aromatic carboxylic acyloxy groups; and
   R³ and R4 each represents a hydrogen atom.
(84) compounds as defined in (62) to (76) above, in which:
   R¹ and R² are the same or different and each represents a hydrogen atom or a hydroxy group; and
   R³ and R⁴ each represents a hydrogen atom.

The compounds of the present invention necessarily have at least one free carboxy group and, depending on the nature of the substituents, may have more than one free carboxy group. These carboxy groups can, of course, form salts and such salts also form part of the present invention. There is no limitation upon the nature of such salts, provided that, where they are to be used for therapeutic purposes, they are pharmaceutically acceptable, which, as is well-known in the art, means that they do not have reduced activity (or unacceptably reduced activity) or increased toxicity (or unacceptably increased toxicity) compared with the free compound of formula (I). Where, however, they are to be used for non-therapeutic purposes, e.g. as intermediates in the preparation of other compounds, even this limitation does not apply. The compounds also contain basic nitrogen atoms, and these likewise can form salts. Examples of suitable salts include: salts with an alkali or alkaline earth metal such as sodium, potassium or calcium; salts with an organic base such as methylamine, ethylamine, morpholine or piperidine; salts with an inorganic acid, such as a hydrogen halide (e.g. hydrofluoric acid, hydrochloric acid, hydrobromic acid or hydroiodic acid), or nitric acid, perchloric acid, sulphuric acid or phosphoric acid; salts with an organic acid, such as a lower alkylsulphonic acid (e.g. methanesulphonic acid, trifluoromethanesulphonic acid or ethanesulphonic acid), an arylsulphonic acid (e.g. benzenesulphonic acid or p-toluenesulphonic acid), or fumaric acid, succinic acid, citric acid, tartaric acid, oxalic acid or maleic acid; and salts with an amino acid such as glutamic acid or aspartic acid.

Because of the presence of asymmetric carbon atoms in the compounds of formula (I) of the present invention, the compounds can exist as various stereoisomers in either the S-configuration or the R-configuration at each of the asymmetric carbon atoms. Although these are all shown by a single formula herein, the present invention embraces both the individual isomers and mixtures thereof. The individual isomers may, in appropriate cases, be prepared by stereospecific synthesis techniques, or a mixture of isomers may be prepared and then separated by techniques for separating isomers well known to those skilled in the art.

Examples of specific compounds of the invention are given in the following formulae (1-1) to (1-12), in which the substituents are as defined in the corresponding one of Tables 1 to 12 [i.e. Table 1 relates to formula (1-1), Table 2 relates to formula (1-2) and so on]. In the Tables, the following abbreviations are used:

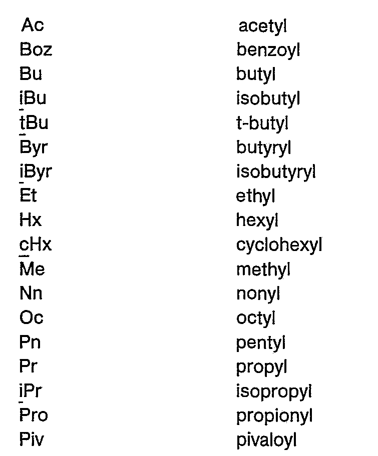

In all of formulae (I-1) to (1-12), the symbol A represents the group of formula (B):

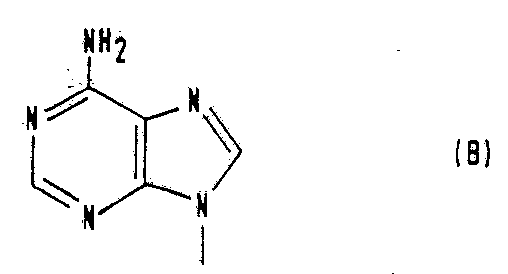

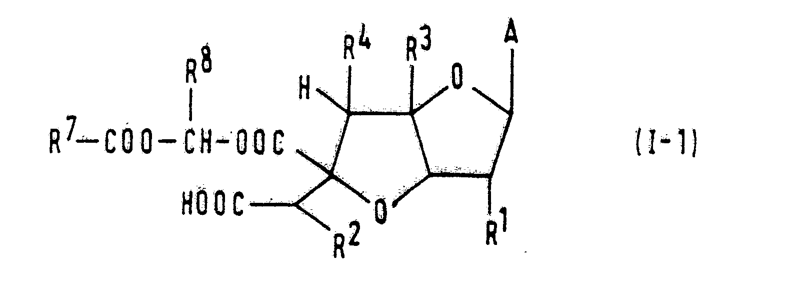

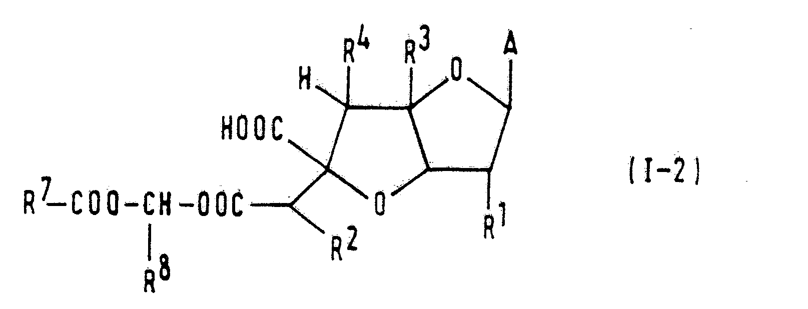

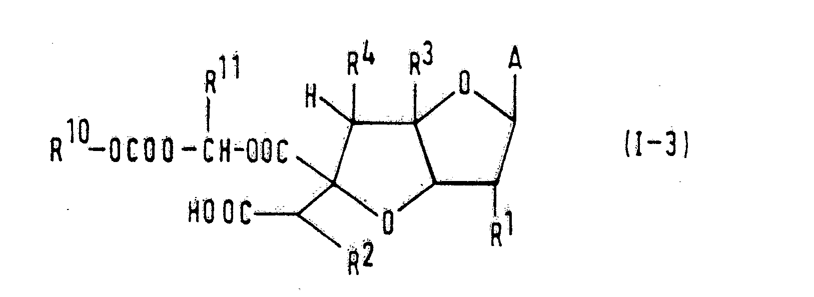

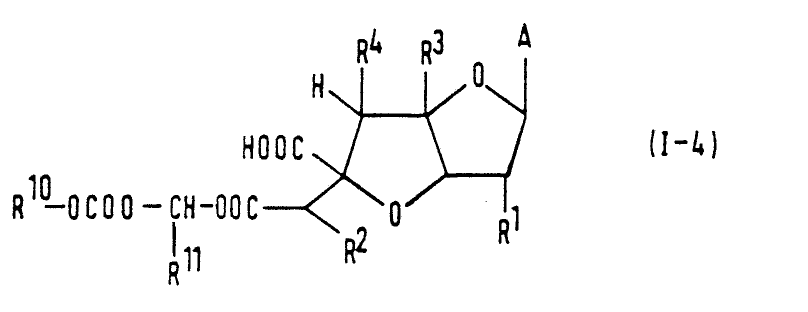

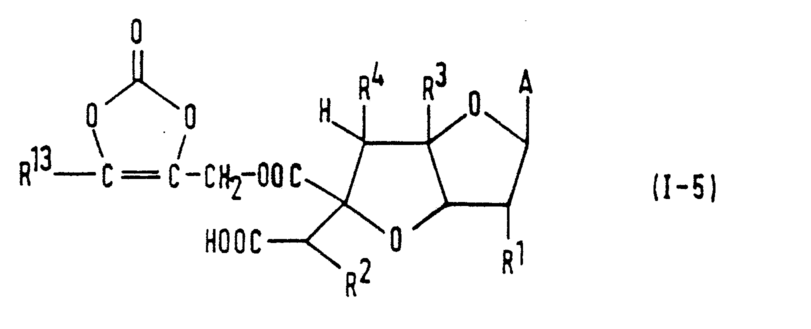

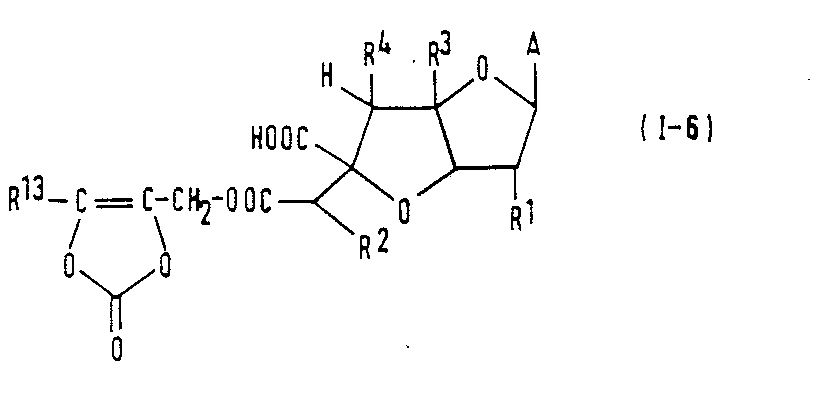

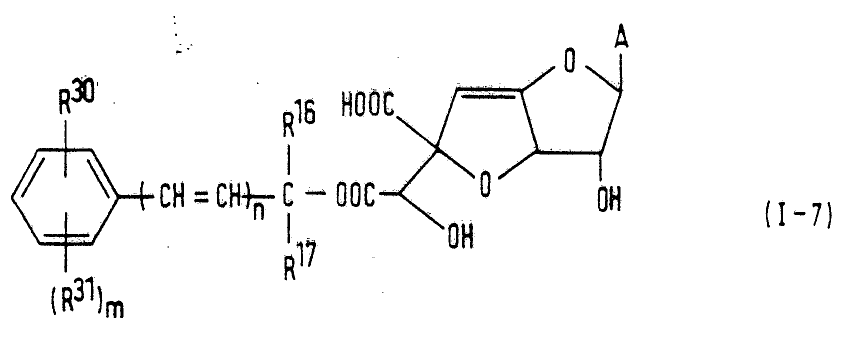

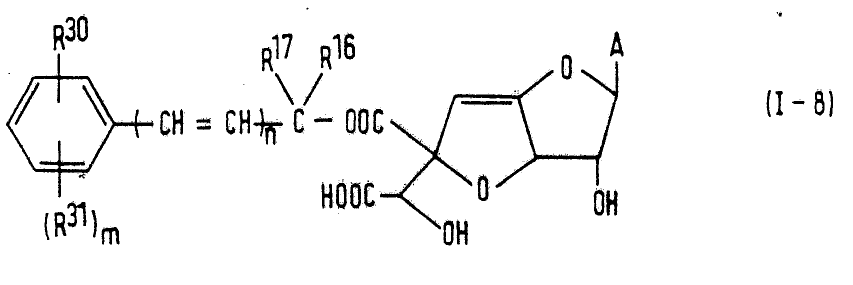

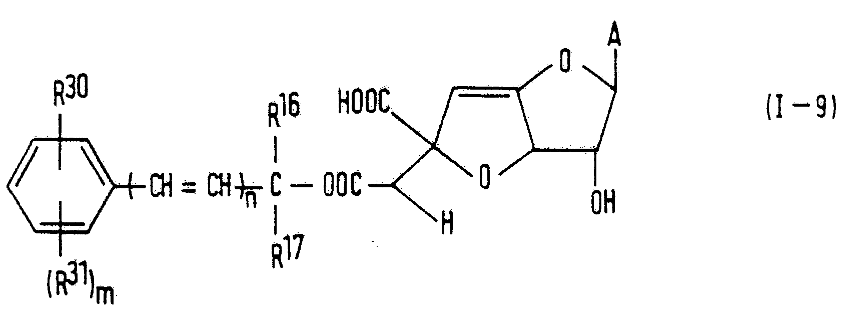

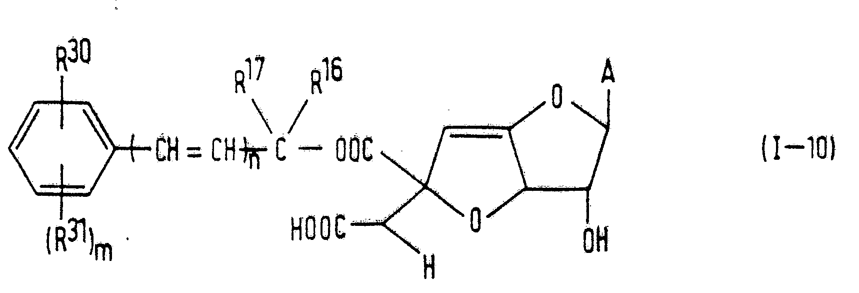

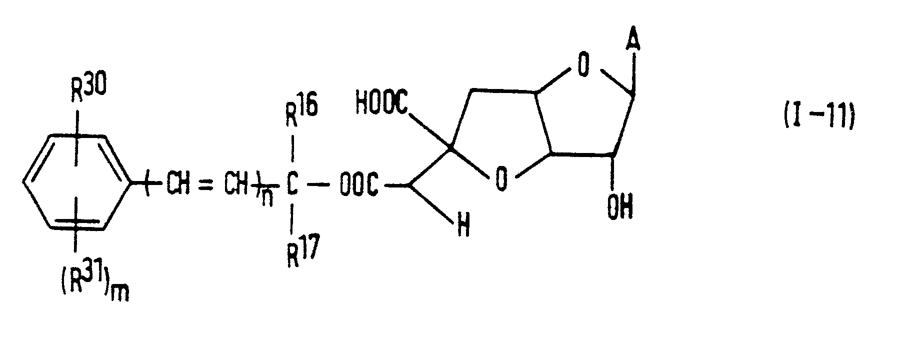

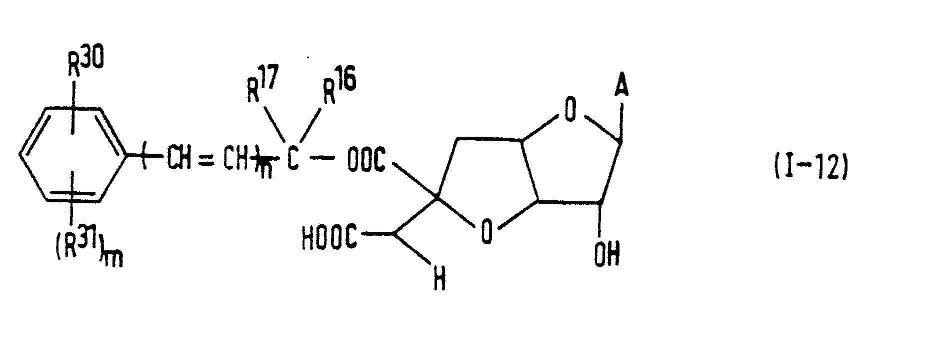

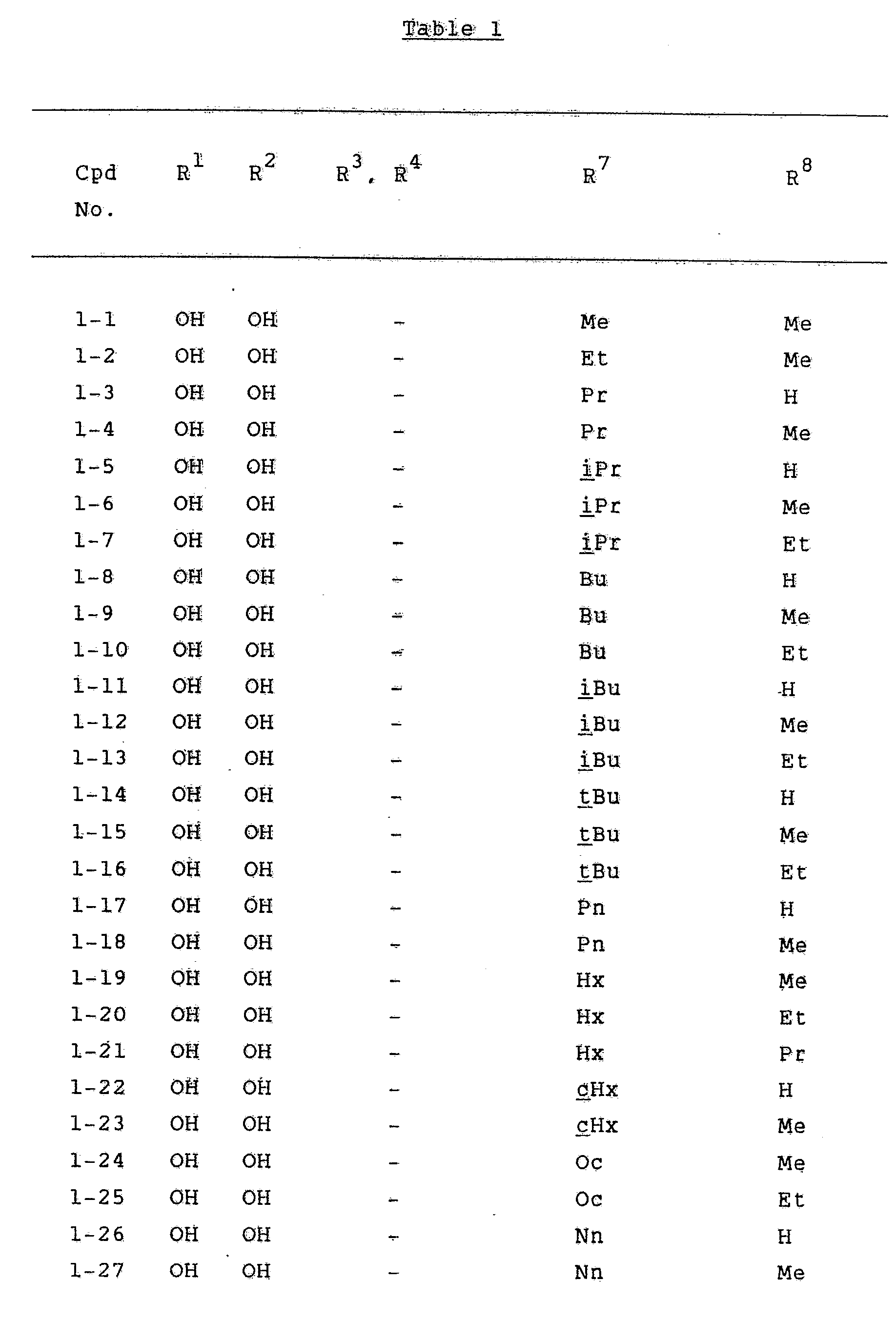

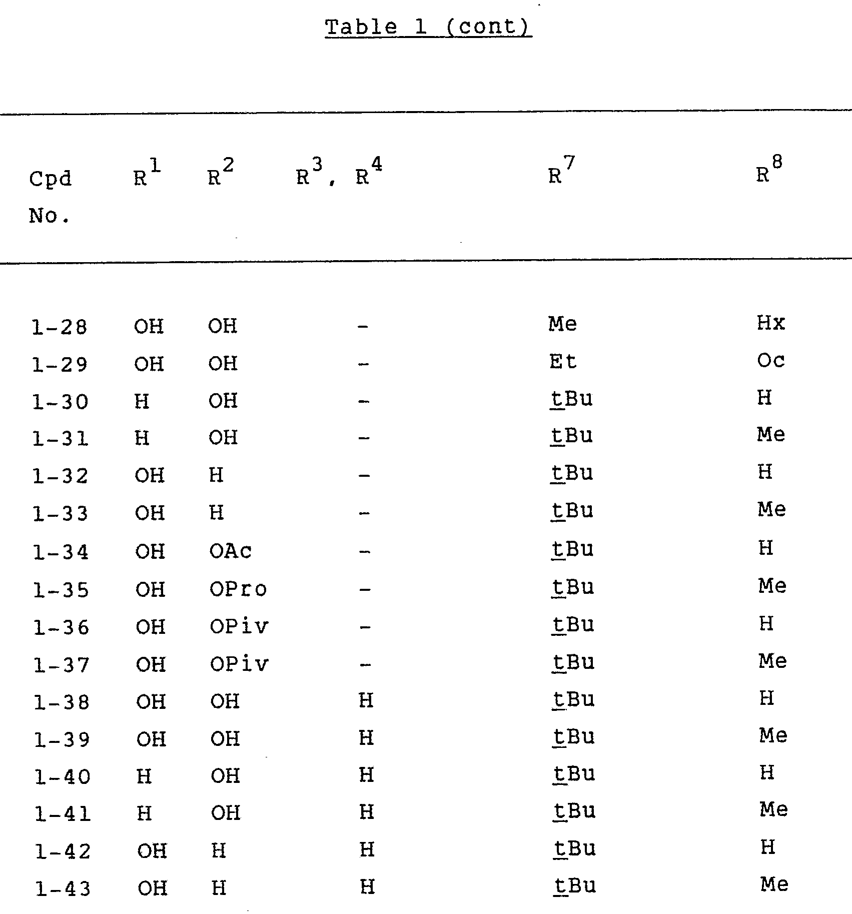

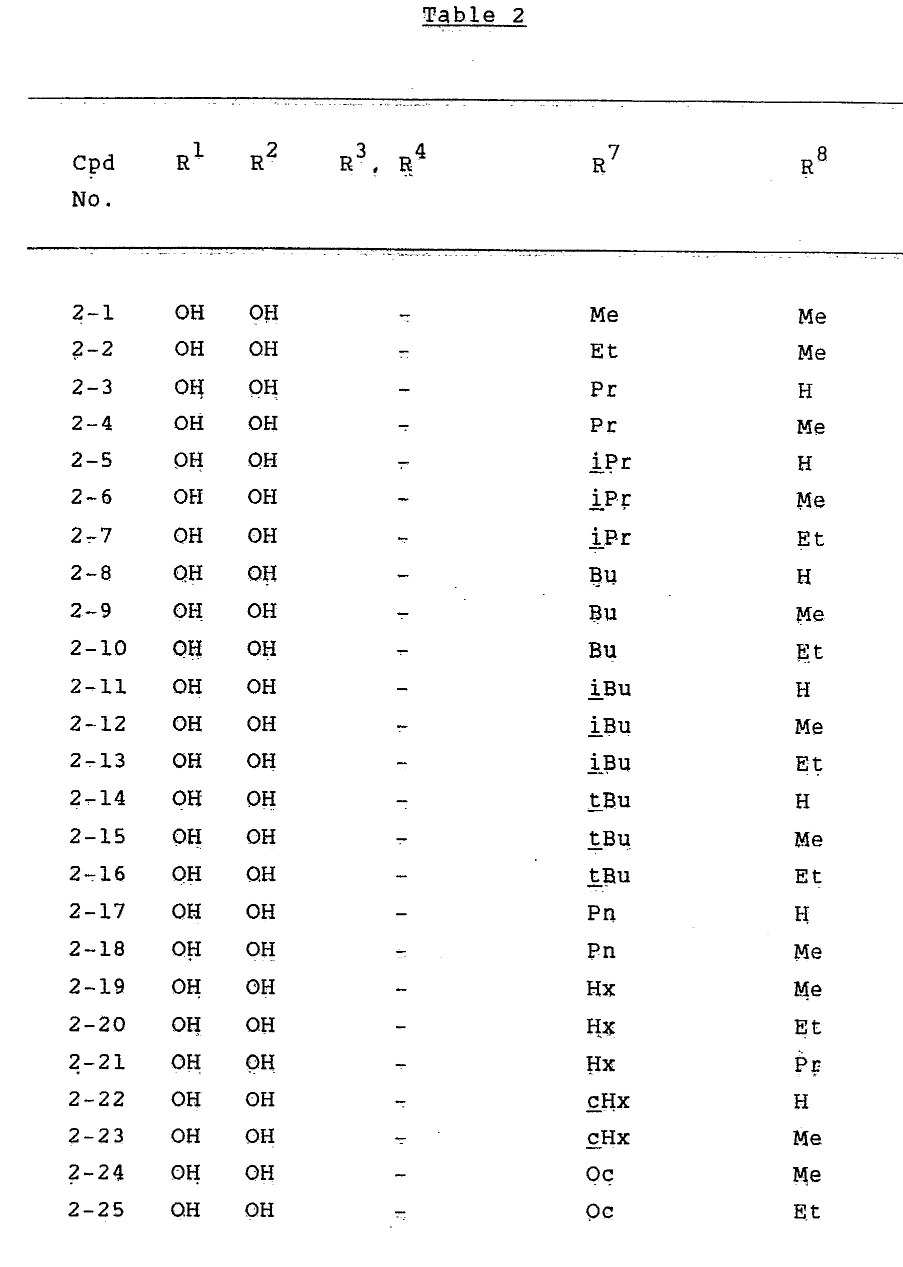

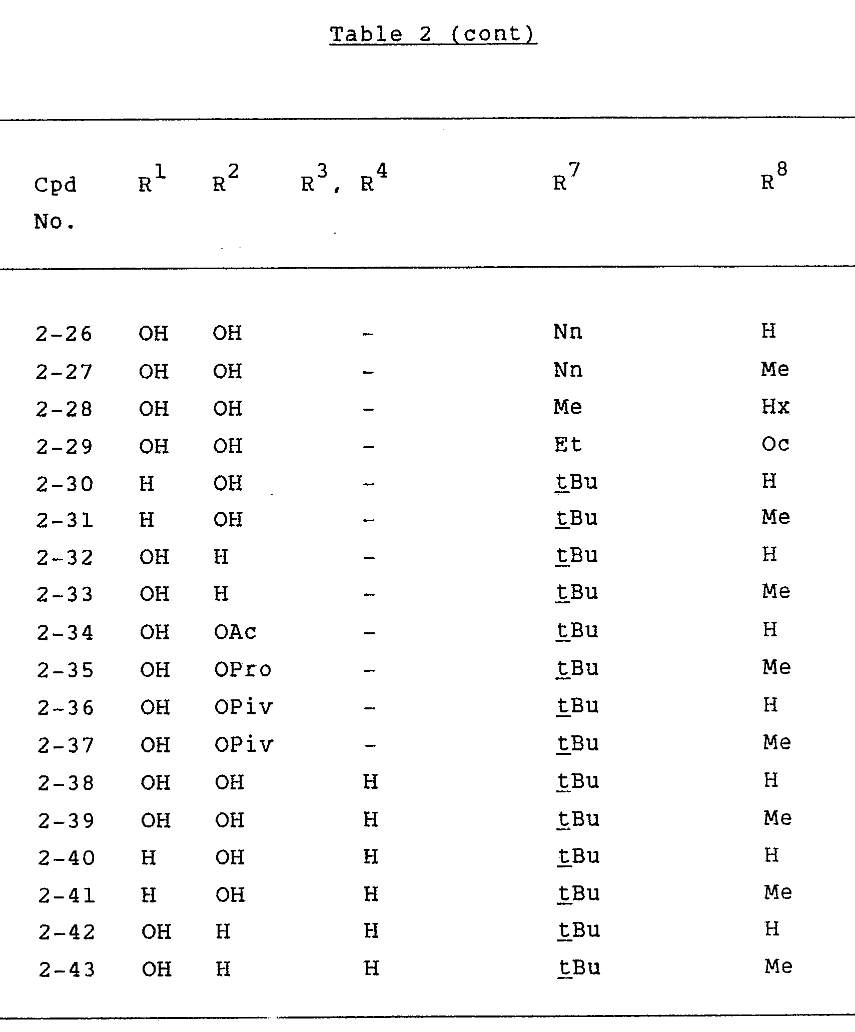

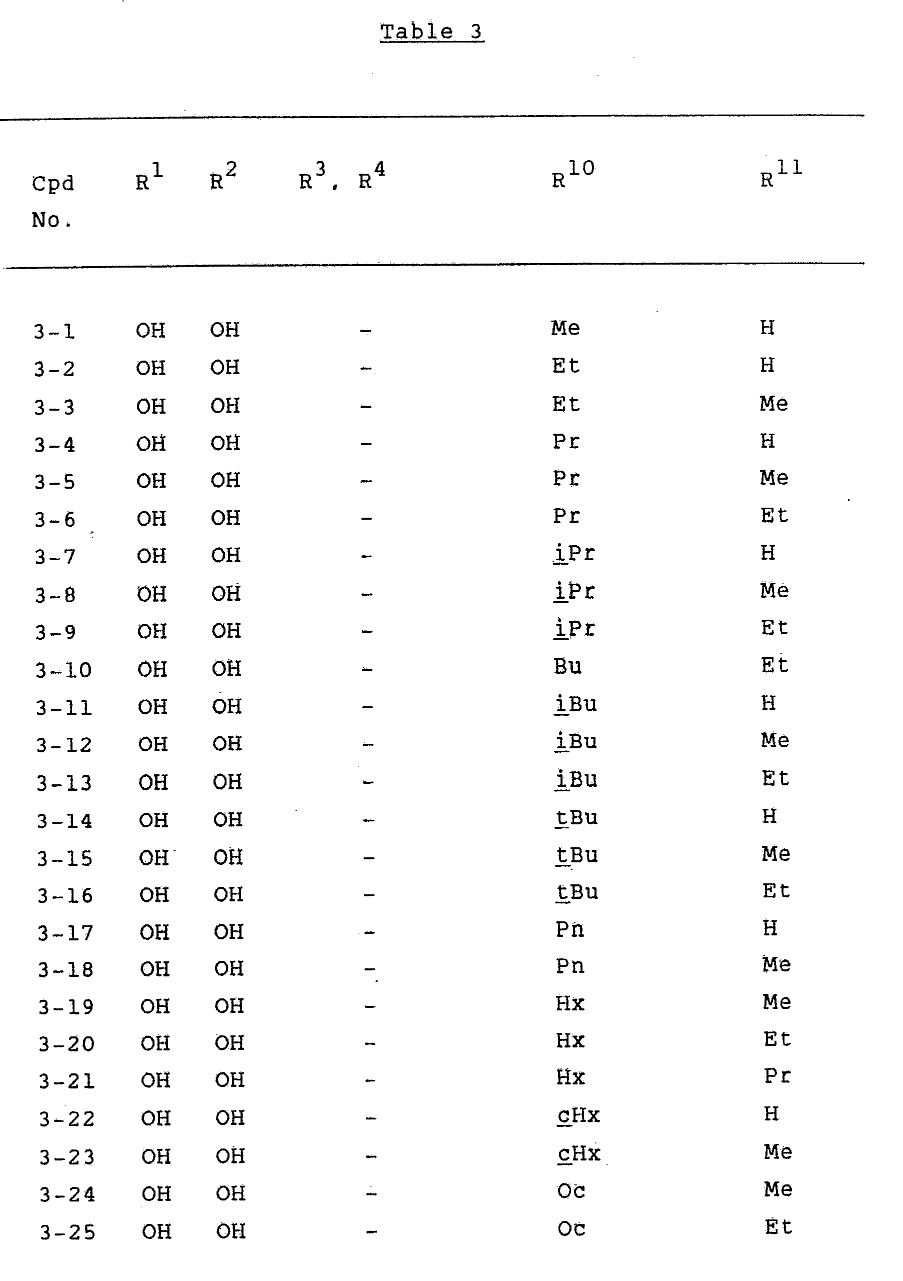

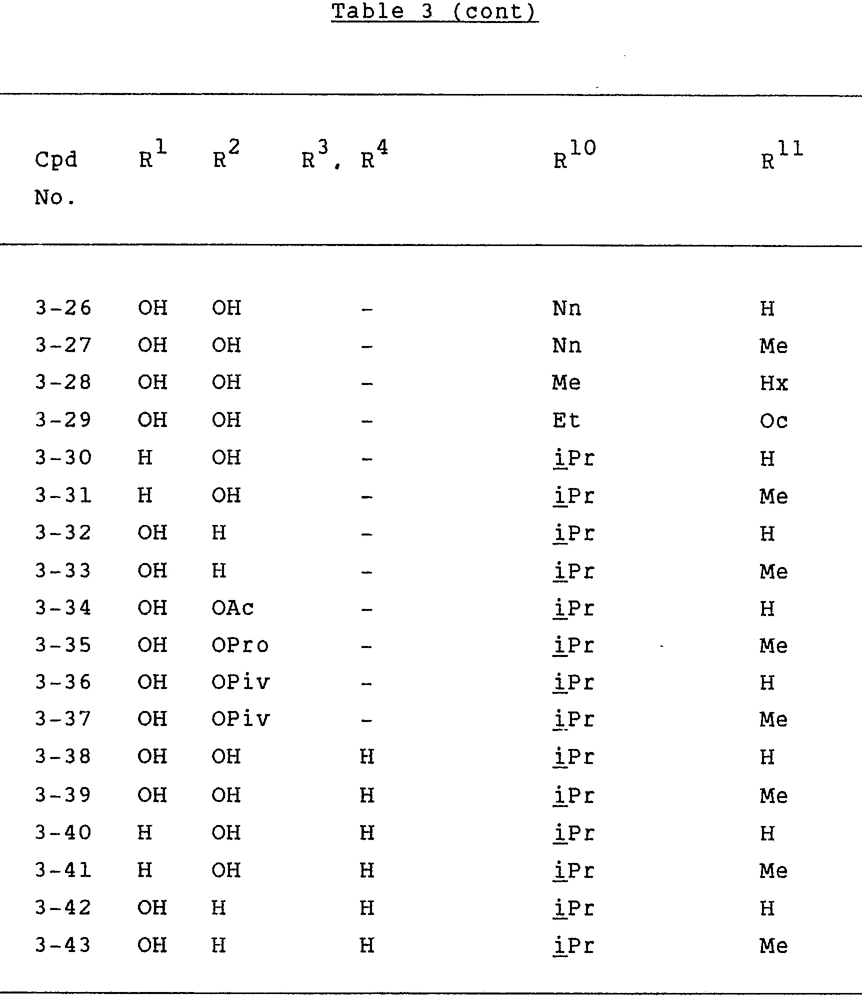

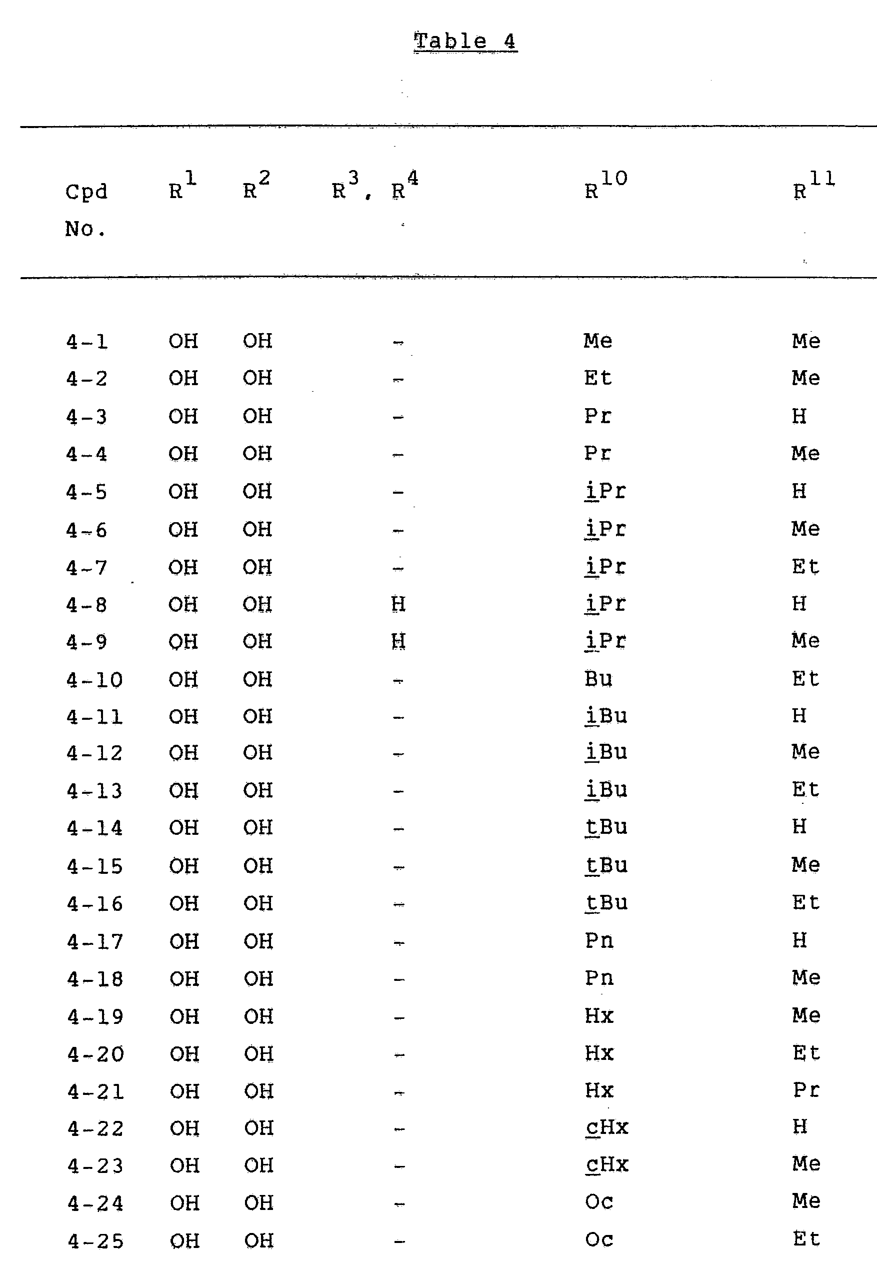

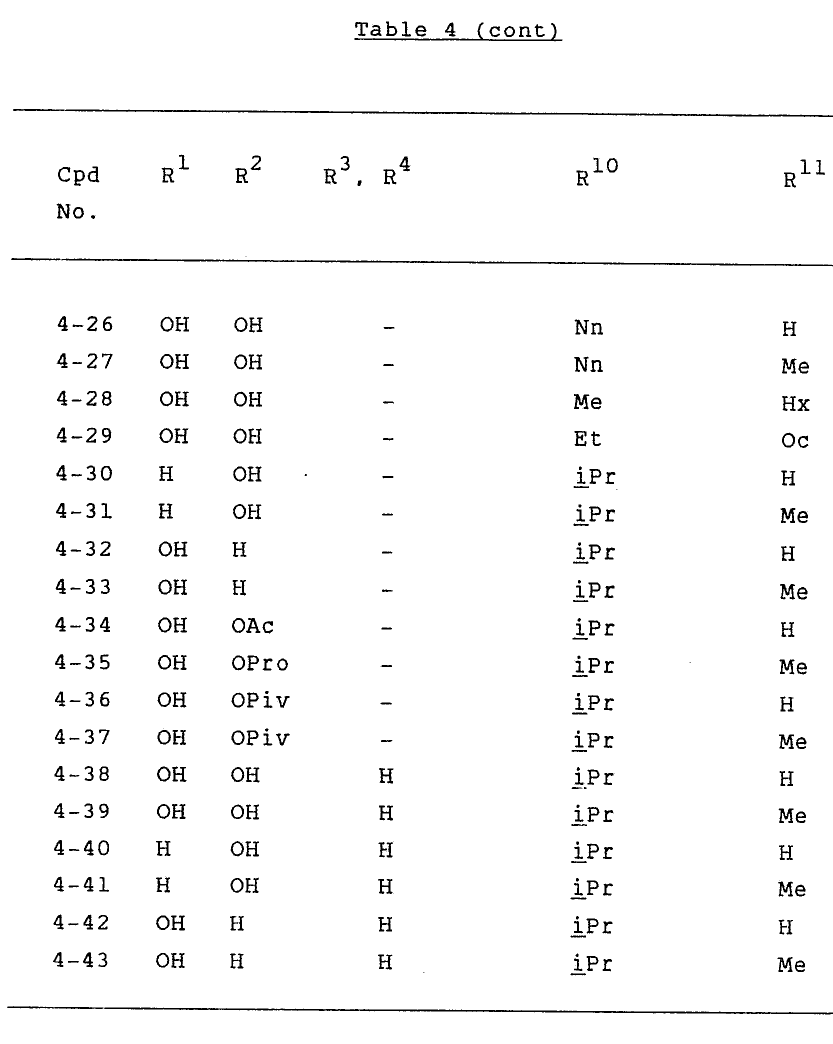

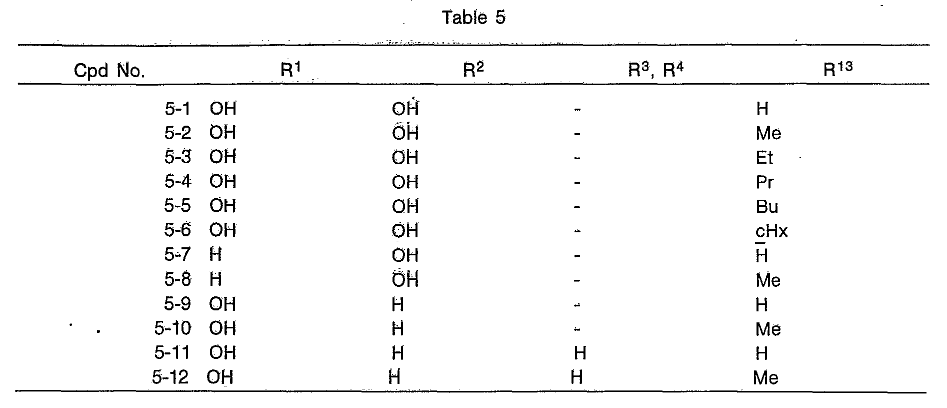

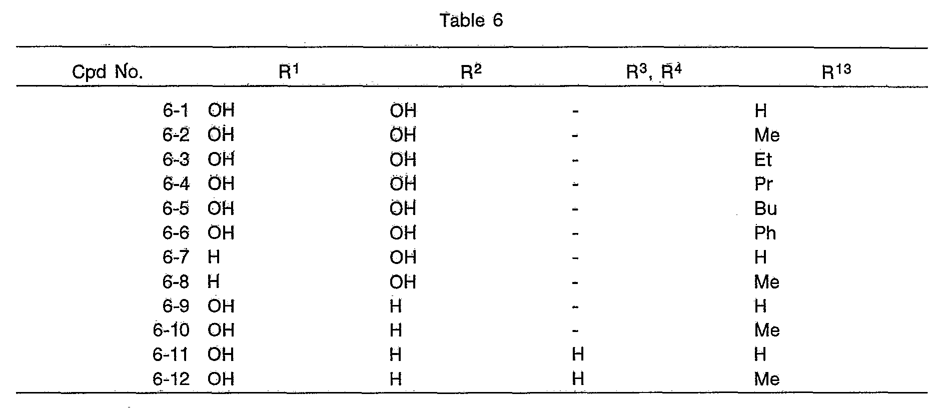

In the following Tables, where R³¹ is shown as "H", this means that m in the relevant one of formulae (I-7) to (I-12) is 0.

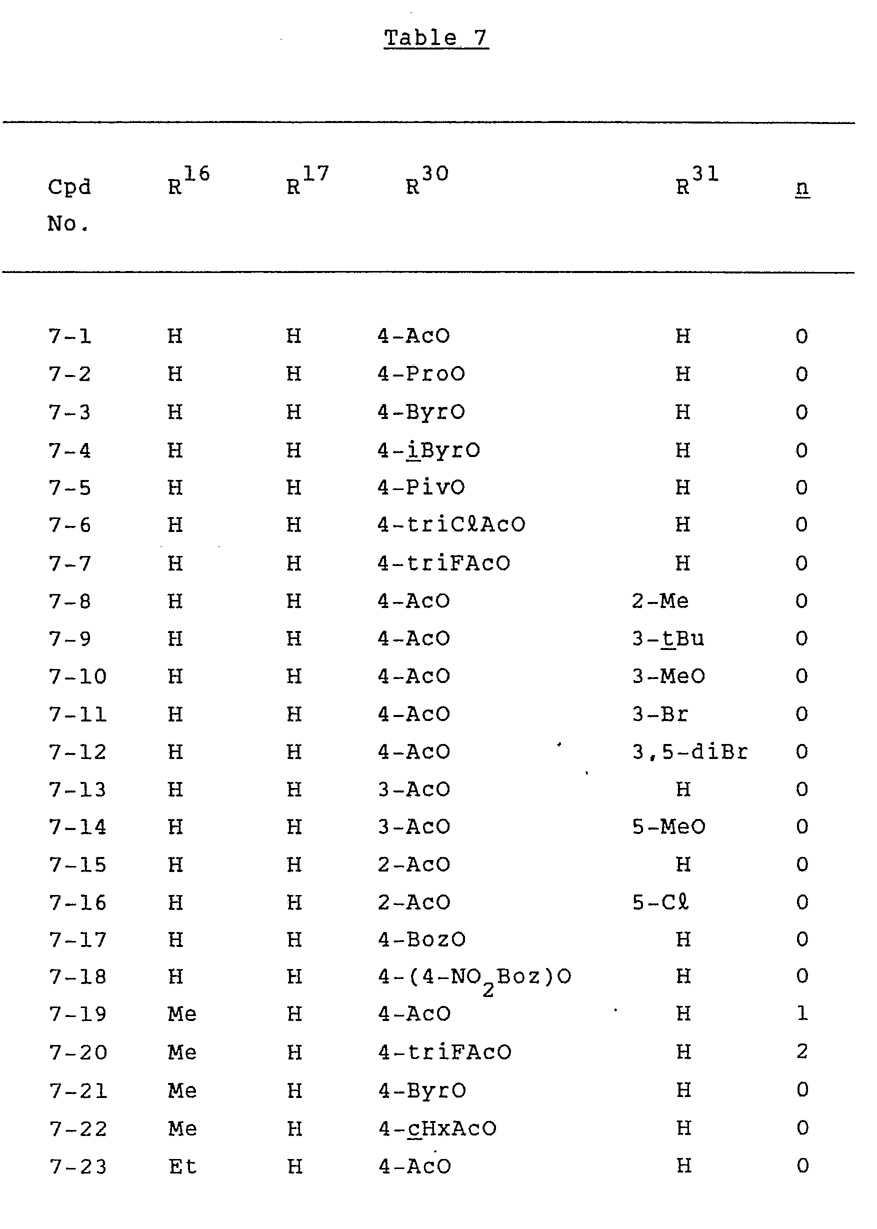

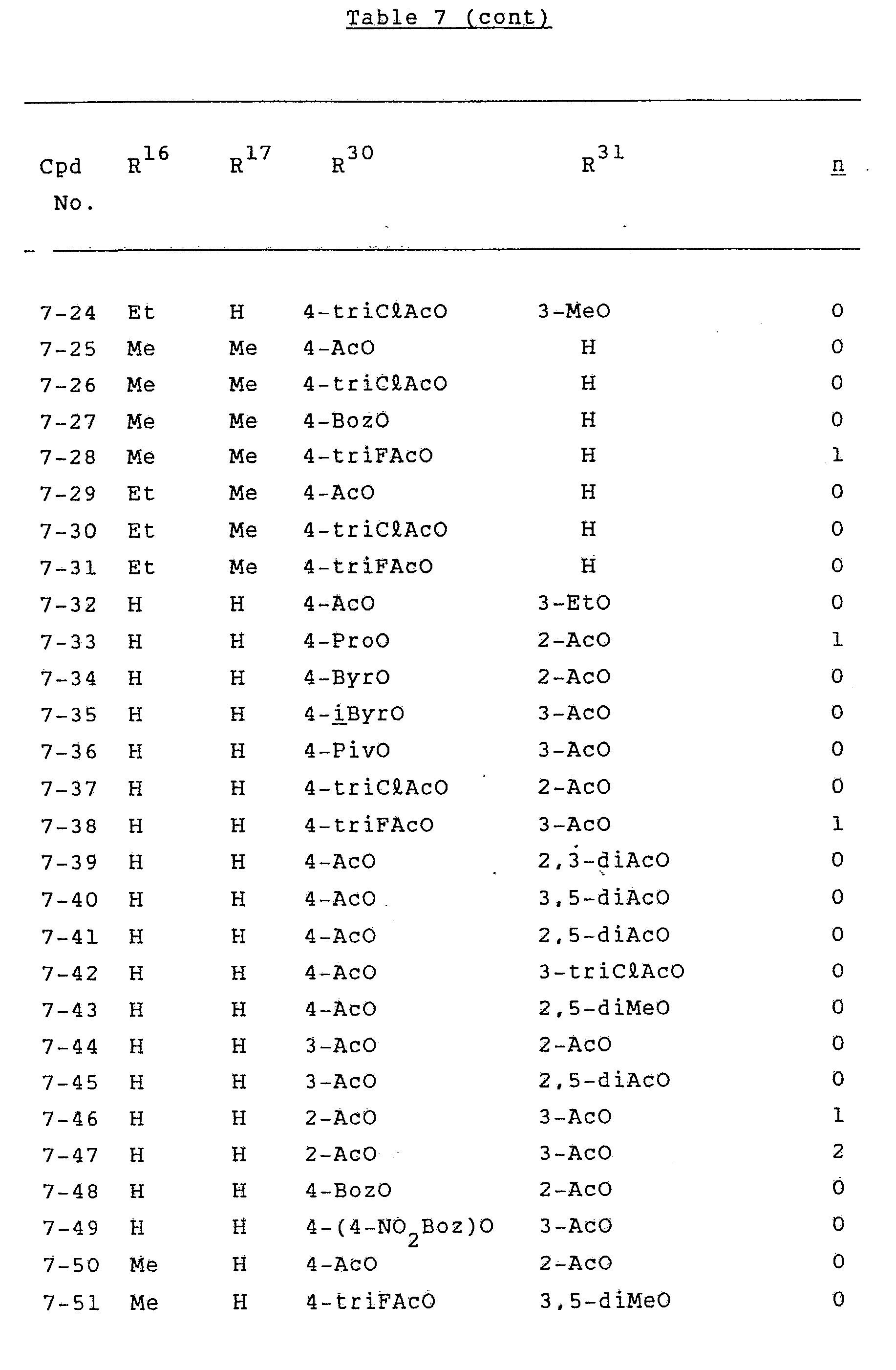

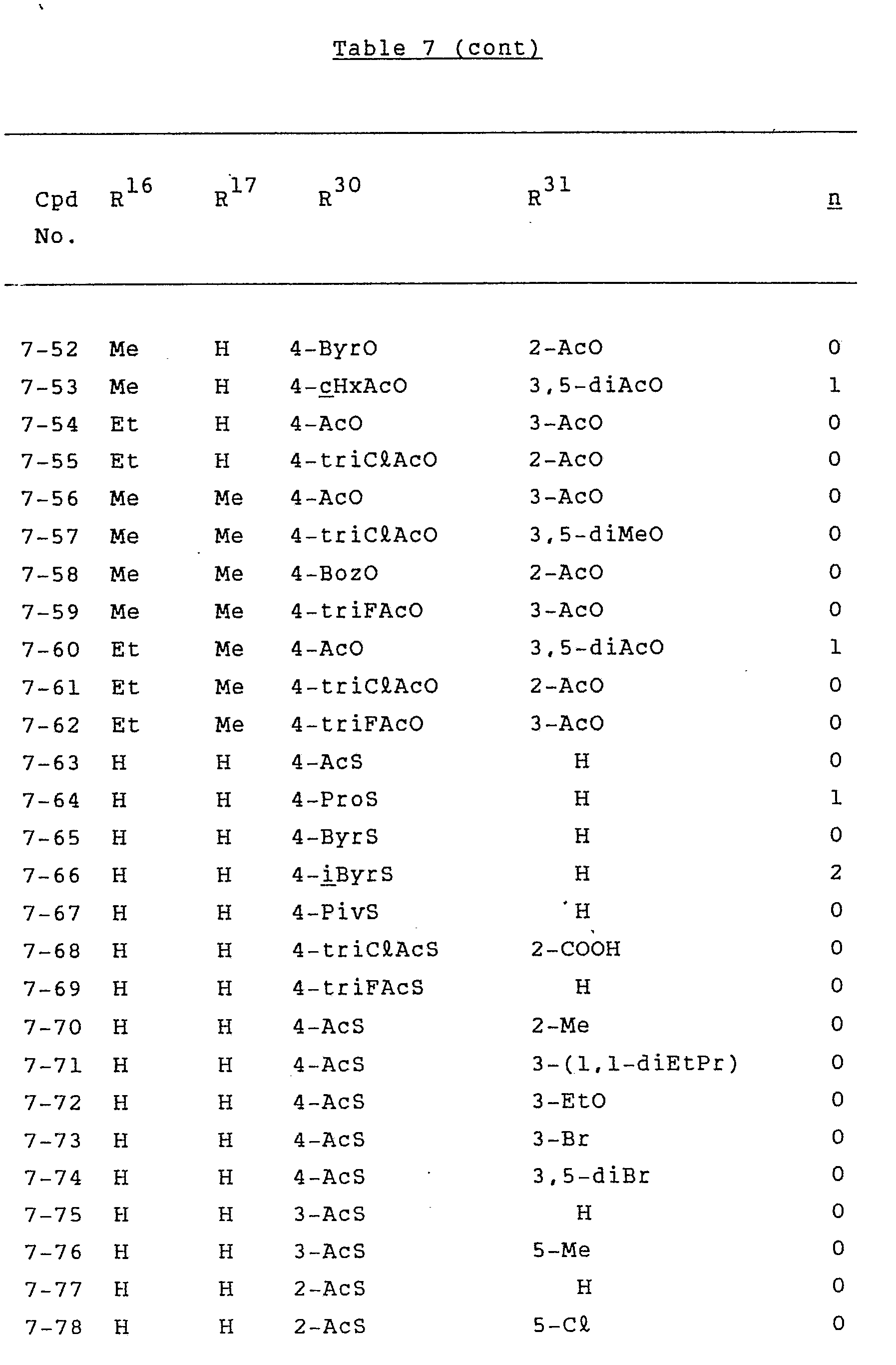

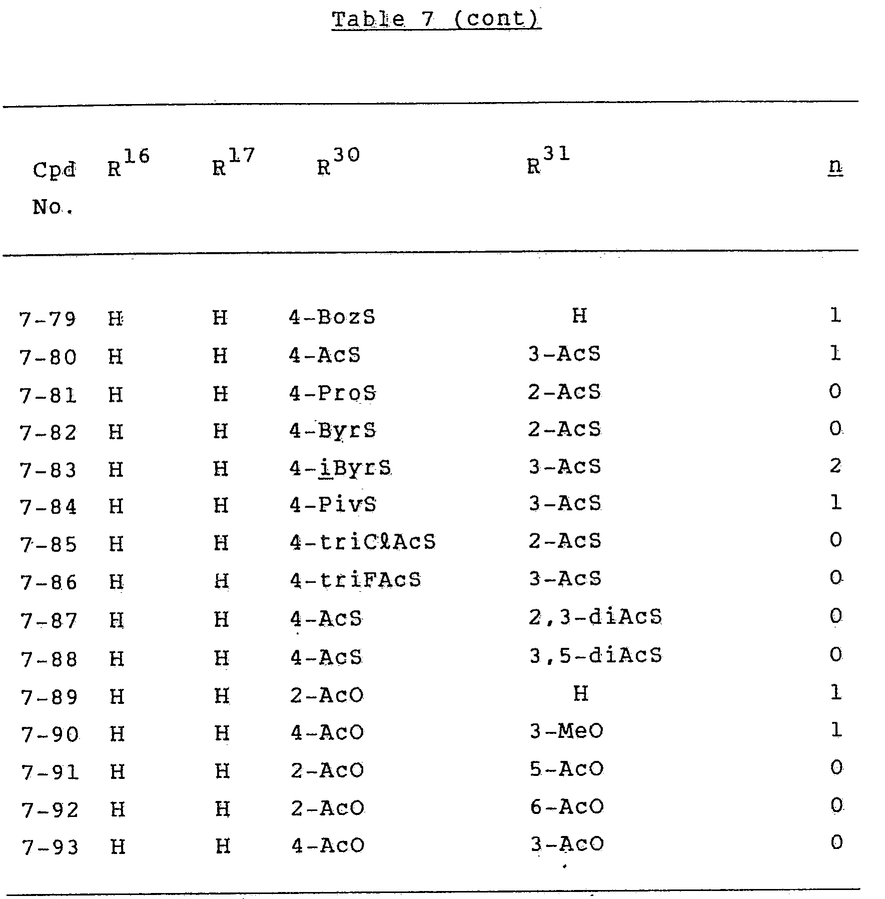

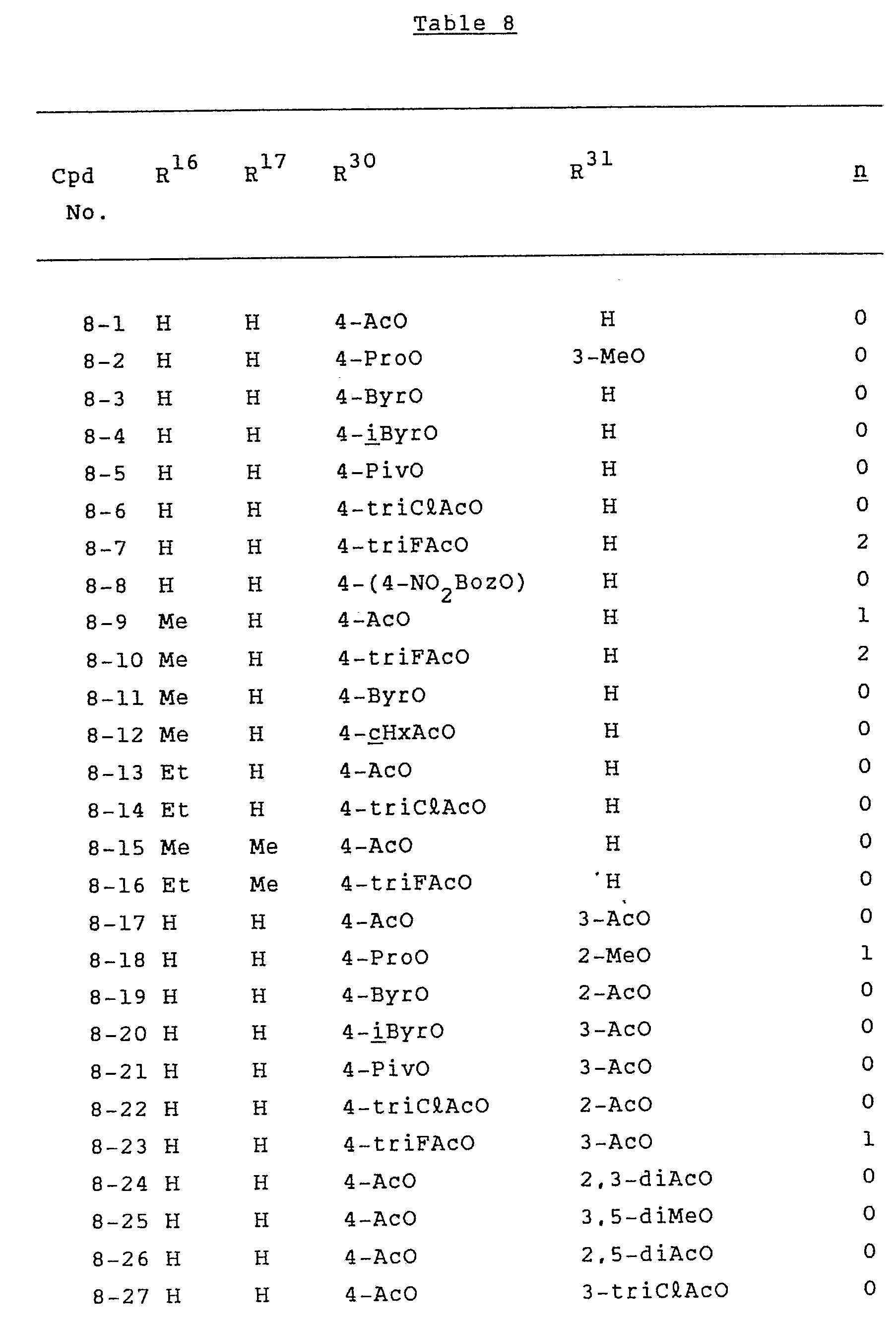

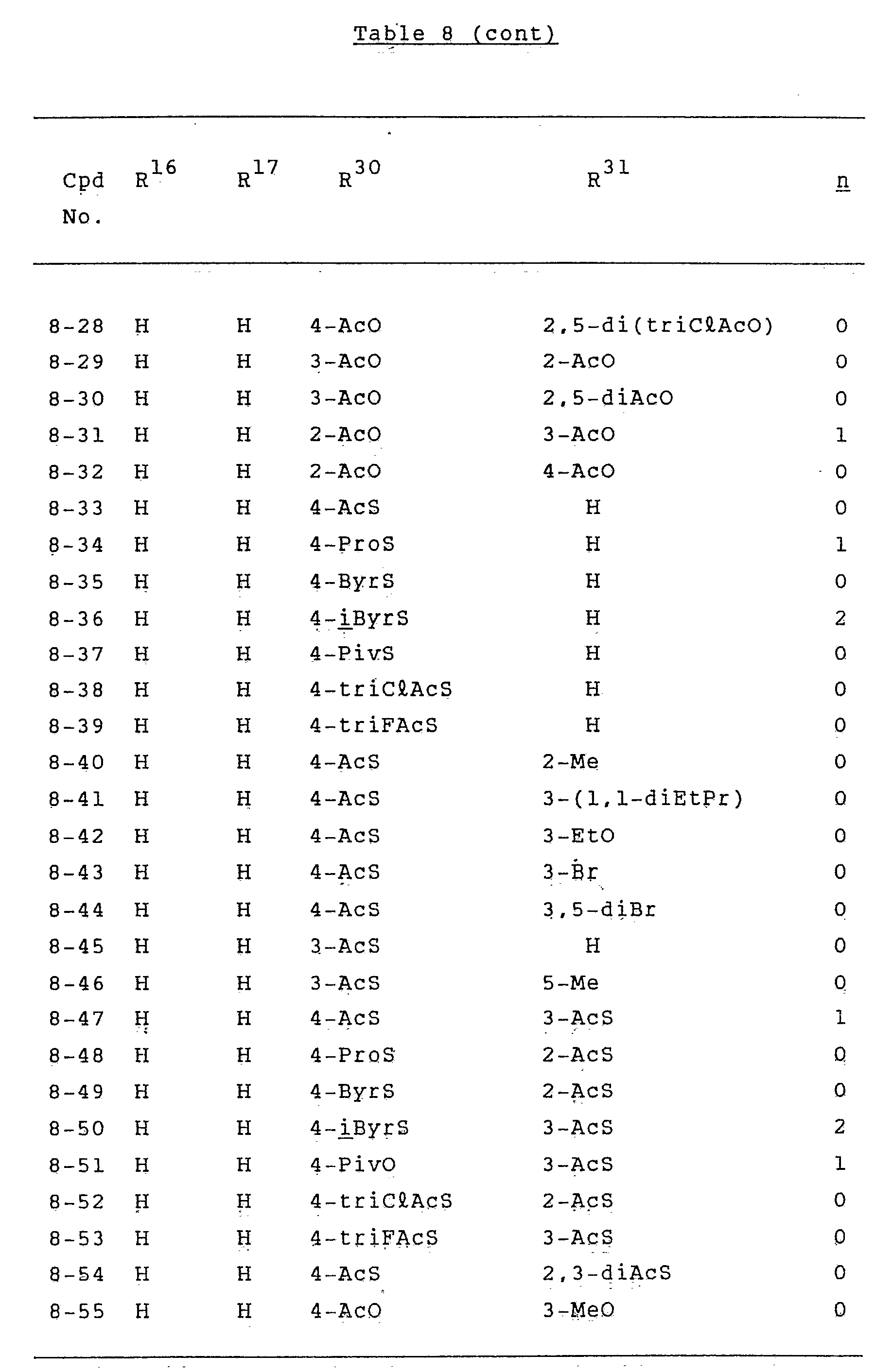

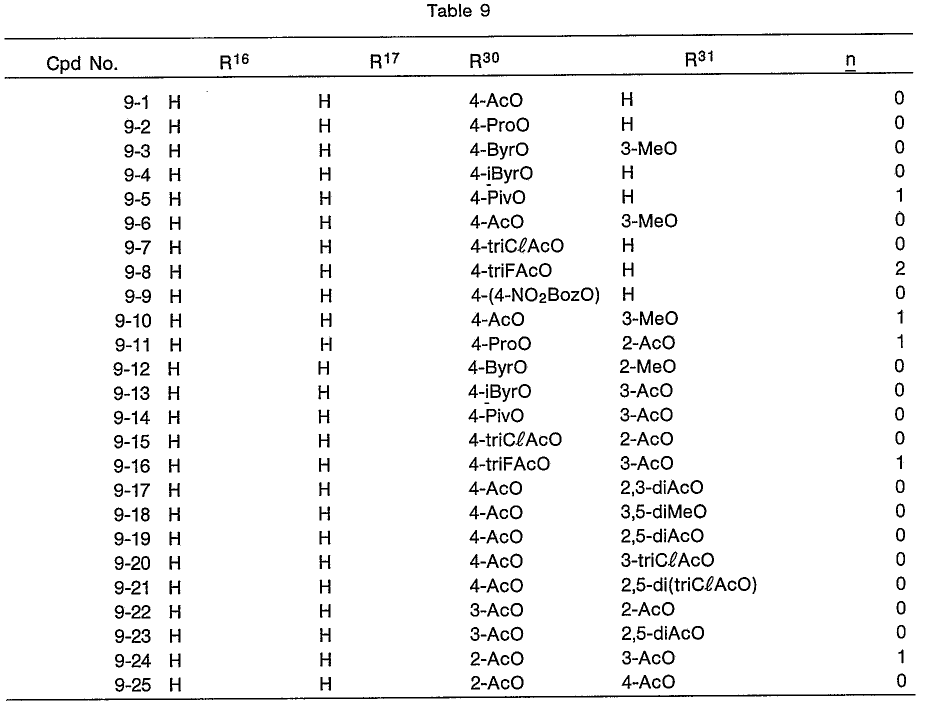

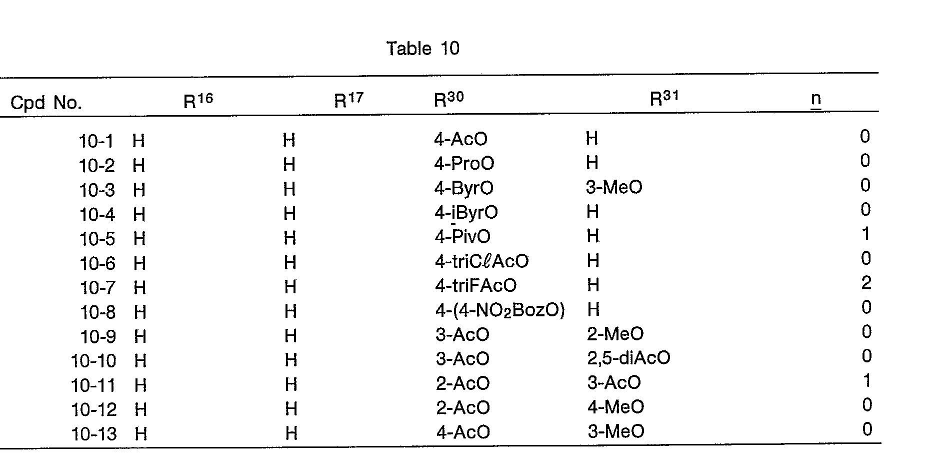

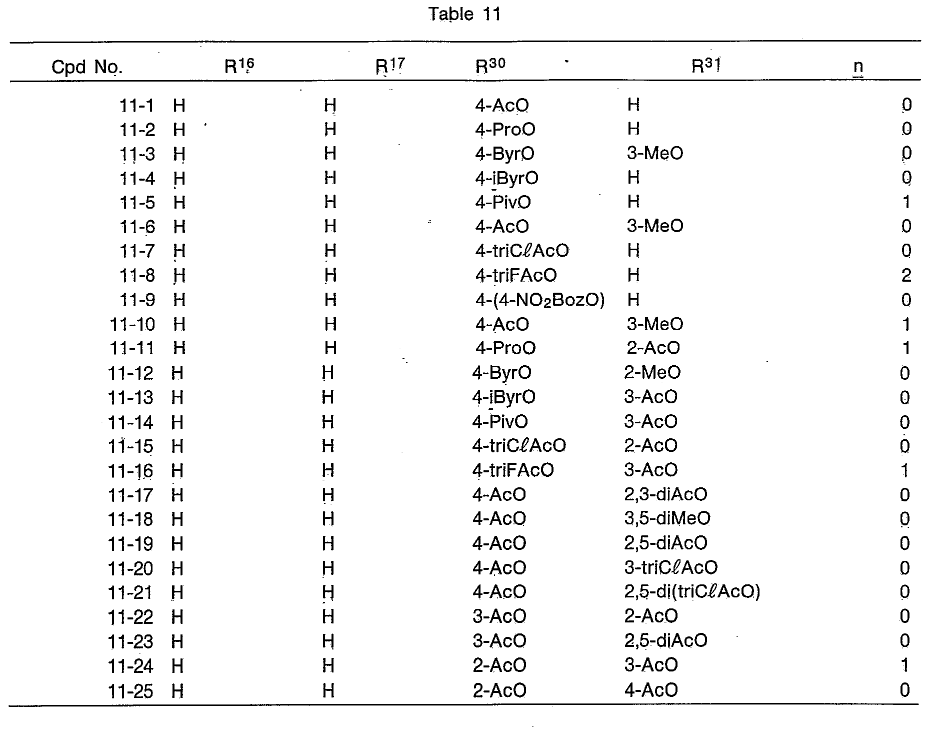

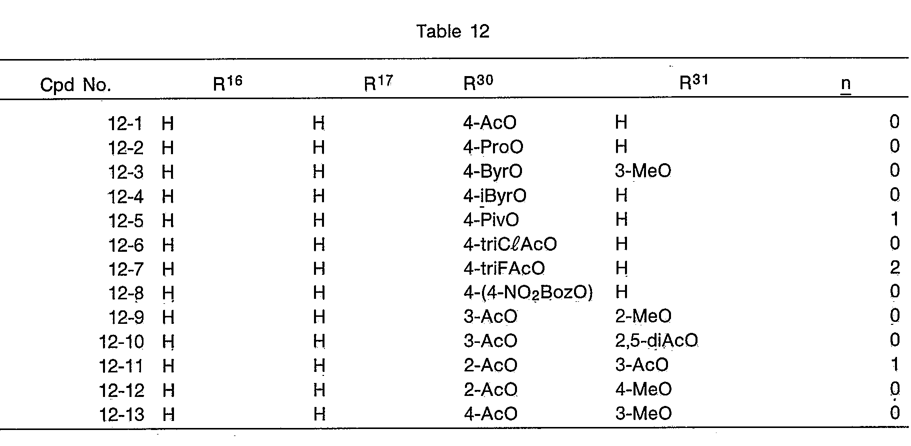

Of the compounds listed above, the preferred compounds are Compounds No.: 1-1, 1-2, 1-3, 1-4, 1-6, 1-7, 1-8, 1-9, 1-10, 1-11, 1-12, 1-13, 1-14, 1-15, 1-16, 1-17, 1-18, 1-19, 1-20, 1-21, 1-22, 1-23, 1-24, 1-27, 1-30, 1-31, 1-32, 1-33, 1-34, 1-35, 1-36, 1-37, 2-4, 2-7, 2-12, 2-14, 2-15, 2-32, 2-42, 2-43, 3-3, 3-4, 3-7, 3-8, 3-9, 3-23, 3-32, 3-33, 3-37, 4-5, 4-6, 4-7, 4-8, 5-1, 5-2, 5-3, 5-8, 5-9, 5-10, 6-1, 6-2, 7-1, 7-2, 7-3, 7-4, 7-5, 7-6, 7-7, 7-10, 7-10, 7-11, 7-12,7-13, 7-15, 7-16, 7-19, 7-23, 7-25, 7-29, 7-32, 7-34, 7-39, 7-40, 7-41, 7-43, 7-44, 7-45, 7-47, 7-50, 7-51, 7-56, 7-63, 7-65, 7-67, 7-72, 7-73, 7-77, 7-80, 7-88, 7-89, 7-90, 7-91, 7-92, 7-93, 8-1, 8-2, 8-3, 8-4, 8-5, 8-15, 8-17, 8-18, 8-24, 8-25, 8-29, 8-32, 8-33, 8-40, 8-42, 8-43, 8-55, 9-1, 9-2, 9-4, 9-5, 9-6, 9-10, 9-12, 9-18, 9-22, 9-25, 10-1, 10-2, 10-4, 10-13, 11-1, 11-2, 11-4, 11-5, 11-6, 11-10, 11-12, 11-18, 11-22, 11-25, 12-1, 12-2, 12-4 and 12-13.

More preferred compounds are Compounds No.: 1-2, 1-3, 1-4, 1-6, 1-7, 1-8, 1-9, 1-14, 1-15, 1-16, 1-18, 1-22, 1-23, 1-32, 1-33, 1-34, 1-35, 1-36, 1-37, 2-4, 2-7, 2-12, 2-14, 2-15, 2-32, 2-42, 2-43, 3-3, 3-4, 3-7, 3-8, 3-23, 3-33, 3-37, 4-6, 5-1,5-2, 5-8, 6-1, 6-2, 7-1, 7-2, 7-3, 7-4, 7-5, 7-10, 7-11, 7-15, 7-32, 7-43, 7-44, 7-47, 7-50, 7-63, 7-89, 7-90, 7-91, 7-92, 7-93, 8-1, 8-2, 8-3, 8-4, 8-17, 8-25, 8-33, 8-42, 8-43, 8-55, 9-1, 9-2, 9-5, 9-6, 9-10, 9-18, 9-22, 9-25, 10-1, 10-2, 10-4, 10-13, 11-1, 11-2, 11-5, 11-6, 11-10, 11-18, 11-22, 11-25, 12-1, 12-2, 12-4 and 12-13. The most preferred compounds are:
2-4. 9'-(1-butyryloxyethyl) griseolate
2-7.9'-(1-isobutyryloxypropyl) griseolate
2-12.9'-(1-isovaleryloxyethyl) griseolate
2-14.9'-pivaloyloxymethyl griseolate
2-15.9'-(1-pivaloyloxyethyl) griseolate
2-32.9'-pivaloyloxymethyl 7'-desoxygriseolate
2-42.9'-pivaloyloxymethyl 7'-desoxy-4',5'-dihydrogriseolate
2-43. 9'-(1-pivaloyloxyethyl) 7'-desoxy-4',5'-dihydrogriseolate
4-6.9'-(1-isopropoxycarbonyloxyethyl) griseolate
6-1. 9'-(5-methyl-2-oxo-1,3-dioxolen-4-yl)methyl griseolate
7-1.9'-(4-acetoxybenzyl) griseolate
7-10.9'-(4-acetoxy-3-methoxybenzyl) griseolate
7-63. 9'-(4-acetylthiobenzyl) griseolate
8-1. 8'-(4-acetoxybenzyl) griseolate
8-33. 8'-(4-acetylthiobenzyl) griseolate
8-55.8'-(4-acetoxy-3-methoxybenzyl) griseolate
9-1.9'-(4-acetoxybenzyl) 7'-desoxygriseolate
9-6.9'-(4-acetoxy-3-methoxybenzyl) 7'-desoxygriseolate
10-1. 8'-(4-acetoxybenzyl) 7'-desoxygriseolate
10-13.8'-(4-acetoxy-3-methoxybenzyl) 7'-desoxygriseolate
11-1. 9'-(4-acetoxybenzyl)7'-desoxy-4',5'-dihydrogriseolate
11-6.9'-(4-acetoxy-3-methoxybenzyl)7'-desoxy-4',5'-dihydrogriseolate
12-1.8'-(4-acetoxybenzyl) 7'-desoxy-4',5'-dihydrogriseolate
12-13.8'-(4-acetoxy-3-methoxybenzyl)7'-desoxy4',5'-dihydrogriseolate

In general terms, the compounds of the present invention may be prepared by a conventional esterification reaction of a compound of formula (IX): 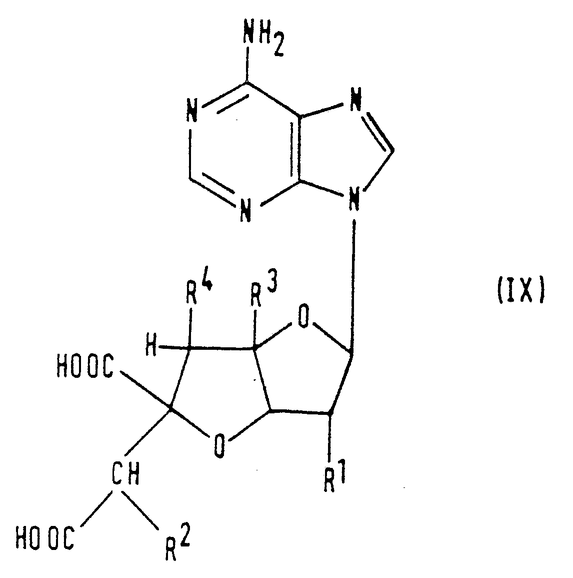 (in which R¹, R², R³ and R⁴ are as defined above) or of a corresponding compound in which one of the carboxy groups (that which it is not desired to esterify) is protected or of a corresponding compound in which one of the carboxy groups (that which it is desired to esterify) is replaced by an active derivative thereof.

The esterification may be effected by conventional means, as described in detail hereafter, and, where one of the carboxy groups is not protected, the quantity of esterifying agent is restricted in order to ensure preferential production of the monoester, rather than the diester. In such a case, a mixture of the 8'- and 9'- monoesters will normally be prepared, and the two esters may be separated by conventional procedures, e.g. the various chromatographic techniques, especially column chromatography.

The reactions involved may be represented by the scheme: 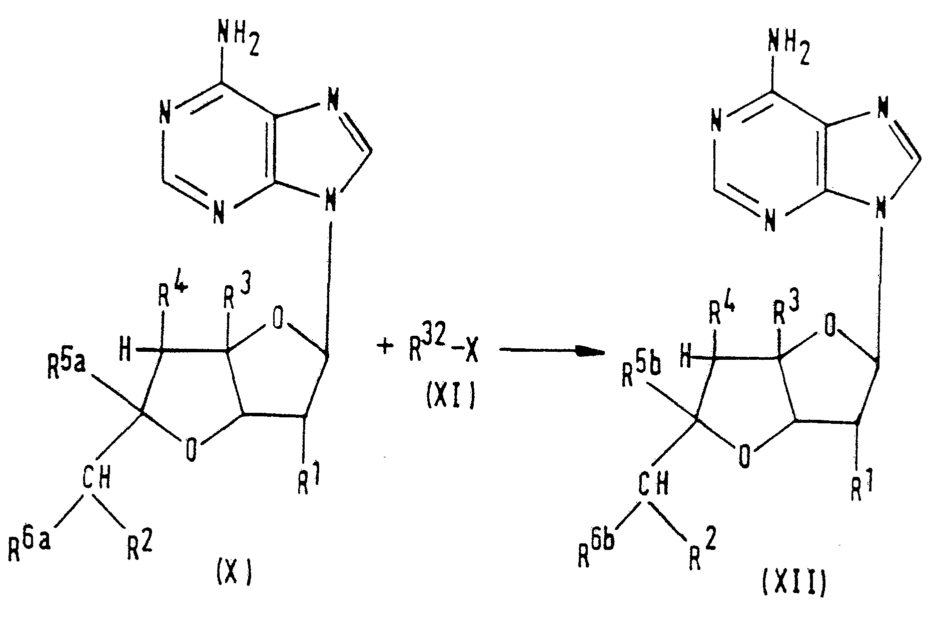 [in which:
R¹, _{R}2, R³ and R⁴ are as defined above;
X represents a leaving group, e.g. as exemplified below;
R³² represents a carboxy-protecting group removable in the biochemical environment of the human eye; one of R^{5a} and R^{6a} represents a carboxy group;
the other one of R^{5a} and R^{6a} represents a carboxy group or a protected carboxy group;
one of R^{5b} and R^{6b} represents a carboxy group or a protected carboxy group; and
the other one of R^{5b} and R^{6b} represents a carboxy-protecting group removable in the biochemical environment of the human eye];
and, where said one of R^{5b} and R^{Sb} represents a protected carboxy group, removing said group without removing said other one of R^{5b} and _{R}6^{b}_{;}
and optionally removing one or both protecting groups represented by R¹ and R².

Examples of groups which may be represented by X include: halogen atoms, such as the chlorine, bromine and iodine atoms; aliphatic acyloxy groups, such as the alkylcarbonyloxy groups (e.g. the acetoxy and propionyloxy groups), the halogenated alkylcarbonyloxy groups (e.g. the chloroacetoxy, dichloroacetoxy, trichloroacetoxy and trifluoroacetoxy groups), the lower alkoxyalkylcarbonyloxy groups (e.g. the methoxyace- toxy group), the unsaturated alkylcarbonyloxy groups (e.g. the (E)-2-methyl-2-butenoyloxy group); aromatic acyloxy groups, such as the arylcarbonyloxy groups (e.g. the benzoyloxy group), the halogenated arylcarbonyloxy groups (e.g. the 2-bromobenzoyloxy and 4-chlorobenzoyloxy groups), the lower alkylated arylcarbonyloxy groups (e.g. the 2,4,6-trimethylbenzoyloxy and 4-toluoyloxy groups), the lower alkoxylated arylcarbonyloxy groups (e.g. the 4-anisoyloxy group), and the nitrated arylcarbonyloxy groups (e.g. the 4-nitrobenzoyloxy and 2-nitrobenzoyloxy groups); the trihalomethoxy groups, such as the trichloromethoxy group; the lower alkanesulphonyloxy groups, such as the methanesulphonyloxy and ethanesulphonyloxy groups; the halogen-substituted lower alkanesulphonyloxy groups, such as the trifluoromethanesulphonyloxy and pentafluoroethanesulphonyloxy groups; the arylsulphonyloxy groups, such as the benzenesulphonyloxy and p-toluenesulphonyloxy groups; and diazo groups.

In more detail, one possible reaction for preparing the compounds of the present invention is as shown in Reaction Scheme A: 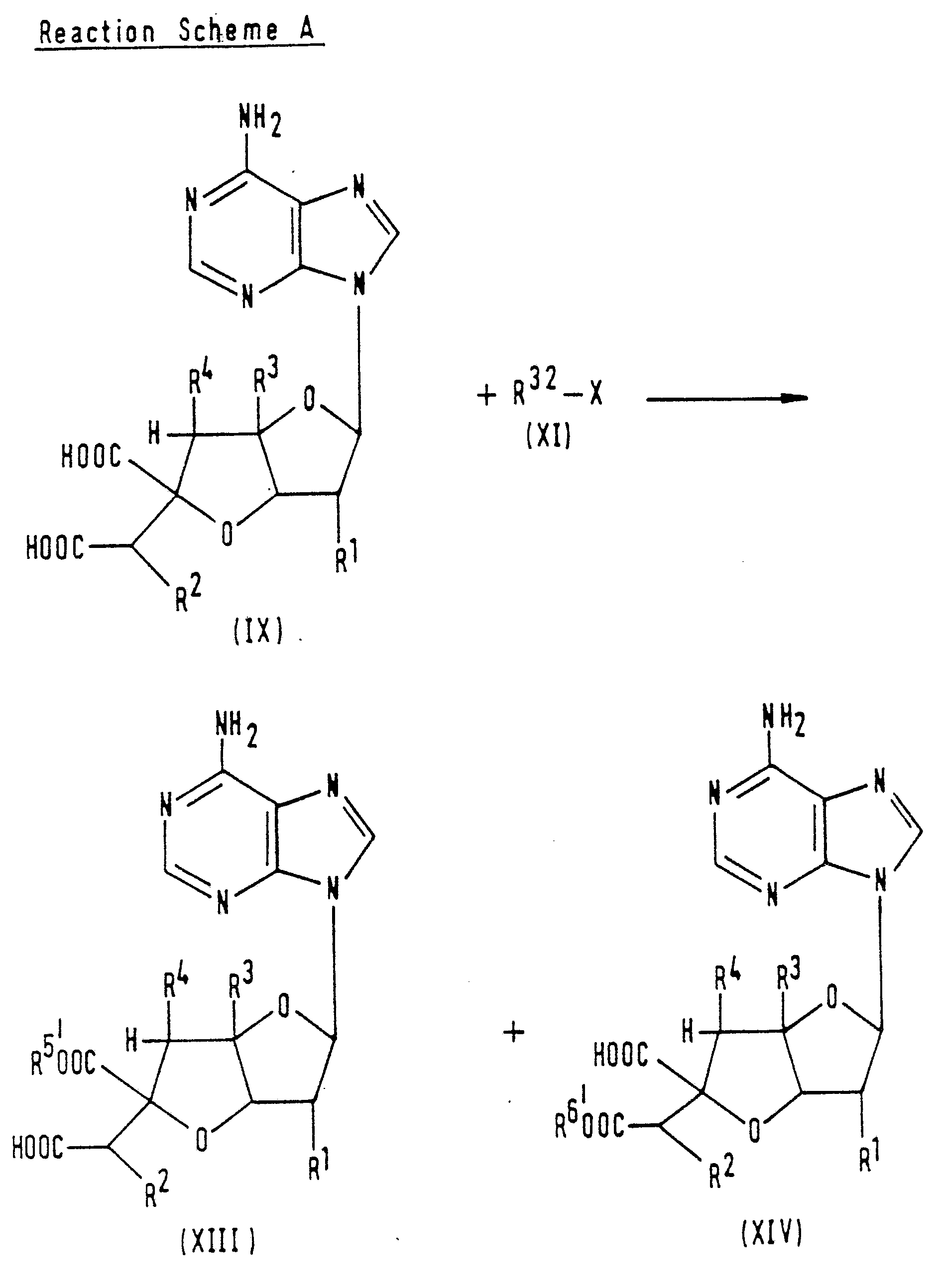 in which:
R¹, _{R}2, R3, _{R}4, R3² and X are as defined above; and
R⁵' and R⁶' are independently selected from carboxy-protecting groups removable in the biochemical environment of the human eye.

In this reaction scheme, a griseolic acid or derivative thereof of formula (IX) is reacted with an active compound containing the ester group that it is desired to introduce and represented by the formula R³² -X (XI), to give a mixture of the two monoesters of formulae (XIII) and (XIV).

Especially where one of R⁵ and R⁶ in the compounds of formula (I) represents a group of formula (II), (III), (IV), (V), (VI), (VII), (VIII), (Ila), (Illa), (IVa) or (Va), the reaction can easily be effected without protecting the other of R⁵ and R⁶. In this case, the compound of formula (XI) is preferably one of the compounds of formula (Xla), (Xlb), (Xlc) or (Xid): 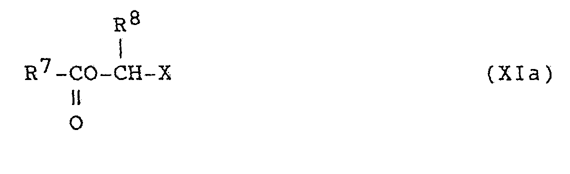 (in which R⁷, R⁸ and X are as defined above); 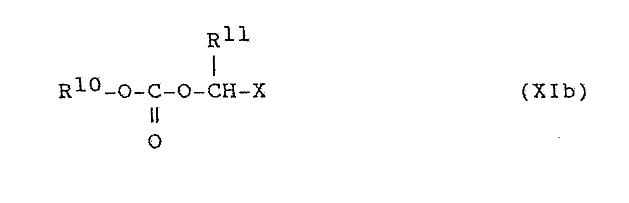 (in which R , R and X are as defined above); 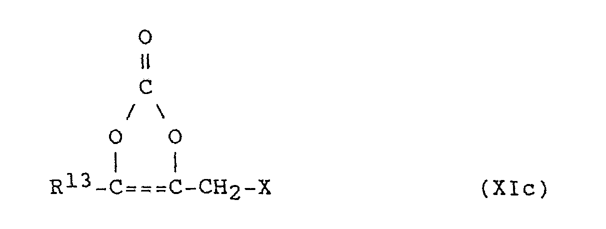 (in which _{R}¹³ and X are as defined above); or 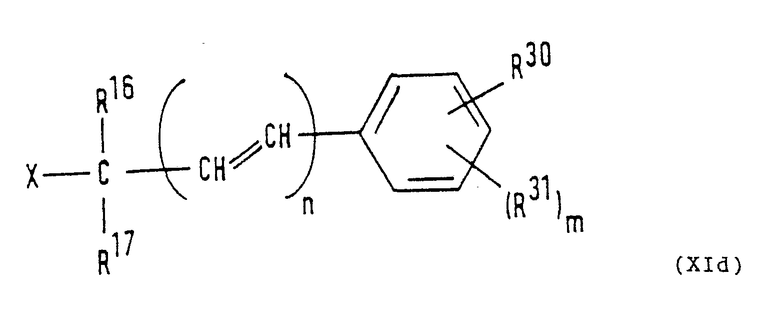 (in which _{R}¹⁶, R¹⁷, R³⁰, R³¹. _{X}, ₘ and ₙ are as defined above).

The reaction is preferably effected in the presence of a solvent and in the presence of an acid-binding agent.

There is no particular restriction on the nature of the solvent to be employed, provided that it has no adverse effect on the reaction or on the reagents involved and provided that it can dissolve the starting materials, at least to some degree. Examples of suitable solvents include: amides, especially fatty acid amides, such as dimethylformamide or dimethylacetamide and other amides, such as hexamethylphosphoric triamide; sulphoxides, such as dimethyl sulphoxide; and nitriles, such as acetonitrile. A single one of these solvents may be used. It is also possible to use a mixture of two or more such solvents.

The nature of the acid-binding agent to be used in this reaction is equally not particularly critical, and any conventional acid-binding agent commonly used in this type of reaction may also be used here. Examples include organic bases, such as triethylamine, diisopropylethylamine, N-methylmorpholine, pyridine, 4-(N,N-dimethylamino)pyridine, N,N-dimethylaniline, 1,5-diazabicyclo[4.3.0]non-5-ene, 1,4-diazabicyclo[2.2.2]octane and 1,8-diazabicyclo[5.4.0]undec-7-ene (DBU), of which DBU is particularly preferred.

The leaving group X to be eliminated in the esterifying reagent is preferably a halogen atom (such as chlorine, bromine or iodine), and particularly preferably an iodine atom.

In order to ensure preferential formation of the monoesters of formulae (XIII) and (XIV), rather than the diester, we prefer to restrict the quantity of the esterifying agent of formula (XI) to around 1 equivalent per equivalent of the compound of formula (IX). Of course, as is well known in the art, amounts above or below this may be employed, and, in special circumstances, such amounts may be desirable for particular purposes, despite the reduction in yields of the monoesters that will result. However, in general, amounts of about 1 equivalent are preferred.

The reaction can take place over a wide range of temperatures, and the precise reaction temperature is not critical to the invention. In general, we find it cpnvenient to carry out the reaction at a temperature from 0 to ₁₀₀° C, preferably from 20 to 70° C, and particularly preferably at room temperature. The time required for the reaction may also vary widely, depending on many factors, notably the reaction temperature and the nature of the reagents and acid-binding agent. However, provided that the reaction is effected under the preferred conditions outlined above, a period of from 1 hour to 4 days will usually suffice.

The nature of the reaction involved in the deprotection step for removing the hydroxy-protecting group will, of course, depend on the type of protecting group, as is well known in the art. Examples of reactions which may be employed are given below, but it will naturally be appreciated that these are solely by way of example and that any other reaction known for this purpose may equally be employed.

When a silyl group is used as the hydroxy-protecting group, the protecting group is usually removed by treatment with a compound which forms a fluorine anion, such as tetrabutylammonium fluoride. This reaction is normally effected in a solvent. Although the nature of the reaction solvent used is not particularly critical, provided that it does not inhibit the reaction, preferred examples include ethers such as tetrahydrofuran and dioxane. The reaction can take place over a wide range of temperatures, and the precise reaction temperature is not critical to the invention. In general, we find it convenient to carry out the reaction at room temperature. The time required for the reaction may also vary widely, depending on many factors, notably the reaction temperature and the nature of the reagents. However, provided that the reaction is effected under the preferred conditions outlined above, a period of from 10 to 18 hours will usually suffice.

When the hydroxy-protecting group is an aralkyloxycarbonyl group or an aralkyl group, it can usually be removed by bringing it into contact with a reducing agent. For example, removal of the protecting group can be achieved by catalytic reduction in the presence of hydrogen, preferably at ambient temperature, using a reduction catalyst, such as palladium-on-carbon, platinum or Raney nickel. The reaction is normally carried out in the presence of a solvent. There is no particular restriction on the nature of the solvent to be employed, provided that it has no adverse effect on the reaction or on the reagents involved. Examples of suitable solvents include: alcohols, such as methanol and ethanol; ethers, such as tetrahydrofuran and dioxane; fatty acids, such as acetic acid; or a mixture of one or more such organic solvents with water. The reaction can take place over a wide range of temperatures, and the precise reaction temperature is not critical to the invention. In general, we find it convenient to carry out the reaction at a temperature from 0° C to room temperature. The time required for the reaction may also vary widely, depending on many factors, notably the reaction temperature and the nature of the reagents and the reducing agent. However, provided that the reaction is effected under the preferred conditions outlined above, a period of from 5 minutes to 12 hours will usually suffice.

Such a protecting group can also be removed by reacting the compound with metallic lithium or sodium in liquid ammonia or in an alcohol, such as methanol and ethanol, at a relatively low temperature, e.g. from -78 to -20° C.

Such protecting groups can also be removed by reaction with aluminium chloride-sodium iodide or with an alkylsilyl halide, such as trimethylsilyl iodide. The reaction is normally carried out in the presence of a solvent. There is no particular restriction on the nature of the solvent to be employed, provided that it has no adverse effect on the reaction or on the reagents involved. Examples of suitable solvents include: nitriles, such as acetonitrile; halogenated hydrocarbons, especially halogenated aliphatic hydrocarbons, such as methylene chloride and chloroform; or a mixture of any two or more of these solvents. The reaction can take place over a wide range of temperatures, and the precise reaction temperature is not critical to the invention. In general, we find it convenient to carry out the reaction at a temperature from 0°C to 50°C.

Incidentally, when a sulphur atom is contained in the reaction substrate, aluminium chloride-sodium iodide is preferably used.

When the hydroxy-protecting group is an aliphatic acyl group, an aromatic acyl group or an alkoxycarbonyl group, the protecting group can be removed by treatment with a base in the presence of a solvent. There is no particular restriction on the nature of the base to be employed, provided that it does not affect other parts of the compound, and the deprotection is preferably carried out by using: a metal alcoholate, such as sodium methoxide; an alkali metal carbonate, such as sodium carbonate or potassium carbonate; aqueous ammonia; an alkali metal hydroxide, such as sodium hydroxide or potassium hydroxide; or concentrated ammonia-methanol. There is no particular restriction on the nature of the solvent to be employed, provided that it has no adverse effect on the reaction or on the reagents involved and can be used for ordinary hydrolytic reactions. Examples of suitable solvents include water and organic solvents such as: alcohols, e.g. methanol, ethanol or propanol; and ethers, e.g. tetrahydrofuran or dioxane; and mixtures of water with one or more such organic solvents. The reaction can take place over a wide range of temperatures, and the precise reaction temperature is not critical to the invention. In general, we find it convenient to carry out the reaction at a temperature from 0°C to 150°C in order to inhibit side reactions. The time required for the reaction may also vary widely, depending on many factors, notably the reaction temperature and the nature of the reagents. However, provided that the reaction is effected under the preferred conditions outlined above, a period of from 1 to 10 hours will usually suffice.

When the hydroxy-protecting group is an alkoxymethyl group, a tetrahydropyranyl group, a tetrahydrofuranyl group or a substituted ethyl group, it can usually be removed by treatment with an acid in a solvent. Acids to be used preferably include hydrochloric acid, acetic acid-sulphuric acid, p-toluenesulphonic acid or acetic acid. There is no particular restriction on the nature of the solvent to be employed, provided that it has no adverse effect on the reaction or on the reagents involved. Examples of suitable solvents include: alcohols, such as methanol and ethanol; ethers, such as tetrahydrofuran and dioxane; and mixtures of water with one or more such organic solvents. The reaction can take place over a wide range of temperatures, and the precise reaction temperature is not critical to the invention. In general, we find it convenient to carry out the reaction at a temperature from 0°C to 50° C. The time required for the reaction may also vary widely, depending on many factors, notably the reaction temperature and the nature of the reagents. However, provided that the reaction is effected under the preferred conditions outlined above, a period of from 10 minutes to 18 hours will usually suffice.

When the hydroxy-protecting group is an alkenyloxycarbonyl group, it can be eliminated by treatment with a base using similar conditions to those used for the removal when the hydroxy-protecting group is an aliphatic acyl group, an aromatic acyl group or an alkoxycarbonyl group. It should be noted that, when the protecting group is an allyloxycarbonyl group, it can most conveniently be removed by using palladium and triphenylphosphine, or nickel tetracarbonyl with no substantial side reactions.

After completion of the reaction, if desired, the 2'- and/or 7'- hydroxy groups can be converted into other functional groups by the method described in European Patent Publication No. 143 557A. This optional reaction can be applied in Steps B4, B5 and C3.

An alternative method of preparing the compounds of the present invention is illustrated in the following Reaction Schemes B and C: 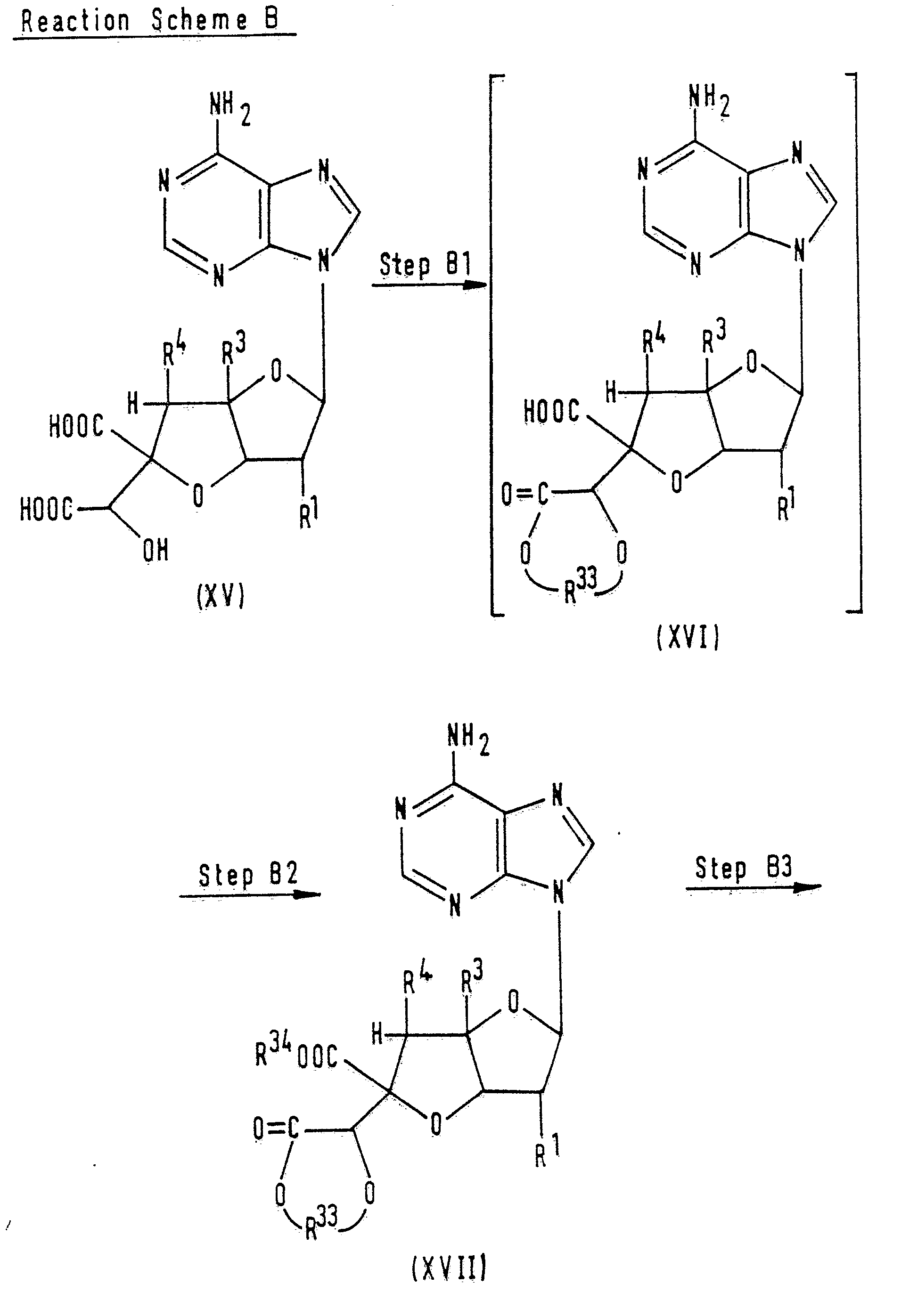 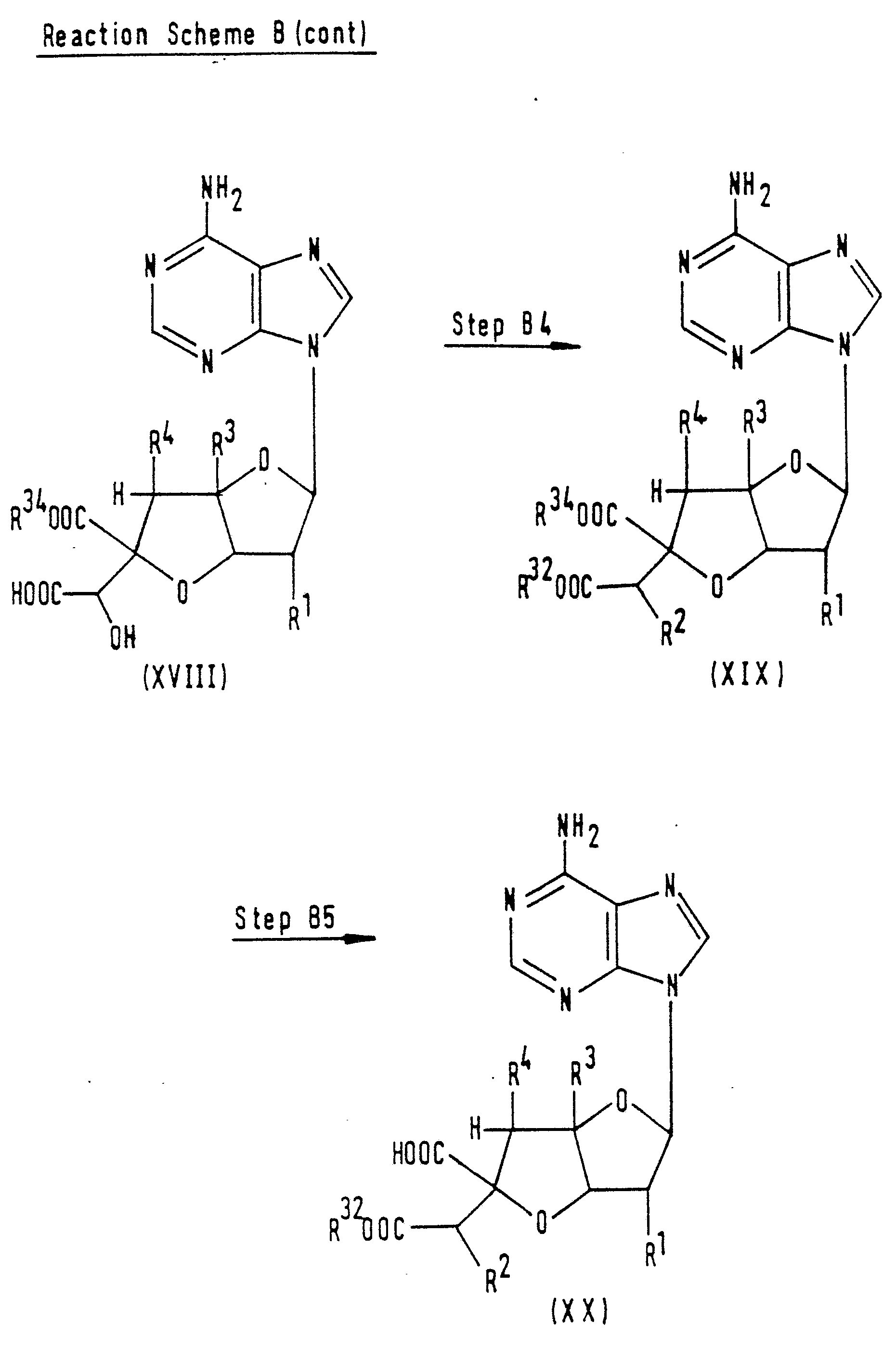 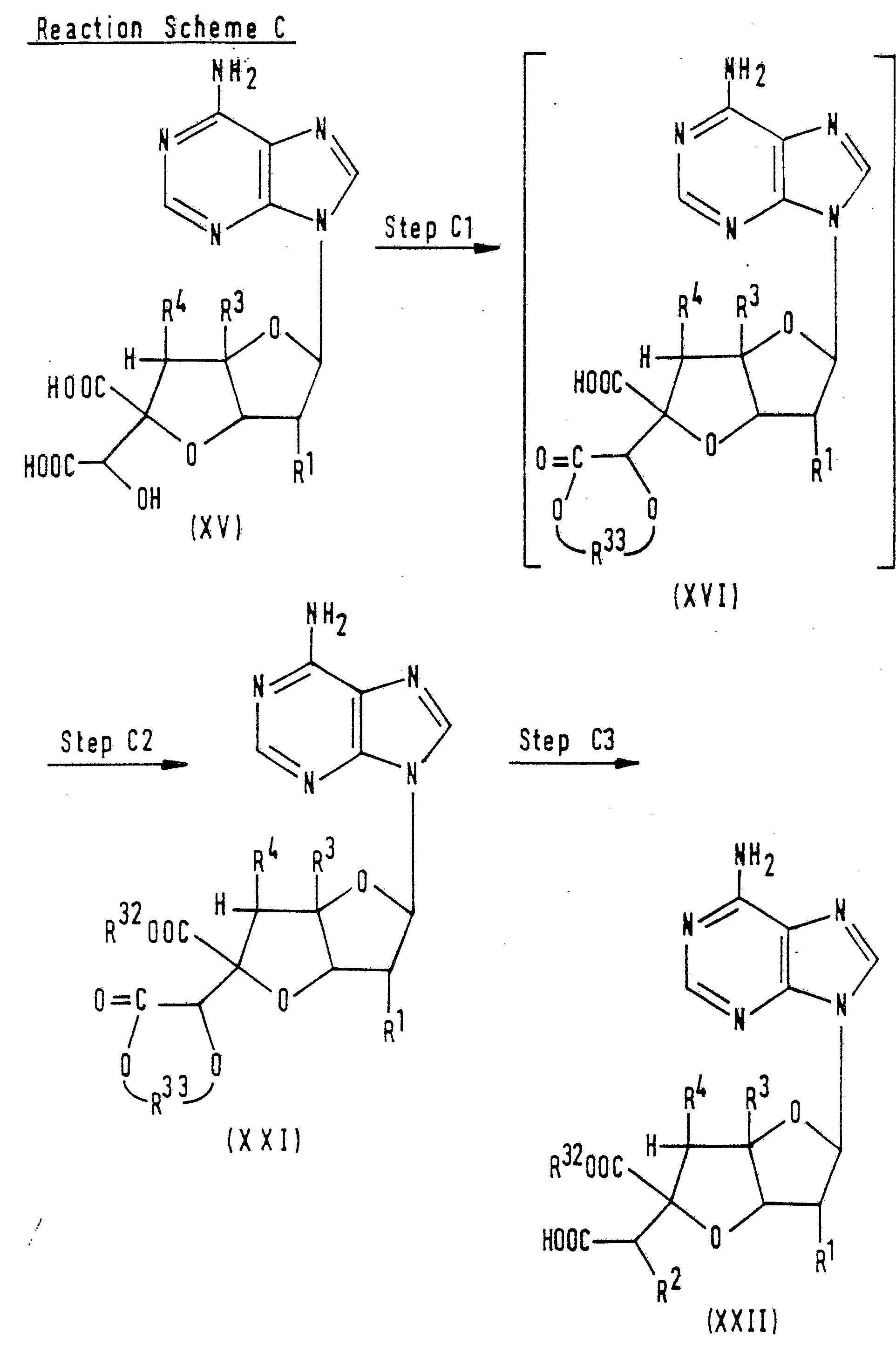 In the above formulae:
_{R}1, _{R}2, R3, R4 and R³² are as defined above.

R³³ represents a protecting group for a dihydroxy group, for example: a lower (e.g. C₁ - C₄, preferably Ci - Cs) alkylidene group, such as a methylidene, ethylidene or isopropylidene group; an aralkylidene group, such as a benzylidene group; or an alkoxyethylidene group (in which the alkoxy part is preferably Ci - C₄, more preferably Ci - C₃), such as a methoxyethylidene or ethoxyethylidene group.

R³⁴ represents a carboxy-protecting group for use during the alkylation reaction, for example: a lower (e.g. C₁ - C₆) alkyl group, such as a methyl, ethyl, propyl, isopropyl, butyl, isobutyl, sec-butyl, t-butyl, pentyl or hexyl group; a halogen-substituted lower (e.g. C₁ - Cₛ, preferably Cᵢ - C₃) alkyl group, such as a 2,2,2-trichloroethyl, 2-bromoethyl, 2-chloroethyl, 2-fluoroethyl or 2,2-dibromoethyl group; or an aralkyl group comprising a lower (e.g. Ci - C₄, preferably Ci - C₃) alkyl group substituted with from 1 to 3 aryl groups (such as a benzyl, phenethyl, 3-phenylpropyl, a-naphthylmethyl, 0-naphthylmethyl, diphenylmethyl, triphenylmethyl, a-naphthyldiphenylmethyl or 9-anthranylmethyl group) or comprising such a lower alkyl group substituted with from 1 to 3 aryl groups which are themselves substituted with a lower alkyl group, a lower alkoxy group, a nitro group, a halogen atom, a cyano group or an alkylenedioxy group [such as a 4-methylbenzyl, 2,4,6-trimethylbenzyl, 3,4,5-trimethylbenzyl, 4-methoxybenzyl, 4,4'-dichlorodiphenylmethyl, 4,4',4"-trichlorotriphenylmethyl, 4-methoxyphenyldiphenylmethyl, 2-nitrobenzyl, 4-nitrobenzyl, 4-chlorobenzyl, 4-bromobenzyl, 4-cyanobenzyl, 4-methoxybenzhydryl, 4,4'-dimethoxybenzhydryl, 4-cyanobenzyldiphenylmethyl, bis(2-nitrophenyl)methyl or piperonyl group].

We prefer to select the protecting group represented by R³⁴ so that it can be eliminated selectively should there be two or more protecting groups, e.g. R¹ as well as R³⁴, in the compound of formula (XIX); accordingly, groups which can easily be removed by an acid, such as an aralkyl group, are preferred.

Reaction Scheme B describes a procedure for preparing 9'-monoester derivatives, and Reaction Scheme C describes a procedure for preparing 8'-monoester derivatives.

In Reaction Scheme B, the carboxy group at the 9'-position is first protected by formation of a cyclic group with the 7'-hydroxy group. The resulting compound is then reacted with a suitable compound to introduce a protecting group at the 8'-carboxy group. With this protected, the protecting group is then removed from the 9'-position and then the 9'-carboxy group is reacted with a suitable compound to introduce the desired ester group at that position.

In Step B1 and Step C1, which are identical, a compound of formula (XVI) is prepared by reacting the 7'-hydroxy group and the hydroxy group forming part of the 9'-carboxy group in a compound of formula (XV) with a suitable compound to form a protecting group for the dihydroxy group. This reaction is normally carried out in the presence of a catalyst and in the presence or absence of a solvent.

In this Step, the reaction is carried out whilst removing the water produced as the reaction proceeds by means of a dehydrating agent, such as anhydrous copper sulphate, anhydrous sodium sulphate, anhydrous calcium carbonate or a molecular sieve, or by using an azeotropic technique.

Examples of suitable compounds to form protecting groups for the dihydroxy group employed in this Step include: lower (e.g. Ci - C₄) alkylcarbonyl compounds, such as formaldehyde, acetaldehyde or acetone; arylcarbonyl compounds, such as benzaldehyde; and lower (e.g. Ci - C₄) alkyl orthoformates, such as trimethyl orthoformate or triethyl orthoformate; of these, we prefer the lower alkylcarbonyl compounds, more preferably acetone.

There is no particular restriction on the nature of the solvent to be employed, provided that it has no adverse effect on the reaction or on the reagents involved and that it can dissolve the starting materials, at least to some extent. Examples of suitable solvents include: aromatic hydrocarbons, such as benzene, toluene or xylene; halogenated hydrocarbons, especially halogenated aliphatic hydrocarbons, such as methylene chloride or chloroform; esters, such as ethyl acetate or propyl acetate; ethers, such as diethyl ether, tetrahydrofuran, dioxane or 1,2-dimethoxyethane; alcohols, such as methanol, ethanol, propanol, isopropanol, butanol, isobutanol or isopentanol; amides, such as dimethylformamide, dimethylacetamide or hexamethylphosphoric triamide; sulphoxides, such as dimethyl sulphoxide; and ketones, such as acetone. When acetone is selected as the protecting reagent, it may simultaneously serve as the solvent.

Any catalyst conventionally used as an acidic catalyst may be used without any particular limitation, but we prefer to use a Bronsted acid (for example: an inorganic acid, such as hydrochloric acid, hydrobromic acid, sulphuric acid or perchloric acid; or an organic acid, such as p-toluenesulphonic acid, acetic acid, trifluoroacetic acid or trifluoromethanesulphonic acid) or a Lewis acid (such as zinc chloride, stannic chloride or boron trifluoride etherate). An organic acid is more preferred and a strong organic acid is most preferred.

The reaction can take place over a wide range of temperatures, and the precise reaction temperature is not critical to the invention. In general, we find it convenient to carry out the reaction at a temperature from -10°C to 100°C, preferably from 0°C to room temperature. The time required for the reaction may also vary widely, depending on many factors, notably the reaction temperature and the nature of the reagents, the solvent and the catalyst. However, provided that the reaction is effected under the preferred conditions outlined above, a period of from 10 minutes to 3 days will usually suffice.

After completion of the reaction, the desired compound can be recovered from the reaction mixture by conventional means. For example, one suitable recovery scheme comprises: adding a water-immiscible organic solvent to the reaction mixture; washing the mixture with water; and then removing the solvent by distillation e.g. under reduced pressure, to afford the desired product. The product, if required, may be further purified by such conventional techniques as recrystallization, reprecipitation or the various chromatography techniques, notably column chromatography. Preferably, the reaction mixture is first neutralized with a base insoluble in an organic solvent (such as: an alkali metal carbonate, e.g. sodium carbonate, potassium carbonate or calcium carbonate; an alkali metal bicarbonate, e.g. sodium bicarbonate or potassium bicarbonate; an alkali metal hydride, e.g. lithium hydride, sodium hydride or potassium hydride; or an alkali metal or alkaline earth metal hydroxide, e.g. sodium hydroxide, potassium hydroxide, calcium hydroxide or barium hydroxide), and, after removing insoluble materials by filtration, the solvent is then removed, e.g. by distillation under reduced pressure, to afford the desired product, which may then be subjected to Step B2 without further purification.

In Step B2, a compound of formula (XVII) is prepared by reacting the compound of formula (XVI), preferably in a solvent and in the presence of an acid-binding agent, with a suitable amount, e.g. from 1 to 1.5 equivalents, of an esterifying agent of formula R^{34 -} X (in which R³⁴ and X are as defined above), to esterify the 8'-carboxy group and thereby to protect it during subsequent reactions.

There is no particular restriction on the nature of the solvent to be employed, provided that it has no adverse effect on the reaction or on the reagents involved and that it can dissolve the starting materials, at least to some extent. Examples of suitable solvents include: amides, such as dimethylformamide, dimethylacetamide or hexamethylphosphoric triamide; sulphoxides, such as dimethyl sulphoxide; nitriles, such as acetonitrile; ketones, such as acetone; ethers, such as tetrahydrofuran, diethyl ether or dioxane; or a mixture of any two or more of these solvents.

There is also no particular restriction on the nature of the acid-binding agent to be used, and any compound conventionally used as an acid-binding agent in this type of reaction may equally be used here. Examples include organic amines, such as triethylamine, diisopropylethylamine, N-methylmorpholine, pyridine, 4-(N,N-dimethylamino)pyridine, N,N-dimethylaniline, 1,5-diazabicyclo[4.3.0]non-5-ene (DBN), 1,4-diazabicy- cio[2.2.2]octane and 1,8-diazabicyclo[5.4.0]undec-7-ene (DBU), of which DBU and DBN are more preferred.

Examples of the leaving group represented by X in the esterifying agent have been given above and include: halogen atoms, such as chlorine, bromine or iodine; and the diazo group, but iodine is most preferred.

The reaction can take place over a wide range of temperatures, and the precise reaction temperature is not critical to the invention. In general, we find it convenient to carry out the reaction at a temperature from 0°C to 100°C, preferably from 20°C to 70°C, and more preferably at room temperature. The time required for the reaction may also vary widely, depending on many factors, notably the reaction temperature and the nature of the reagents, the solvent and the acid-binding agent employed. However, provided that the reaction is effected under the preferred conditions outlined above, a period of from 1 hour to 4 days will usually suffice.

In Step B3 a compound of formula (XVIII) is prepared by removing the dihydroxy₋protecting group R³³ by treatment with an acidic catalyst in the presence or absence of a solvent.

There is no particular restriction on the nature of the solvent which may be employed, provided that it has no adverse effect on the reaction or on the reagents involved and that it can dissolve the starting materials, at least to some extent. Examples of suitable solvents include: aromatic hydrocarbons, such as benzene, toluene or xylene; halogenated hydrocarbons, especially halogenated aliphatic hydrocarbons, such as methylene chloride or chloroform; esters, such as ethyl acetate or propyl acetate; ethers, such as diethyl ether, tetrahydrofuran, dioxane or 1,2-dimethoxyethane; alcohols, such as methanol, ethanol, propanol, isopropanol, butanol, isobutanol or isopentanol; amides, such as dimethylformamide, dimethylacetamide or hexamethylphosphoric triamide; sulphoxides, such as dimethyl sulphoxide; and ketones, such as acetone.

Any catalyst conventionally used as an acidic catalyst may be used without any particular limitation, but we prefer to use an aqueous solution of a Bronsted acid (for example: an inorganic acid, such as hydrochloric acid, hydrobromic acid, sulphuric acid or perchloric acid; or an organic acid, such as p-toluenesulphonic acid, acetic acid, citric acid, trifluoroacetic acid or trifluoromethanesulphonic acid). Of these, we prefer to use an aqueous organic acid, more preferably aqueous trifluoroacetic acid.

The reaction can take place over a wide range of temperatures, and the precise reaction temperature is not critical to the invention. In general, we find it convenient to carry out the reaction at a temperature from -10°C to 100° C, preferably from 0°C to room temperature. The time required for the reaction may also vary widely, depending on many factors, notably the reaction temperature and the nature of the reagents, the solvent and the acidic catalyst employed. However, provided that the reaction is effected under the preferred conditions outlined above, a period of from 10 minutes to 1 day will usually suffice.

The protecting group can also be removed without the acidic catalyst by heating simply in one of the aqueous solvents mentioned above.

After completion of the reaction, the desired compound of formula (XVIII) can be recovered from the reaction mixture by conventional means. For example, a suitable recovery procedure comprises: adding a water-immiscible organic solvent to the reaction mixture; washing the mixture with water; and finally removing the solvent by distillation e.g. under reduced pressure, to afford the desired product. The product, if required, may be further purified using such conventional techniques as, for example, recrystallization, reprecipitation or the various chromatography techniques, notably column chromatography.

In Step B4 a compound of formula (XIX) is prepared by esterifying the 9'-carboxy group in the compound of formula (XVIII) with a compound of formula R^{32 -} X (in which R³² and X are as defined above). This reaction is the same as, and may be carried out according to the same procedures as, described in Step B2.

In Step B5 a compound of formula (XX) is prepared by removing the protecting group R³⁴ from the 8'-carboxy group in the compound of formula (XIX).

The method of removing the protecting group will, of course, vary depending upon the nature of that group, but the reaction can generally be carried out by the various known methods described, merely by way of example, below.

When the protecting group is a lower alkyl group or an aryl group, the group can be removed by treating the compound of formula (XIX) with an acid or a base. Suitable acids include the acidic catalysts mentioned in Step B1. There is no particular restriction on the nature of the base which may be be used in this reaction, provided that it has no effect on other parts of the compound. However, in general, we prefer to use: an alkali metal or alkaline earth metal carbonate, such as sodium carbonate, potassium carbonate or calcium carbonate; an alkali metal hydroxide, such as sodium hydroxide or potassium hydroxide; or concentrated methanolic ammonia.

The reaction is preferably effected in the presence of a solvent. There is no particular restriction on the nature of the solvent to be employed, provided that it has no adverse effect on the reaction or on the reagents involved and any commonly used in hydrolysis reactions may equally be used here. Examples of suitable solvents include: water, or a mixture of water with an organic solvent, for example: an alcohol, such as methanol, ethanol or propanol; or an ether, such as tetrahydrofuran or dioxane. The reaction can take place over a wide range of temperatures, and the precise reaction temperature is not critical to the invention. The time required for the reaction may also vary widely, depending on many factors, notably the reaction temperature and the nature of the reagents. In general, we find it convenient to carry out the reaction at a temperature from 0°C to 150°C for from 1 to 10 hours so as to suppress side reactions.

When the protecting group is a diaryl-substituted methyl group, such as a diphenylmethyl group, the group can usually be removed in a solvent under acidic conditions. There is no particular restriction on the nature of the solvent to be employed, provided that it has no adverse effect on the reaction or on the reagents involved. Examples of suitable solvents include: alcohols, such as methanol, ethanol or propanol; ethers, such as tetrahydrofuran or dioxane; and organic acids, such as acetic acid. The acid used is preferably one of the acidic catalysts mentioned in Step B1. The reaction can take place over a wide range of temperatures, and the precise reaction temperature is not critical to the invention. In general, we find it convenient to carry out the reaction at about room temperature. The time required for the reaction may also vary widely, depending on many factors, notably the reaction temperature and the nature of the reagents. However, provided that the reaction is effected under the preferred conditions outlined above, a period of from 10 minutes to 3 days will usually suffice.

When the protecting group is an aralkyl group or a halogen-substituted lower alkyl group, the group can usually be removed by contacting the compound with a reducing agent. In the case of the halogen-substituted lower alkyl groups, the protecting group can preferably be removed with zinc-acetic acid, and, in the case of the aralkyl groups, the protecting group can preferably be removed by catalytic reduction using palladium-on-carbon or platinum as the catalyst, or by treating the compound with an alkali metal sulphide, such as potassium sulphide or sodium sulphide.

The reaction is preferably carried out in the presence of a solvent. There is no particular restriction on the nature of the solvent to be employed, provided that it has no adverse effect on the reaction or on the reagents involved. Examples of suitable solvents include: alcohols, such as methanol or ethanol; ethers, such as tetrahydrofuran or dioxane; aliphatic acids, such as acetic acid; or a mixture of water with one or more of these organic solvents. The reaction can take place over a wide range of temperatures, and the precise reaction temperature is not critical to the invention. In general, we find it convenient to carry out the reaction at a temperature from 0°C to room temperature. The time required for the reaction may also vary widely, depending on many factors, notably the reaction temperature and the nature of the reagents. However, provided that the reaction is effected under the preferred conditions outlined above, a period of from 5 minutes to 12 hours will usually suffice.

When the protecting group is an alkoxymethyl group, the group can usually be removed by treating the compound with an acid. The acid used is preferably one of the acidic catalysts mentioned in Step B1. The reaction is preferably carried out in the presence of a solvent. There is no particular restriction on the nature of the solvent to be employed, provided that it has no adverse effect on the reaction or on the reagents involved. Examples of suitable solvents include: alcohols, such as methanol or ethanol; ethers, such as tetrahydrofuran or dioxane; or a mixture of water and one or more of these organic solvents. The reaction can take place over a wide range of temperatures, and the precise reaction temperature is not critical to the invention. In general, we find it convenient to carry out the reaction at a temperature from 0°C to 50°C. The time required for the reaction may also vary widely, depending on many factors, notably the reaction temperature and the nature of the reagents. However, provided that the reaction is effected under the preferred conditions outlined above, a period of from 10 minutes to 18 hours will usually suffice.

After completion of the reaction, the desired compound can be recovered from the reaction mixture by conventional means. For example, one suitable recovery procedure comprises: adding a water-immiscible organic solvent to the reaction mixture; washing the mixture with water; and then removing the solvent by distillation, e.g. under reduced pressure, to afford the desired product. This product may, if required, be further purified by such conventional techniques as, for example, recrystallization, reprecipitation or the various chromatography techniques, notably column chromatography.

In Reaction Scheme C, the 9'-carboxy group is protected, as in Reaction Scheme B, and then the protected compound is reacted with a compound to introduce the desired group R³² at the 8'-position.

Step C1 is the same process as Step B1 and may be carried out under the same conditions and using the same reagents.

Step C2 is a process for preparing a compound of formula (XXI) by esterifying the 8'-carboxy group in the compound of formula (XVI) with a compound of formula R³²⁻X (in which R³² and X are as defined above). This reaction is the same as, and may be carried out using the same reagents and reaction conditions as, that described in Step B2.

Step C3 is a process for preparing a compound of formula (XXII) by removing the dihydroxy-protecting group from the compound (XXI) with an acidic catalyst in the presence or absence of a solvent. This reaction is the same as, and may be carried out using the same reagents and reaction conditions as, that described in Step B3.

After completion of the reaction, the desired compound can be recovered from the reaction mixture by conventional means. For example, one suitable recovery procedure comprises: adding a water-immiscible organic solvent to the reaction mixture; washing the mixture with water; and then removing the solvent by distillation, e.g. under reduced pressure, to afford the desired product. This product may, if required, be further purified by such conventional techniques as, for example, recrystallization, reprecipitation or the various chromatography techniques, notably column chromatography.

The starting materials used in the present invention are known compounds which can be prepared according to the methods described, for example, in Japanese Patent Application Kokai (i.e. as laid open to public inspection) No. 68695/81, Japanese Patent Application Kokai No. 94992/85, Japanese Patent Application Kokai No. 149394/85, Japanese Patent Application Kokai No. 246396/85 or Japanese Patent Application Kokai No. 100593/86.

The esterifying agent of general formula R^{32 -} X where R³² represents a group of formula (V), especially a group of formula (Va), may be prepared, for example, by the following method or may be obtained commercially. 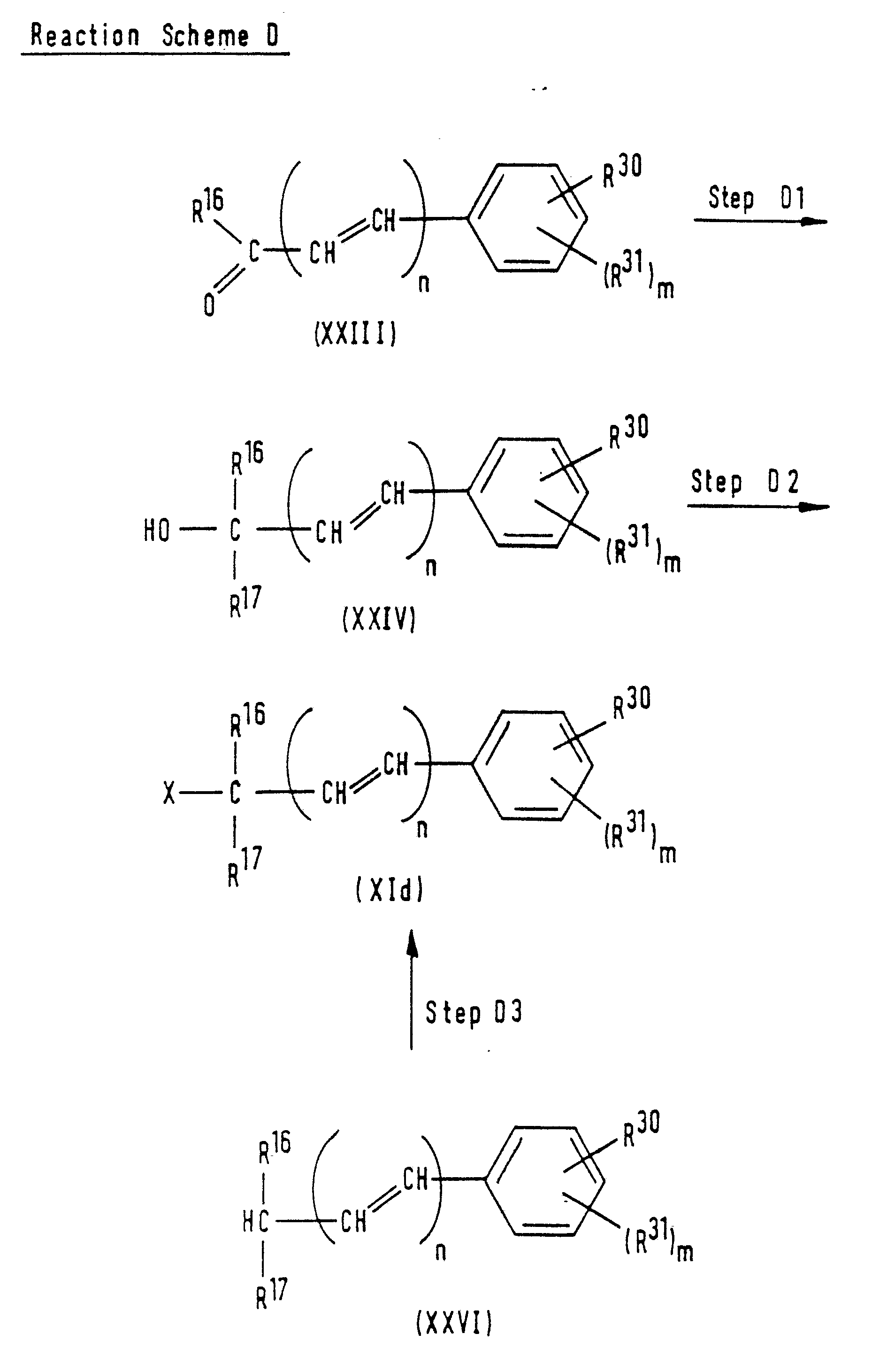 In the above formulae, R¹⁶, R¹⁷, R³⁰, R³¹, m, n and X are as defined above.

Thus, in Step D1, a hydroxy compound of formula (XXIV) is prepared by reduction or by alkylation with a Grignard reagent of a compound of formula (XXIII) which is a known compound or can be prepared without difficulty from a known compound. In Step D2, the hydroxy group thus formed is then converted into a leaving group, such as a halogen atom, to prepare the esterifying agent of formula (Xld), or a compound of formula (XXVI), which is a known compound or can be prepared without difficulty from a known compound, is halogenated at the benzyl or allyl position with a halogenating agent, such as N-bromosuccinimide, and the halogen atom thus introduced, if required, is further converted into another leaving group by a conventional method to prepare the esterifying agent of formula (Xld).

Other esterifying agents employed in the processes of this invention, especially those represented by the formulae (Xla), (Xlb) or (Xlc); 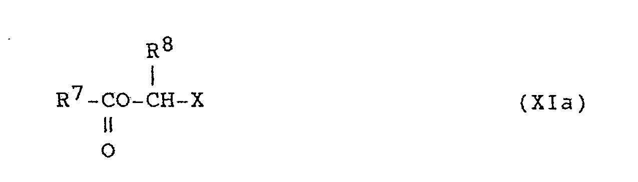 (in which R⁷, R⁸ and X are as defined above); 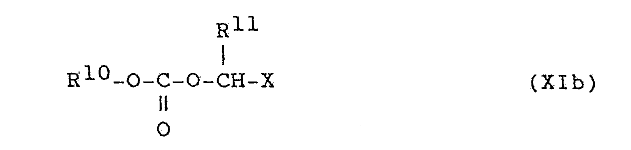 (in which R , R¹¹ and X are as defined above); or 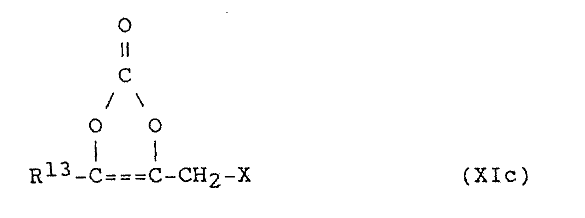 (in which _{R}¹³ and X are as defined above). may be prepared by the processes shown in Reaction Scheme E: 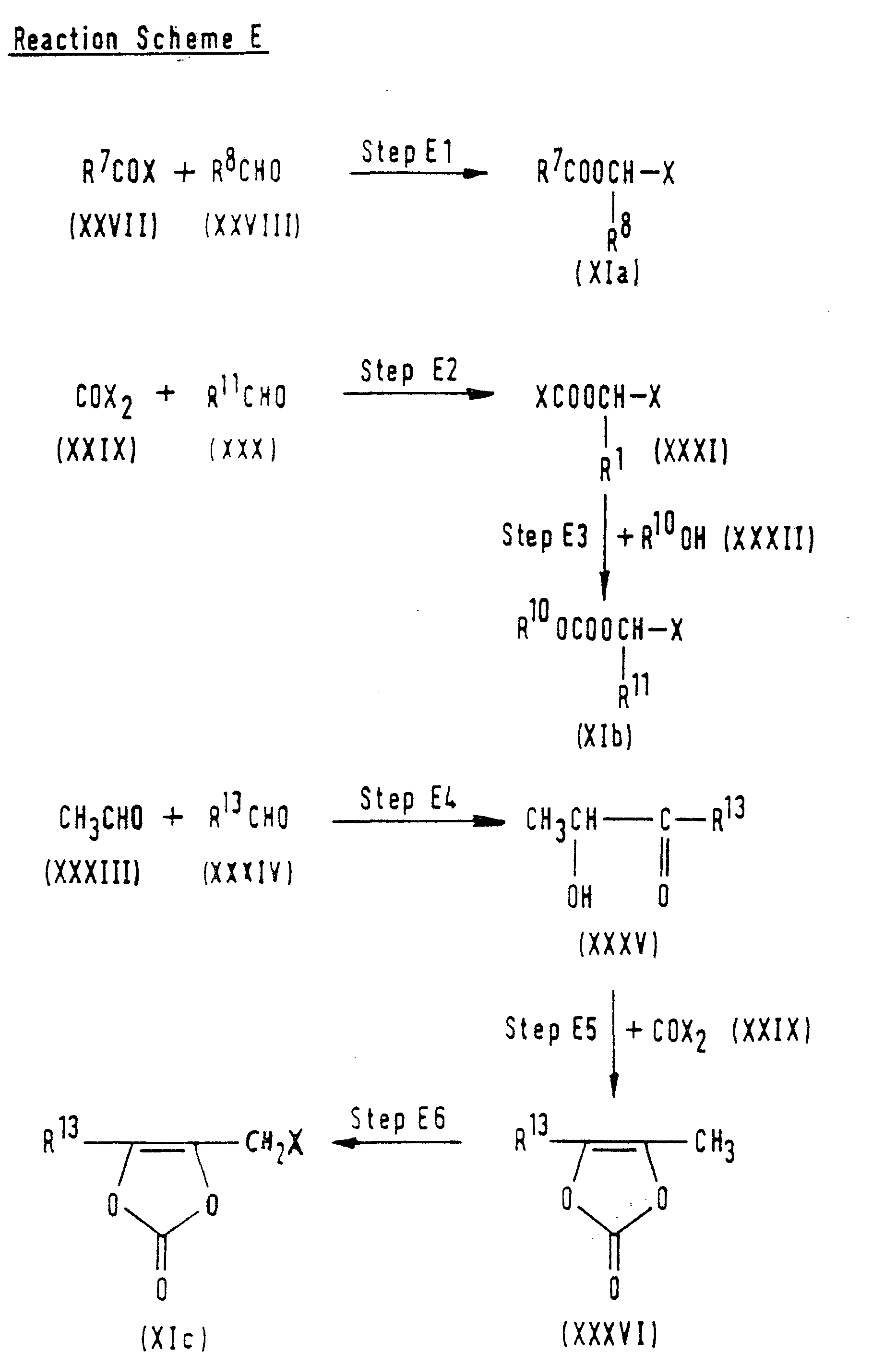 in which R⁷, R⁸, R¹⁰, R¹¹, R¹³ and X are as defined above, and the different groups X may be the same or different.

Alternatively, they may be obtained commercially.

In more detail, in Step E1, the active derivative of formula R⁷COX (XXVII) of a carboxylic acid of formula R⁷COOH and an aldehyde compound of formula R⁸CHO (XXVIII) are reacted by a conventional method in a solvent and in the presence of a base to prepare the compound of formula (Xla).

In Step E2, a phosgene derivative of formula COX₂ (XXIX) and an aldehyde compound of formula R 11 CHO (XXX) are reacted by a conventional method in a solvent and in the presence of a base, to produce a compound of formula (XXXI). In Step E3, the resulting reaction mixture is further reacted with an alcoholic compound of formula R^{IO}OH (XXXII) by a conventional method to prepare the compound of formula (Xlb).

In Step E4, according to a known method [e.g. the method described in Chemical Pharmaceutical Bulletin, 32, pages 4316 and 22418 (1984) or 33, page 4870 1985)], an aldehyde compound of formula R¹³CHO (XXXIV) and acetaldehyde (XXXIII) are subjected to an acyloin condensation by a conventional method and the resulting product of formula (XXXV) is reacted in Step E5 with a phosgene derivative (XXIX) in a solvent. The methyl group in the product of formula (XXXVI) is halogenated in Step E6 by a radical reaction with a halogen-containing compound by a conventional method and, if desired, the halogen atom is replaced by another X group to prepare the compound of formula (Xlc).

The compounds of formula (I) of the present invention are preferably administered in the form of a conventional pharmaceutical composition for ophthalmological use and suitable for local application to the eye, for example in the form of solutions, suspensions, gels, ointments or solid inserts. These compositions preferably contain the compound of the invention in an amount of from 0.01 to 10⁰/o, more preferably from 0.1 to 50/0, by weight. The composition may contain not only the compound of the invention as a single component but may also, if desired, contain a β-blocker such as timolol maleate or a parasympathetic nerve stimulator such as pilocarpine in addition.

The pharmaceutical composition of the present invention containing the active ingredient is preferably used in admixture with a non-toxic pharmaceutically acceptable inorganic or organic carrier. Typical phamaceuti- cally acceptable carriers include: water; a mixture of a water-miscible solvent, such as a lower alkanol or aralkanol, with water; a vegetable oil; a polyalkylene glycol; a jelly based on petroleum; ethylcellulose; ethyl oleate; carboxymethylcellulose; polyvinylpyrrolidone; isopropyl myristate; or any other pharmaceutically acceptable carrier commonly used for this purpose. The pharmaceutical composition may contain, if necessary, other non-toxic additives, as is well known in the art, for example: emulsifiers, antiseptics, wetting agents, and vehicles. Examples include: polyethylene glycol 200, 300, 400 and 600; Carbowax 1000, 1,500, 4,000 6,000 and 10,000; quarternary ammonium compounds known to be bactericidal at low temperature and which are non-toxic; antibacterial agents such as phenylmercury salts; buffer components such as sodium chloride, sodium borate and sodium acetate; thimerosal; methyl- or propylparaben; benzyl alcohol; phenylethanol; gluconate buffer; sorbitan monolaurate; triethanolamine; polyoxyethylenesorbitan monopalmitate; dioctyl sodium sulphosuccinate; monothioglycerol; thiosorbitol; and ethylenediamine tetraacetate. In addition, any suitable vehicle for ophthalmological use may be employed as a vehicle in this invention. Such vehicles include in general phosphate buffer vehicles, isotonic boric acid vehicles, istonic sodium chloride vehicles and isotonic sodium borate vehicles.

The pharmaceutical composition may, if desired, have either the form of solid insert that can remain nearly intact after administration, or the form of bio-degradable insert that is soluble in lacrymal fluids or which can be degraded in any other way.

In general, for an adult human patient, a daily dose of the compound of the present invention of from 0.001 to 50 mg/kg, preferably from 0.01 to 20 mg/kg, is employed. Depending on the daily dosage required, it may be administered in a single dose or in divided doses.

The invention is further illustrated by: the following Examples, which illustrate the preparation of the compounds of the present invention; the subsequent Preparations, which illustrate the preparation of ophthalmic compositions; and the subsequent Experiments, which illustrate the biological activity of the compounds of the present invention. In the Examples, the nuclear magnetic resonance spectra are measured at 270 MHZ.

### EXAMPLE 1

### 9'-Pivaloyloxymethyl griseolate

5 g of griseolic acid were dissolved in 15 ml of dimethyl sulphoxide, and then 2.17 ml of 1,8-diaza bicyclo[5.4.0]-7-undecene were added under a stream of nitrogen. 15 ml of acetonitrile and then 3.83 g of iodomethyl pivalate were added to the resulting mixture whilst ice-cooling, and then the mixture was allowed to react at room temperature for five hours. At the end of this time, the acetonitrile was removed by evaporation under reduced pressure, and the residue was dissolved in 300 ml of ethyl acetate and 300 ml of water. A cold 10°/o w/v aqueous solution of sodium bicarbonate was then added to the resulting solution to cause the aqueous layer to separate. The ethyl acetate layer was then further extracted with 100 ml of water, and the aqueous extract and the previously separated aqueous layer were combined. The combined aqueous solution was adjusted to a pH value of 3.1 by the addition of 1N aqueous hydrochloric acid, and then the solution was purified by chromatography through a prepacked column Rp-8 (available from Merck & Co., Ltd.), eluted with water containing successively 5%, 100/o and 20% by volume of acetonitrile. Fractions containing the desired compound were collected and lyophilized to give 688 mg (yield 10.6%) of the title compound.

Nuclear Magnetic Resonance Spectrum (hexadeuterated dimethyl sulphoxide) 8 ppm:
8.33 (1H, singlet);
8.18 (1H, singlet);
6.49 (1H, singlet);
6.03 (1H, doublet of doublets, J = 2.4 & 4.9 Hz);
5.08 (1H, doublet, J = 2.4 Hz);
4.58 (2H, multiplet);
7.40 (2H, broad singlet);
6.26 (1H, doublet, J = 4.9 Hz);
5.73 (2H, singlet);
1.16 (9H, singlet).

### EXAMPLE 2

### 9'-[1-(Isopropoxycarbonyloxy)ethyl] griseolate

2.0 g of griseolic acid were dissolved in 7 ml of dimethyl sulphoxide, and then 0.87 ml of 1,8-diazabicyclo[5.4.0]-7-undecene was added to the resulting solution under a stream of nitrogen. 5 ml of acetonitrile and then 1.33 g of 1-bromoethyl isopropoxycarboxylate were added to the resulting mixture whilst ice-cooling, and the mixture was allowed to react at room temperature for three hours. At the end of this time, the procedures described in Example 1 were repeated in an analogueous way, to give 740 mg (yield 18.5%) of the title compound as a lyophilized product.

Nuclear Magnetic Resonance Spectrum (hexadeuterated dimethyl sulphoxide) 8 ppm:
8.34 (1H, singlet);
8.18 (1H, singlet);
7.38 (2H, broad singlet);
6.6 (1H, multiplet);
6.49 (1H, singlet);
6.29 and 6.25 (1H, each doublet, J = 4.9 Hz);
6.06 (1H, doublet of doublets, J = 2.4 & 4.9 Hz);
5.5 (1H, multiplet);
5.08 (1H, doublet, J = 2.4 Hz);
4.59 (2H, multiplet);
1.2-1.5 (9H, multiplet).

### EXAMPLE 3

### 9'-(5-Methyl-2-oxo-1,3-dioxolen-4-yl)methyl griseolate

A procedure similar to that described in Example 1 was repeated, except that 3 g of griseolic acid was used and the iodomethyl pivalate was replaced by 7.6 g of (5-methyl-2-oxo-1,3-dioxoten-4-yl)methyl iodide, to give 0.14 g of the title compound.

Nuclear Magnetic Resonance Spectrum (hexadeuterated dimethyl sulphoxide) δ ppm:
8.32 (1H, singlet);
8.16 (1H, singlet);
7.36 (2H, broad singlet);
6.43 (1H, singlet);
5.97 (1H, doublet of doublets, J = ₂.0 & 4.9 Hz);
5.00 (2H, singlet);
4.95 (1H, doublet, J = 2.0 Hz);
4.44 (1H, doublet, J = 4.9 Hz);
4.03 (1H, broad singlet);
2.17 (3H, singlet).

### EXAMPLE 4

### 9'-(1-Isovaleryloxyethyl) griseolate

500 mg of griseolic acid, followed by 0.24 ml of 1,8-diazabicyclo[5.4.0]-7-undecene, were dissolved in 2.5 ml of dimethyl sulphoxide under a stream of nitrogen. 2 ml of acetonitrile and then 1.01 g of 1-iodoethyl isovalerate were then added to the resulting mixture, whilst ice-cooling. The mixture was then allowed to react at room temperature for three to four hours, after which it was left for stand at 5°C for 16 hours. At the end of this time, the acetonitrile was removed by evaporation under reduced pressure, and the residue was dissolved in 15 ml of dilute hydrochloric acid and 20 ml of ethyl acetate. The ethyl acetate layer was then separated. The residual aqueous layer was saturated with sodium chloride and then extracted twice with ethyl acetate. The ethyl acetate layer and the ethyl acetate extract were combined, and the resulting solution was extracted twice with a cold 5% w/v aqueous solution of sodium bicarbonate. The aqueous layers were combined and adjusted to a pH value of 2.8 by the addition of 1 N aqueous hydrochloric acid. The resulting aqueous solution was purified by chromatography through a prepacked column Rp-8 (available from Merck & Co., Ltd.), eluted with water containing successively 10%, 150/o and 20% v/v acetonitrile. Fractions containing the title compound were collected and lyophilized to give 137 mg (yield 19.0%) of the title compound.

Nuclear Magnetic Resonance Spectrum (hexadeuterated dimethyl sulphoxide) 8 ppm:
8.34 (1H, singlet);
8.18 (1H, singlet);
7.40 (2H, broad singlet);
6.79 (1H, multiplet);
6.49 (1H, singlet);
6.32 and 6.25 (1H, each doublet, J = 5.2 Hz);
6.06 (1H, doublet of doublets, J = 2.4 & 4.9 Hz);
5.50 (1H, broad singlet);
5.08 (1 H, doublet, J = 2.0 Hz);
4.57 (1H, doublet, J = 5.2 Hz);
4.56 (1H, singlet);
2.20 (2H, doublet, J = 7.0 Hz);
2.00 (1H, multiplet);
1.41 (3H, multiplet);
0.91 (6H, multiplet).

### EXAMPLE 5

### 9'-(1-Pivaloyloxyethyl) griseolate

A procedure similar to that described in Example 4 was repeated, except that 500 mg of griseolic acid were used and that the 1-iodoethyl isovalerate was replaced by 0.68 g of 1-iodoethyl pivalate to give 98 mg of the title compound.

Nuclear Magnetic Resonance Spectrum (hexadeuterated dimethyl sulphoxide) 8 ppm:
8.34 (1H, singlet);
8.18 (1H, singlet);
7.40 (2H, broad singlet);
6.74 (1H, multiplet);
6.49 (1H, singlet);
6.31 and 6.25 (1H, each doublet, J = 4.9 Hz);
5.49 (1H, broad multiplet);
6.05 (1H, doublet of doublets, J = 2.4 & 4.9 Hz);
5.08 (1H, doublet, J = 2.4 Hz);
4.57 (1H, doublet, J = 4.9 Hz);
4.58 (1H, singlet);
1.42 (3H, multiplet);
1.14 (9H, multiplet).

### EXAMPLE 6

### 9'-(1-Isobutyryloxypropyl) griseolate

A procedure similar to that described in Example 4 was repeated, except that 500 mg of griseolic acid were used and that the 1-iodoethyl isovalerate was replaced by 676 mg of 1-iodopropyl isobutyrate, to give 100 mg of the title compound.

Nuclear Magnetic Resonance Spectrum (hexadeuterated dimethyl sulphoxide) δ ppm:
8.34 (1H, singlet);
8.18 (1H, singlet);
7.40 (2H, broad singlet);
6.67 (1 H, multiplet);
6.48 (1H, singlet);
6.31 and 6.23 (1H, each doublet, J = 4.4 Hz);
6.06 (1H, doublet of doublets J = 2.4 & 4.9 Hz);
5.07 (1H, doublet, J = 2.4 Hz);
4.58 (2H, broad singlet);
2.5 (1H, multiplet, overlapping with DMSO resonance);
1.7 (1H, multiplet);
1.0 - 1.1 (6H, multiplet);
0.89 (3H, multiplet).

### EXAMPLE 7

### 9'-(1-Butyryloxyethyl) griseolate

A procedure similar to that described in Example 4 was repeated, except that 500 mg of griseolic acid were used and that the 1-iodoethyl isovalerate was replaced by 0.64 g of 1-iodoethyl butyrate, to give 41 mg of the title compound.

Nuclear Magnetic Resonance Spectrum (hexadeuterated dimethyl sulphoxide) 8 ppm:
8.33 (1H, singlet);
8.19 (1H, singlet);
7.39 (2H, broad singlet);
6.77 (1H, multiplet);
6.47 (1H, singlet);
6.32 and 6.22 (1H, each broad doublet);
6.03 (1H, doublet of doublets, J = 2.9 & 5.4 Hz);
5.08 (1H, broad doublet);
4.54 (2H, multiplet);
2.29 (2H, triplet, J = 7.3 Hz);
1.54 (2H, multiplet);
1.42 (3H, multiplet);
0.93 (3H, multiplet).

### EXAMPLE 8

### 9'-(1-Cyclohexylcarbonyloxyethyl) griseolate

A procedure similar to that described in Example 4 was repeated, except that 500 mg of griseolic acid were used and that the 1-iodoethyl isovalerate was replaced by 0.74 g of 1-iodoethyl cyclohexylcarboxylate to give 24 mg of the title compound.

Nuclear Magnetic Resonance Spectrum (hexadeuterated dimethyl sulphoxide) 8 ppm:
8.35 (1H, singlet);
8.19 (1H, singlet);
7.45 (2H, broad singlet);
6.76 (1H, multiplet);
6.50 (1H, singlet);
6.30 and 6.36 (1H, each doublet, J = 4.9 Hz);
6.06 (1H, doublet of doublets, J = 2.4 & 4.9 Hz);
5.08 (1H, doublet, J = 2.0 Hz);
4.58 (2H, multiplet);
2.33 (1H, multiplet);
1.1 - 1.9 (13H, multiplet).

### EXAMPLE 9

### 9'-(1-Pivaloyloxyethyl) T-desoxygriseolate

500 mg of 7'-desoxygriseolic acid were dissolved in 4 ml of dimethyl sulphoxide, and then 0.24 ml of 1,8-diazabicyclo[5.4.0]-7-undecene were added to the resulting solution, under a stream of nitrogen. 4 ml of acetonitrile and then 693 mg of 1-iodoethyl pivalate were added to the resulting mixture, whilst ice-cooling. The resulting mixture was left for stand at room temperature for 2.5 hours and then at 5°C for 16 hours. At the end of this time, the acetonitrile was removed by evaporation under reduced pressure, and the residue was dissolved in a mixture of 70 ml of ethyl acetate and 70 ml of a 3% w/v aqueous solution of sodium bicarbonate containing ice. The aqueous layer was separated and then the ethyl acetate layer was extracted with 30 ml of water. The aqueous layer was combined with the aqueous extract, and the resulting mixture was adjusted to a pH value of 2.9 by the addition of 1 N aqueous hydrochloric acid. The mixture was then treated with activated carbon. The treated mixture was purified by chromatography through a prepacked column Rp-8 (available from Merck & Co., Ltd.), eluted with water containing successively 100/₀, 200/o and 30% v/v acetonitrile. Fractions containing the desired compound were collected and lyophilized to give 18 mg of the title compound.

Nuclear Magnetic Resonance Spectrum (a 1 : 1 by volume mixture of CD₃CN + D₂0) δ ppm:
8.19 (1H, singlet);
8.08 (1H, singlet);
6.77 (1 H, quartet, J = 5.5 Hz);
6.43 (1H, singlet);
5.81 (1H, doublet of doublets, J = 2.6 & 4.8 Hz);
5.00 (1 H, doublet, J = 2.6 Hz);
4.64 (1H, doublet, J = 4.8 Hz);
3.00 (1H, doublet, J = 16.1 Hz);
2.67 (1H, doublet, J = 16.1 Hz);
1.48 (3H, doublet, J = 5.5 Hz);
1.03 (9H, singlet).

### EXAMPLE 10

### 8'-Pivaloyloxyrnethyl griseolate

10(a) 1 g of griseolic acid and 2.6 g of molecular sieve 4A were suspended in 26 ml of acetone. 0.83 ml of trifluoromethanesulphonic acid was added, whilst ice-cooling, to the resulting suspension, and the mixture was allowed to react for two hours. At the end of this time, 0.35 g of calcium hydroxide was added to the reaction mixture, the mixture was stirred for 10 minutes; and then 0.94 g of calcium carbonate was added and the resulting mixture was stirred for a further 10 minutes. At the end of this time, the insolubles were filtered off and the solvent was removed by evaporation under reduced pressure, to give 7',9'-O-isopropylidenegriseolic acid.

10(b) The whole of the 7',9'-O-isopropylidenegriseolic acid obtained as described in step (a) above was dissolved in 5 ml of dimethyl sulphoxide, and then 5 ml of acetonitrile, followed by 0.12 ml of 1,8-diazabicyclo[5.4.0]-7-undecene, were added to the resulting solution. 2.6 g of 1-iodomethyl pivalate were then added to the resulting mixture, whilst ice-cooling, and the mixture was allowed to react at room temperature for three hours. At the end of this time, the acetonitrile was removed by evaporation under reduced pressure, and the residue was dissolved in 70 ml of ethyl acetate and 70 ml of water. The organic layer was then separated, washed twice with water, and then dried over anhydrous magnesium sulphate. The solvent was then removed by evaporation under reduced pressure, and the residue was separated by silica gel column chromatography (using methylene chloride containing 3⁰/₀ v/v ethanol as eluent), to give 1.4 g (yield 99%) of 8'-pivaloyloxymethyl 7,9'-O-isopropylidene griseolate.

Nuclear Magnetic Resonance Spectrum (hexadeuterated dimethyl sulphoxide) δ ppm:
8.31 (1 H, singlet);
8.19 (1H, singlet);
7.39 (2H, broad singlet);
6.54 (1H, singlet);
6.08 (1H, doublet of doublets, J = 2.4 & 5.4 Hz);
6.05 (1 H, doublet, J = 4.4 Hz)
5.83 (1 H, doublet, J = 5.9 Hz)
5.80 (1H, doublet, J = 5.9 Hz);
5.21 (1H, doublet, J = 2.4 Hz);
4.96 (1H, singlet);
4.67 (1H, triplet, J = 4.4 Hz);
1.56 (6H, singlet);
1.07 (9H, singlet).

10(c) 240 mg of the 8'-pivaloyloxymethyl 7'9'-O-isoprooylidene griseolate obtained as described in step (b) above were dissolved, whilst ice-cooling, in 5 ml of trifluoroacetic acid containing 1⁰/₀ v/v water, and the mixture was allowed to react at room temperature for five hours. At the end of this time, the solvent was removed by evaporation under reduced pressure, and the residue was dissolved in a 30/₀ w/v aqueous solution of sodium bicarbonate, whilst ice-cooling. The resulting mixture was adjusted to a pH value of 2.3 by the addition of 1N aqueous hydrochloric acid and eluted through a prepacked column Rp-8 (available from Merck & Co., Ltd.) with water containing successively 10% and 20% v/v of acetonitrile. Fractions containing the desired compound were collected and lyophilized to give 213 mg (yield 95.9%) of the title compound.

Nuclear Magnetic Resonance Spectrum (hexadeuterated dimethyl sulphoxide) 8 ppm:
8.33 (1H, singlet);
8.31 (1H, singlet);
7.39 (2H, broad singlet);
6.50 (1H, singlet);
6.08 (1H, doublet of doublets, J = 2.4 & 4.9 Hz);
5.79 (1H, doublet, J = 5.9 Hz);
5.76 (1H, doublet, J = 5.9 Hz);
5.05 (1H, doublet, J = 2.4 Hz);
4.61 (1H, doublet, J = 4.9 Hz);
4.46 (1H, singlet);
1.10 (9H, singlet).

### EXAMPLE 11

### 8'-(1-Isovaleryloxyethyl) griseolate

In the purification procedure described in Example 4, the chromatography column was eluted with water containing 20% v/v acetonitrile. Following elution of the compound described in Example 4, the title compound of the present Example was eluted. These fractions were collected and lyophilized to give 22 mg of the title compound.

Nuclear Magnetic Resonance Spectrum (hexadeuterated dimethyl sulphoxide) δ ppm:
8.36 (1H, singlet);
8.21 (1H, singlet);
7.51 (2H, broad singlet);
6.81 (1H, quartet, J = 5.4 Hz);
6.50 (1H, singlet);
6.25 (1H, broad singlet);
6.08 (1H, doublet of doublets, J = 2.4 & 4.9 Hz);
5.37 (1H, broad singlet);
4.99 (1H, doublet, J = 2.4 Hz);
4.61 (1H, broad doublet);
4.46 (1H, singlet);
2.18 (2H, multiplet);
1.95 (1H, multiplet);
1.43 (3H, doublet, J = 5.4 Hz);
0.86 (6H, multiplet).

### EXAMPLE 12

### 8'-(1-Pivaloyloxyethyl) griseolate

In the purification procedure described in Example 5, the chromatography column was eluted with water containing 20% v/v acetonitrile. Following elution of the compound described in Example 5, the title compound of the present Example was eluted. These fractions were collected and lyophilized to give 20 mg of the title compound.

Nuclear Magnetic Resonance Spectrum (hexadeuterated dimethyl sulphoxide) δ ppm:
8.36 (1H, singlet);
8.20 (1H, singlet);
7.56 (2H, broad singlet);
6.78 (1H, quartet, J = 5.4 Hz);
6.52 (1H, singlet);
6.26 (1H, broad singlet);
6.08 (1H, doublet of doublets, J = ₂.₄ & 4.9 Hz);
5.30 (1H, broad singlet);
5.00 (1H, doublet, J = 2.4 Hz);
4.59 (1H, doublet, J = 4.9 Hz);
4.48 (1H, singlet);
1.44 (3H, doublet, J = 5.4 Hz);
1.11 (9H, singlet).

### EXAMPLE 13

### 8'-(1-Isobutyryloxypropyl) griseolate

In the purification procedure described in Example 6, the chromatography column was eluted with water containing 20% v/v acetonitrile. Following elution of the compound described in Example 6, the title compound of the present Example was eluted. These fractions were collected and lyophilized to give 20 mg of the title compound.

Nuclear Magnetic Resonance Spectrum (hexadeuterated dimethyl sulphoxide) 8 ppm:
8.33 (1 H, singlet);
8.17 (1H, singlet);
7.41 (2H, broad singlet);
6.68 (1H, triplet, J = 5.4 Hz);
6.51 (1H, singlet);
6.21 (1H, broad singlet);
6.12 (1H, doublet of doublets, J = ₂.₄ & 4.9 Hz);
5.36 (1H, broad singlet);
5.02 (1H, doublet, J = 2.0 Hz);
4.59 (1H, doublet, J = 4.9 Hz);
4.49 (1H, singlet);
2.50 (2H, multiplet, overlapping with DMSO resonance);
1.08 (6H, multiplet);
0.91 (3H, triplet, J = 7.3 Hz).

### EXAMPLE 14

### 8'-(1-Butyryloxyethyl) griseolate

In the purification procedure described in Example 7, the chromatography column was eluted with water containing 20% v/v acetonitrile. Following elution of the compound described in Example 7, the title compound of the present Example was eluted. These fractions were collected and lyophilized to give 15 mg of the title compound.

Nuclear Magnetic Resonance Spectrum (hexadeuterated dimethyl sulphoxide) δ ppm:
8.38 (1H, singlet);
8.22 (1H, singlet);
7.58 (2H, broad singlet);
6.81 (1 H, quartet, J = 5.9 Hz);
6.51 (1H, singlet);
6.28 (1H, broad singlet);
6.08 (1 H, doublet of doublets, J = 2.4 & 4.9 Hz);
5.38 (1H, broad singlet);
5.02 (1H, doublet, J = 2.4 Hz);
4.60 (1H, doublet, J = 4.9 Hz);
4.48 (1H, singlet);
2.29 (2H, triplet, J = 7.3 Hz);
1.52 (2H, quartet, J = 7.3 Hz);
1.43 (3H, doublet, J = 5.4 Hz);
0.85 (3H, triplet, J = 7.3 Hz).

### EXAMPLE 15

### 8'-(1-Cyclohexylcarbonyloxyethyl) griseolate

In the purification procedure described in Example 8, the chromatography column was eluted with water containing 20% v/v acetonitrile. Following elution of the compound described in Example 8, the title compound of the present Example was eluted. These fractions were collected and lyophilized to give 22 mg of the title compound.

Nuclear Magnetic Resonance Spectrum (hexadeuterated dimethyl sulphoxide) δ ppm:
8.34 (1H, singlet);
8.18 (1H, singlet);
7.41 (2H, broad singlet);
6.79 (1H, quartet, J = 5.4 Hz);
6.50 (1H, singlet);
6.21 (1H, broad singlet);
6.11 (1H, doublet of doublets, J = 2.4 & 4.9 Hz);
5.34 (1H, broad singlet);
5.00 (1H, doublet, J = 2.0 Hz);
4.60 (1H, doublet, J = 5.4 Hz);
4.46 (1H, singlet);
2.30 (1H, multiplet);
1.1 - 1.8 (13H, multiplet).

### EXAMPLE 16

### 8'-(1-Pivaloyloxyethyl) T-desoxygriseolate

In the purification procedure described in Example 9, the chromatography column was eluted with water containing 20% v/v acetonitrile. Following elution of the compound described in Example 9, the title compound of the present Example was eluted. These fractions were collected and lyophilized to give 15 mg of the title compound.

Nuclear Magnetic Resonance Spectrum (hexadeuterated dimethyl sulphoxide) δ ppm:
8.31 (1H, singlet);
8.16 (1H, singlet);
7.40 (2H, broad singlet);
6.76 (1H, quartet, J = 5.4 Hz);
6.46 (1H, singlet);
6.05 (1H, doublet of doublets, J = 2.4 & 4.9 Hz);
5.97 (1H, doublet, J = 3.9 Hz);
4.98 (1H, doublet, J = 2.4 Hz);
4.56 (1H, triplet, J = 4.4 Hz);
3.11 (1H, doublet, J = 17.1 Hz);
2.77 (1H, doublet, J = 17.1 Hz);
1.43 (3H, doublet, J = 5.4 Hz);
1.10 (9H, singlet).

### EXAMPLE 17

### 9'-(1-Isopropoxycarbonyloxyethyl) 7'-pivaloylgriseolate

A procedure similar to that described in Example 2 was repeated, except that 46 mg of 7'-pivaloylgriseolic acid was used in place of the griseolic acid to give 13 mg of the title compound.

Nuclear Magnetic Resonance Spectrum (hexadeuterated dimethyl sulphoxide) 8 ppm:
8.36 (1H, singlet);
8.17 (1H, singlet);
7.40 (2H, broad singlet);
6.49 (1H, singlet);
6.46 (1H, multiplet);
6.05 (1H, doublet of doublets, J = 2.4 & 4.9 Hz);
5.52 and 5.63 (1H, each singlet);
4.98 and 5.03 (1H, each doublet, J = 2.4 Hz);
4.79 (1H, multiplet);
4.61 (1H, doublet of doublets, J = 4.4 & 4.9 Hz);
1.44 (3H, doublet, J = 5.4 Hz);
1.1 - 1.3 (15H, multiplet).

### EXAMPLE 18

### 18(a) 8'-Benzhydryl griseolate

3.38ml of trifluoromethanesulphonic acid were added dropwise, whilst ice-cooling, to a suspension of 4.84 g of griseolic acid and 12 g of molecular sieve 4A in 120 ml of acetone, and the mixture was then stirred for 1.5 hours at room temperature. At the end of this time, 1.41 g of calcium hydroxide were added to the reaction mixture, the mixture was stirred for 15 minutes, and insoluble materials were removed by filtration. 3.8 g of calcium carbonate were added to the filtrate, the mixture was stirred for 20 minutes, and then neutralized, after which insoluble materials were removed by filtration. 3.7 g of diphenyldiazomethane were added to the filtrate, and the mixture was stirred at room temperature for 2 hours. At the end of this time, the solvent was removed by evaporation under reduced pressure, and the residue was dissolved in a mixture of 70 ml of methanol and 30 ml of water and heated, with stirring, for 1 hour. The crystals which deposited were collected by filtration, washed with cold methanol, and dried to afford 5.18 g of the title compound.

Nuclear Magnetic Resonance Spectrum (hexadeuterated dimethyl sulphoxide) 8 ppm:
8.31 (1H, singlet);
8.12 (1H, singlet);
7.45-7.23 (10H, multiplet);
6.81 (1 H, singlet);
6.52 (1H, singlet);
6.20 (1H, doublet of doublets, J = 2.0 & 4.9 Hz);
5.20 (1H, doublet, J = 2.0 Hz);
4.61 (1H, doublet, J = 4.9 Hz);
4.61 (1H, singlet).

### 18(b) 9'-(3-Acetoxybenzyl) 8'-benzhydryl griseolate

230 mg of 3-acetoxybenzyl bromide were added to a solution of 380 mg of 8'-benzhydryl griseolate [prepared as described in step 18(a) above] and 130 mg of 1,5-diazabicyclo[4.3.0]-5-nonene in 3 ml of dimethylformamide, and the mixture was stirred at room temperature overnight. At the end of this time, the reaction mixture was extracted with ethyl acetate, the extract was washed in turn with water, a saturated aqueous solution of sodium bicarbonate and a saturated aqueous solution of sodium chloride, and dried over anhydrous magnesium sulphate, and the solvent was removed by distillation under reduced pressure. The residue was purified by column chromatography (through silica gel; eluted with 2% v/v methanol in methylene chloride) to afford 215 mg of the title compound, melting at 109-111°C.

Nuclear Magnetic Resonance Spectrum (CDCℓ₃) δ ppm:
8.32 (1H, singlet);
8.12 (1H, singlet);
7.41 -7.02 (14H, multiplet);
6.71 (1 H, singlet);
6.53 (1H, singlet);
6.23 (1 H, doublet of doublets, J = 2.4 & 5.3 Hz);
5.25 (1H, doublet, J = 2.4 Hz);
5.04 (1H, doublet, J = 12.7 Hz);
4.94 (1H, doublet, J = 12.7 Hz);
4.78 (1H, singlet);
4.62 (1H, doublet, J = 5.3 Hz);
2.27 (3H, singlet).

### 18(c) 9'-(3-Acetoxybenzyl) griseolate

86 mg of boron trifluoride etherate were added to a solution of 208 mg of 9'-(3-acetoxybenzyl) 8'-benzhydryl griseolate [prepared as described in step 18(b) above] in 2 ml of acetic acid, and the mixture was stirred at room temperature for 1.5 hours. At the end of this time, 20 ml of diethyl ether were added to the reaction mixture, and the crystalline compound which deposited was collected by filtration. This compound was suspended and stirred in a layer each of water and of ethyl acetate, collected again by filtration, and dried to afford 85 mg of the title compound.

Nuclear Magnetic Resonance Spectrum (hexadeuterated dimethyl sulphoxide) 8 ppm:
8.33 (1H, singlet);
8.18 (1H, singlet);
7.41 -7.13 (4H, multiplet);
6.49 (1H, singlet);
6.06 (1H, doublet of doublets, J = 2.4 & 4.9 Hz);
5.20 (1H, doublet, J = 12.7 Hz);
5.10 (1H, doublet, J = 12.7 Hz);
5.09 (1H, doublet, J = 2.4 Hz);
4.66 (1H, singlet);
4.58 (1H, doublet, J = 4.9 Hz);
2.26 (3H, singlet).

### EXAMPLE 19

### 19(a) 9'-(4-Acetoxybenzyl) 8'-benzhydryl griseolate

A procedure similar to that described in Example 18(b) was repeated, except that 230 mg of 4-acetoxybenzyl bromide were used instead of the 3-acetoxybenzyl bromide, and that 440 mg of 8'-benzhydryl griseolate, 130 mg of 1,5-diazabicyclo[4.3.0]-5-nonene and 6 ml of dimethylformamide were used, to prepare 380 mg of the title compound.

Nuclear Magnetic Resonance Spectrum (CDCℓ₃) 8 ppm:
8.32 (1H, singlet);
8.13 (1H, singlet);
7.42 - 7.04 (14H, multiplet);
6.66 (1H, singlet);
6.53 (1H, singlet);
6.25 (1H, doublet of doublets, J = 2.4 & 5.3 Hz);
5.24 (1H, doublet, J = 2.4 Hz);
5.03 (1H, doublet, J = 12.7 Hz);
4.93 (1H, doublet, J = 12.7 Hz);
4.78 (1H, singlet);
4.64 (1H, doublet, J = 5.3 Hz);
2.28 (3H, singlet).

### 19(b) 9'-(4-Acetoxybenzyl) griseolate

Following a procedure similar to that described in Example 18(c), but using 1.2 g of 9'-(4-acetoxybenzyl) 8'-benzhydryl griseolate [prepared as described in step 19(a) above], 850 mg of boron trifluoride etherate and 10 ml of acetic acid, 640 mg of the title compound were prepared.

Nuclear Magnetic Resonance Spectrum (hexadeuterated dimethyl sulphoxide) δ ppm:
8.34 (1H, singlet);
8.21 (1H, singlet);
7.42 (2H, doublet, J = 8.8 Hz);
7.13 (2H, doublet, J = 8.8 Hz);
6.49 (1H, singlet);
6.07 (1H, doublet of doublets, J = 2.2 & 4.9 Hz);
5.19 (1H, doublet, J = 12.7 Hz);
5.08 (1H, doublet, J = 12.7 Hz);
5.10 (1H, doublet, J = 2.2 Hz);
4.67 (1H, singlet);
4.61 (1H, doublet, J = 4.9 Hz);
2.27 (3H, singlet).

### EXAMPLE 20

### 20(a) 9'-(2-Acetoxybenzyl) 8'-benzhydryl griseolate

A procedure similar to that described in Example 18(b) was repeated, except that 230 mg of 2-acetoxybenzyl bromide were used instead of the 3-acetoxybenzyl bromide, and that 440 mg of 8'-benzhydryl griseolate, 130 mg of 1,5-diazabicyclo[4.3.0]-5-nonene and 5 ml of dimethylformamide were used, to prepare 380 mg of the title compound, melting at 120-122°C.

Nuclear Magnetic Resonance Spectrum (CDCℓ₃) 8ppm:
8.32 (1H, singlet);
8.12 (1H, singlet);
7.44 - 7.12 (14H, multiplet);
6.75 (1H, singlet);
6.53 (1H, singlet);
6.24 (1H, doublet of doublets, J = 2.4 & 5.3 Hz);
5.24 (1H, doublet, J = 2.4 Hz);
5.03 (1H, doublet, J = 12.7 Hz);
4.85 (1H, doublet, J = 12.7 Hz);
4.73 (1H, singlet);
4.62 (1H, doublet, J = 5.3 Hz);
2.22 (3H, singlet).

### 20(b) 9'-(2-Acetoxybenzyl) griseolate

Following a procedure similar to that described in Example 18(c), but using 208 mg of 9'-(2-acetoxybenzyl) 8'-benzhydryl griseolate [prepared as described in step 20(a) above], 150 mg of boron trifluoride etherate and 5 ml of acetic acid, 85 mg of the title compound were prepared.

Nuclear Magnetic Resonance Spectrum (hexadeuterated dimethyl sulphoxide) 8 ppm:
8.32 (1H, singlet);
8.20 (1H, singlet);
7.51 - 7.14 (14H, multiplet);
6.48 (1H, singlet);
6.04 (1 H, doublet of doublets, J = 2.4 & 4.9 Hz);
5.14 (1H, doublet, J = 12.7 Hz);
4.99 (1H, doublet, J = 12.7 Hz);
5.11 (1H, doublet, J = 2.4 Hz);
4.61 (1H, singlet);
4.59 (1H, doublet, J = 4.9 Hz);
2.29 (3H, singlet).

### EXAMPLE 21

### 21 (a) 9'-(4-Pivaloyloxybenzyl) 8'-benzhydryl griseolate

A procedure similar to that described in Example 18(b) was repeated, except that 406 mg of 4-pivaloyloxybenzyl bromide were used instead of the 3-acetoxybenzyl bromide, and that 545 mg of 8'-benzhydryl griseolate, 186 mg of 1,5-diazabicyclo[4.3.0]-5-nonene and 5 ml of dimethylformamide were used, to prepare 620 mg of the title compound.

Nuclear Magnetic Resonance Spectrum (CDCℓ₃) δ ppm:
8.28 (1H, singlet);
7.81 (1H, singlet);
7.33 - 6.92 (14H, multiplet);
6.81 (1H, singlet);
6.38 (1H, singlet);
6.18 (1H, doublet of doublets, J = 2.5 & 5.1 Hz);
5.13 (1H, doublet, J = 2.5 Hz);
5.05 (1H, doublet, J = 12.2 Hz);
4.90 (1H, doublet, J = 12.2 Hz);
4.89 (1H, singlet);
4.65 (1H, doublet, J = 5.1 Hz);
1.35 (9H, singlet).

### 21 (b) 9'-(4-Pivaloyloxybenzyl) griseolate

Following a procedure similar to that described in Example 18(c), but using 368 mg of 9'-(4-pivaloyloxybenzyl) 8'-benzhydryl griseolate [prepared as described in step 21 (a) above], 142 mg of boron trifluoride etherate and 4 ml of acetic acid, 95 mg of the title compound were prepared.

Nuclear Magnetic Resonance Spectrum (hexadeuterated dimethyl sulphoxide) δ ppm:
8.33 (1H, singlet);
8.19 (1H, singlet);
7.43 (2H, doublet, J = 8.8 Hz);
7.10 (2H, doublet, J = 8.8 Hz);
6.48 (1H, singlet);
6.04 (1H, doublet of doublets, J = ₂.₄ & 4.8 Hz);
5.18 (1H, doublet, J = 12.7 Hz);
5.07 (1H, doublet, J = 12.7 Hz);
5.09 (1H, doublet, J = 2.4 Hz);
4.64 (1H, singlet);
4.58 (1H, doublet, J = 4.8 Hz);
1.30 (9H, singlet).

### EXAMPLE 22

### 22(a) 9'-(3,4-Diacetoxybenzyl) 8'-benzhydryl griseolate

A procedure similar to that described in Example 18(b) was repeated, except that 430 mg of 3,4-diacetoxybenzyl bromide were used instead of the 3-acetoxybenzyl bromide, and that 545 mg of 8'-benzhydryl griseolate, 186 mg of 1,5diazabicyclo[4.3.0]-5-nonene and 4 ml of dimethylformamide were used, to prepare 230 mg of the title compound, melting at 124 -126°C.

Nuclear Magnetic Resonance Spectrum (CDCℓ₃) δ ppm:
8.28 (1H, singlet);
7.81 (1H, singlet);
7.33 - 7.01 (13H, multiplet);
6.88 (1H, singlet);
6.37 (1H, singlet);
6.12 (1H, doublet of doublets, J = 2.5 & 4.7 Hz);
5.13 (1H, doublet, J = 2.5 Hz);
4.96 (1H, doublet, J = 12.4 Hz);
4.87 (1H, doublet, J = 12.4 Hz);
4.90 (1H, singlet);
4.65 (1H, doublet, J = 4.7 Hz);
2.29 (3H, singlet);
2.28 (3H, singlet).

### 22(b) 9'-(3,4-Diacetoxybenzyl) griseolate

Following a procedure similar to that described in Example 18(c), but using 220 mg of 9'-(3,4-diacetoxybenzyl) 8'-benzhydryl griseolate [prepared as described in step 22(a) above], 85 mg of boron trifluoride etherate and 2 ml of acetic acid, 130 mg of the title compound were prepared.

Nuclear Magnetic Resonance Spectrum (hexadeuterated dimethyl sulphoxide) 8 ppm:
8.34 (1H, singlet);
8.19 (1H, singlet);
7.31 -7.24 (3H, multiplet);
6.49 (1H, singlet);
6.06 (1H, doublet of doublets, J = 2.4 & 4.9 Hz);
5.15 (1H, doublet, J = 14.6 Hz);
5.09 (1H, doublet, J = 14.6 Hz);
5.09 (1H, doublet, J = 2.4 Hz);
4.68 (1H, singlet);
4.58 (1H, doublet, J = 4.₉ Hz);
2.28 (3H, singlet);
2.27 (3H, singlet).

### EXAMPLE 23

### 23(a) 9'-(4-lsobutyryloxybenzyl) 8'-benzhydryl griseolate

A procedure similar to that described in Example 18(b) was repeated, except that 380 mg of 4-isobutyryloxybenzyl bromide were used instead of the 3-acetoxybenzyl bromide, and that 545 mg of 8'-benzhydryl griseolate, 186 mg of 1,5-diazabicyclo[4.3.0]-5-nonene and 3 ml of dimethylformamide were used, to prepare 190 mg of the title compound, melting at 193-195°C.

Nuclear Magnetic Resonance Spectrum (CDCℓ₃) δ ppm:
8.32 (1H, singlet);
8.12 (1H, singlet);
7.43-7.01 (14H, multiplet);
6.70 (1 H, singlet);
6.53 (1H, singlet);
6.23 (1H, doublet of doublets, J = 2.4 & 4.9 Hz);
5.25 (1H, doublet, J = 2.4 Hz);
5.01 (1H, doublet, J = 12.7 Hz);
4.95 (1H, doublet, J = 12.7 Hz);
4.77 (1H, singlet);
4.62 (1H, doublet, J = 4.9 Hz);
2.82 (1H, doublet of triplets, J = 6.8 Hz);
1.24 (6H, doublet, J = 6.8 Hz).

### 23(b) 9'-(4-lsobutyryloxybenzyl) griseolate

Following a procedure similar to that described in Example 18(c), but using 110 mg of 9'-(4-isobutyryloxybenzyl) 8'-benzhydryl griseolate [prepared as described in step 23(a) above], 56 mg of boron trifluoride etherate and 1 ml of acetic acid, 60 mg of the title compound were prepared.

Nuclear Magnetic Resonance Spectrum (hexadeuterated dimethyl sulphoxide) δ ppm:
8.43 (1H, singlet);
8.29 (1H, singlet);
7.42 (2H, doublet, J = 8.3 Hz);
7.11 (2H, doublet, J = 8.3 Hz);
6.51 (1H, singlet);
6.01 (1H, doublet of doublets, J = 2.0 & 5.3 Hz);
5.19 (1H, doublet, J = 12.7 Hz);
5.10 (1 H, doublet, J = 12.7 Hz);
5.14 (1H, doublet, J = 2.0 Hz);
4.67 (1H, singlet);
4.60 (1H, doublet, J = 5.3 Hz);
2.81 (1H, doublet of triplets, J = 6.8 Hz);
1.23 (6H, doublet, J = 6.8 Hz).

### EXAMPLE 24

### 24(a) 9'-(4-Acetoxy-3-bromobenzyl) 8'-benzhydryl griseolate

A procedure similar to that described in Example 18(b) was repeated, except that 308 mg of 4-acetoxy-3-bromobenzyl bromide were used instead of the 3-acetoxy- benzyl bromide, and that 381 mg of 8'-benzhydryl griseolate, 124 mg of 1,5-diazabicyclo[4.3.0]-5-nonene and 3 ml of dimethylformamide were used, to prepare 280 mg of the title compound, melting at 118 - 120°c.

Nuclear Magnetic Resonance Spectrum (CDC₃) 8 ppm:
8.27 (1H, singlet);
7.81 (1H, singlet);
7.49 - 6.99 (13H, multiplet);
6.86 (1H, singlet);
6.39 (1H, singlet);
6.17 (1H, doublet of doublets, J = 1.9 & 4.9 Hz);
5.15 (1H, doublet, J = 1.9 Hz);
4.92 (1 H, singlet);
4.93 (1H, doublet, J = 10.2 Hz);
4.89 (1H, doublet, J = 10.2 Hz);
4.66 (1H, doublet, J = 4.9 Hz);
2.34 (3H, singlet).

### 24(b) 9'-(4-Acetoxy-3-bromobenzyl) griseolate

Following a procedure similar to that described in Example 18(c), but using 255 mg of 9'-(4-acetoxy-3-bromobenzyl) 8'-benzhydryl griseolate [prepared as described in step 24(a) above], 99 mg of boron trifluoride etherate and 2 ml of acetic acid, 180 mg of the title compound were prepared.

Nuclear Magnetic Resonance Spectrum (hexadeuterated dimethyl sulphoxide) δ ppm:
8.39 (1H, singlet);
8.24 (1H, singlet);
7.76 - 7.27 (3H, multiplet);
6.51 (1H, singlet);
6.05 (1H, doublet of doublets, J = ₁.₅ & 5.3 Hz);
5.20 (1H, doublet, J = 13.2 Hz);
5.11 (1H, doublet, J = 13.2 Hz);
4.69 (1H, singlet);
4.59 (1 H, doublet, J = 5.3 Hz);
2.32 (3H, singlet).

### EXAMPLE 25

### 25(a) 9'-(4-Acetoxy-3-t-butylbenzyl) 8'-benzhydryl griseolate

A procedure similar to that described in Example 18(b) was repeated, except that 313 mg of 4-acetoxy-3-t-butylbenzyl bromide were used instead of the 3-acetoxybenzyl bromide, and that 381 mg of 8'-benzhydryl griseolate, 124 mg of 1,5-diazabicyclo[4.3.0]-5-nonene and 3 ml of dimethylformamide were used, to prepare 230 mg of the title compound, melting at 124 - 125°C.

Nuclear Magnetic Resonance Spectrum (CDCℓ₃) 8 ppm: 8.27 (1H, singlet);
7.80 (1H, singlet);
7.30 - 6.90 (13H, multiplet);
6.77 (1H, singlet);
6.38 (1H, singlet);
6.15 (1H, doublet of doublets, J = 2.5 & 5.1 Hz);
5.14 (1H, doublet, J = 2.5 Hz);
5.00 (1H, doublet, J = 12.0 Hz);
4.91 (1H, doublet, J = 12.0 Hz);
4.89 (1H, singlet);
4.64 (1H, doublet, J = 5.1 Hz);
2.33 (3H, singlet);
1.34 (9H, singlet).

### 25(b) 9'-(4-Acetoxy-3-t-butylbenzyl) griseolate

Following a procedure similar to that described in Example 18(c), but using 220 mg of 9'-(4-acetoxy-3-t-butylbenzyl) 8'-benzhydryl griseolate [prepared as described in step 25(a) above], 83 mg of boron trifluoride etherate and 2 ml of acetic acid, 50 mg of the title compound were prepared.

Nuclear Magnetic Resonance Spectrum (hexadeuterated dimethyl sulphoxide) 8 ppm:
8.34 (1H, singlet);
8.19 (1H, singlet);
7.40 - 7.01 (3H, multiplet);
6.49 (1H, singlet);
6.09 (1H, doublet of doublets, J = 2.4 & 4.9 Hz);
5.14 - 5.10 (3H, multiplet);
4.66 (1H, singlet);
4.58 (1 H, doublet, J = 4.9 Hz);
2.31 (3H, singlet);
1.31 (9H, singlet).

### EXAMPLE 26

### 26(a) 9'-(4-Acetoxy-3-methoxybenzyl) 8'-benzhydryl griseolate

A procedure similar to that described in Example 18(b) was repeated, except that 259 mg of 4-acetoxy-3-methoxybenzyl bromide were used instead of the 3-acetoxybenzyl bromide, and that 381 mg of 8'-benzhydryl griseolate, 124 ml of 1,5-diazabicyclo[4.3.0]-5-nonene and 6 ml of dimethylformamide were used, to prepare 405 mg of the title compound, melting at 118 -120°C.

Nuclear Magnetic Resonance Spectrum (CDCℓ₃) δ ppm:
8.28 (1H, singlet);
7.80 (1H, singlet);
7.30 - 6.89 (3H, multiplet);
6.77 (1H, singlet);
6.37 (1H, singlet);
6.17 (1H, doublet of doublets, J = 2.2 & 4.9 Hz);
5.15 (1H, doublet, J = 2.2 Hz);
4.95 (2H, broad singlet);
4.90 (1 H, singlet);
4.65 (1H, doublet, J = 4.9 Hz);
3.84 (3H, singlet);
2.31 (3H, singlet).

### 26(b) 9'-(4-Acetoxy-3-methoxybenzyl) griseolate

Following a procedure similar to that described in Example 18(c), but using 333 mg of 9'-(4-acetoxy-3-methoxybenzyl) 8'-benzhydryl griseolate [prepared as described in step 26(a) above], 142 mg of boron trifluoride etherate and 4 ml of acetic acid, 120 mg of the title compound were prepared.

Nuclear Magnetic Resonance Spectrum (hexadeuterated dimethyl sulphoxide) 8 ppm:
8.34 (1H, singlet);
8.20 (1H, singlet);
7.16-6.93 (3H, multiplet);
6.50 (1H, singlet);
6.10 (1H, doublet of doublets, J = 2.5 & 4.9 Hz);
5.15 (2H, singlet);
5.13 (1H, doublet, J = 2.5 Hz);
4.70 (1H, singlet);
4.60 (1H, doublet, J = 4.9 Hz);
3.82 (3H, singlet);
2.25 (3H, singlet).

### EXAMPLE 27

### 27(a) 9'-(2,5-Diacetoxybenzyl) 8'-benzhydryl griseolate

A procedure similar to that described in Example 18(b) was repeated, except that 287 mg of ₂,5-diacetoxybenzyl bromide were used instead of the 3-acetoxybenzyl bromide, and that 381 mg of 8'-benzhydryl griseolate, 124 ml of 1,5-diazabicyclo[4.3.0]-5-nonene and 6 ml of dimethylformamide were used, to prepare 280 mg of the title compound, melting at 124-126°C.

Nuclear Magnetic Resonance Spectrum (CDCℓ₃) 8 ppm:
8.26 (1H, singlet);
7.79 (1H, singlet);
7.33-7.04 (13H, multiplet);
6.92 (1H, singlet);
6.35 (1H, singlet);
6.12 (1H, doublet of doublets, J = ₂.₂ & 4.7 Hz);
5.11 (1H, doublet, J = 2.2 Hz);
5.06 (1H, doublet, J = 12.9 Hz);
4.86 (1H, doublet, J = 12.9 Hz);
4.89 (1H, singlet);
4.67 (1H, doublet, J = 4.7 Hz);
2.30 (3H, singlet);
2.29 (3H, singlet).

### 27(b) 9'-(2,5-Diacetoxybenzyl) griseolate

Following a procedure similar to that described in Example 18(c), but using 240 mg of 9'-(2,5-diacetoxybenzyl) 8'-benzhydryl griseolate [prepared as described in step 27(a) above], 100 mg of boron trifluoride etherate and 3 ml of acetic acid, 150 mg of the title compound were prepared.

Nuclear Magnetic Resonance Spectrum (hexadeuterated dimethyl sulphoxide) δ ppm:
8.33 (1H, singlet);
8.18 (1H, singlet);
7.26 - 7.14 (3H, multiplet);
6.49 (1H, singlet);
6.07 (1H, doublet of doublets, J = 1.8 & 4.9 Hz);
5.14 (1H, doublet, J = 13.1 Hz);
5.00 (1H, doublet, J = 13.1 Hz);
5.11 (1H, doublet, J = 1.8 Hz);
4.63 (1H, singlet);
4.57 (1H, doublet, J = 4.9 Hz);
2.30 (3H, singlet);
2.27 (3H, singlet).

### EXAMPLE 28

### 28(a) 9'-(2,6-Diacetoxybenzyl) 8'-benzhydryl griseolate

A procedure similar to that described in Example 18(b) was repeated, except that 287 mg of 2,6-diacetoxybenzyl bromide were used instead of the 3-acetoxybenzyl bromide, and that 381 mg of 8'-benzhydryl griseolate, 124 ml of 1,5-diazabicyclo[4.3.0]-5-nonene and 6 ml of dimethylformamide were used, to prepare 400 mg of the title compound, melting at 121 - 123°C.

Nuclear Magnetic Resonance Spectrum (CDCℓ₃) 8 ppm:
8.26 (1H, singlet);
7.77 (1H, singlet);
7.41 - 6.98 (13H, multiplet);
6.89 (1 H, singlet);
6.34 (1H, singlet);
6.11 (1H, doublet of doublets, J = 2.5 & 5.1 Hz);
5.37 (1H, doublet, J = 11.7 Hz);
4.79 (1H, doublet, J = 11.7 Hz);
5.07 (1H, doublet, J = 2.5 Hz);
4.74 (1 H, singlet);
4.67 (1H, doublet, J = 5.1 Hz);
2.31 (3H, singlet);
2.31 (3H, singlet).

### 28(b) 9'-(2,6-Diacetoxybenzyl) griseolate

Following a procedure similar to that described in Example 18(c), but using 360 mg of 9'-(2,6-diacetoxybenzyl) 8'-benzhydryl griseolate [prepared as described in step 28(a) above], 142 mg of boron trifluoride etherate and 4 ml of acetic acid, 190 mg of the title compound were prepared.

Nuclear Magnetic Resonance Spectrum (hexadeuterated dimethyl sulphoxide) δ ppm:
8.33 (1H, singlet);
8.19 (1H, singlet);
7.51 - 7.11 (3H, multiplet);
6.48 (1H, singlet);
6.05 (1H, doublet of doublets, J = 2.4 & 4.9 Hz);
5.14 (1H, doublet, J = 12.2Hz);
4.92 (1H, doublet, J = 12.2Hz);
5.09 (1H, doublet, J = 2.4 Hz);
4.58 (1H, doublet, J = 4.9 Hz);
4.46 (1H, singlet);
2.31 (3H, singlet);
2.31 (3H, singlet).

### EXAMPLE 29

### 29(a) 9'-(2,3-Diacetoxybenzyl) 8'-benzhydryl griseolate

A procedure similar to that described in Example 18(b) was repeated, except that 287 mg of 2,3-diacetoxybenzyl bromide were used instead of the 3-acetoxybenzyl bromide, and that 381 mg of 8'-benzhydryl griseolate 124 mg of 1,5-diazabicyclo[4.3.0]-5-nonene and 6 ml of dimethylformamide were used, to prepare 270 mg of the title compound, melting at 123-124°C.

Nuclear Magnetic Resonance Spectrum (CDCℓ₃) 8 ppm:
8.25 (1H, singlet);
7.77 (1H, singlet);
7.34-7.12 (13H, multiplet);
6.91 (1H, singlet);
6.36 (1H, singlet);
6.11 (1H, doublet of doublets, J = 1.8 & 4.8 Hz);
5.21 (1H, doublet, J = 12.1 Hz);
4.82 (1H, doublet, J = 12.1 Hz);
5.11 (1H, doublet, J = 1.8 Hz);
4.87 (1H, singlet);
4.65 (1H, doublet, J = 4.8 Hz);
2.30 (3H, singlet);
2.26 (3H, singlet).

### 29(b) 9'-(2,3-Diacetoxybenzyl) griseolate

Following a procedure similar to that described in Example 18(c), but using 270 mg of 9'-(2,3-diacetoxybenzyl) 8'-benzhydryl griseolate [prepared as described in step 29(a) above], 102 mg of boron trifluoride etherate and 2 ml of acetic acid, 100 mg of the title compound were prepared.

Nuclear Magnetic Resonance Spectrum (hexadeuterated dimethyl sulphoxide) δ ppm:
8.35 (1H, singlet);
8.20 (1H, singlet);
7.40 - 7.25 (3H, multiplet);
6.49 (1H, singlet);
6.06 (1H, doublet of doublets, J = 2.4 & 5.4 Hz);
5.16 (1H, doublet, J = 13.1 Hz);
5.02 (1 H, doublet, J = 13.1 Hz);
5.12 (1H, doublet, J = 2.4 Hz);
4.62 (1H, singlet);
4.60 (1H, doublet, J = 5.4 Hz);
2.31 (3H, singlet);
2.27 (3H, singlet).

### EXAMPLE 30

### 9'-(4-Propionyloxybenzyl) griseolate

A procedure similar to that described in Example 18(b) was repeated, except that 232 mg of 4-propionyloxybenzyl bromide were used instead of the 3-acetoxybenzyl bromide, and that 381 mg of 8'-benzhydryl griseolate, 124 mg of 1,5-diazabicyclo[4.3.0]-5-nonene and 6 ml of dimethylformamide were used, to prepare 240 mg of 9'-(4-propionyloxybenzyl) 8'-benzhydryl griseolate. Then, following a procedure similar to that described in Example 18(c), but using the whole of this 9'-(4-propionyloxybenzyl) 8'-benzhydryl griseolate, together with 142 mg of boron trifluoride etherate and 3 ml of acetic acid, in the same reaction vessel and without intermediate isolation, 120 mg of the title compound were prepared.

Nuclear Magnetic Resonance Spectrum (hexadeuterated dimethyl sulphoxide) 8 ppm:
8.34 (1H, singlet);
8.19 (1 H, singlet);
7.42 (2H, doublet, J = 10.5 Hz);
7.13 (2H, doublet, J = 10.5 Hz);
6.49 (1H, singlet);
6.06 (1H, doublet of doublets, J = ₂.₄ & 4.9 Hz);
5.19 (1 H, doublet, J = 12.7 Hz);
5.09 (1H, doublet, J = 12.7 Hz);
5.11 (1H, doublet, J = 2.4 Hz);
4.66 (1H, singlet);
4.59 (1H, doublet, J = 4.9 Hz);
2.51 (2H, quartet, J = 7.8 Hz);
1.13 (3H, triplet, J = 7.8 Hz).

### EXAMPLE 31

### 31 (a) 9'-(2-Acetoxycinnamyl) 8'-benzhydryl griseolate

A procedure similar to that described in Example 18(b) was repeated, except that 265 mg of 2-acetoxycinnamyl bromide were used instead of the 3-acetoxybenzyl bromide, and that 1.09 g of 8'-benzhydryl griseolate, 372 mg of 1,5-diazabicyclo[4.3.0]-5-nonene and 5 ml of dimethylformamide were used, to prepare 290 mg of the title compound, melting at 111 -113°C.

Nuclear Magnetic Resonance Spectrum (CDCℓ₃) 8 ppm:
8.26 (1H, singlet);
7.78 (1H, singlet);
7.40-7.02 (14H, multiplet);
6.96 (1H, singlet);
6.58 (1H, doublet, J = 16.1 Hz);
6.37 (1H, singlet);
6.16 (1H, doublet of doublets, J = 1.8 & 4.8 Hz);
5.95 (1H, doublet of triplets, J = 6.2 & 16.1 Hz);
5.15 (1H, doublet, J = 1.8 Hz);
4.90 (1H, singlet);
4.64 (1H, doublet, J = 4.8 Hz);
4.59 - 4.53 (2H, multiplet);
2.36 (3H, singlet).

### 31 (b) 9'-(2-Acetoxycinnamyl) griseolate

Following a procedure similar to that described in Example 18(c), but using 255 mg of 9'-(2-acetoxycinnamyl) 8'-benzhydryl griseolate [prepared as described in step 31 (a) above], 100 mg of boron trifluoride etherate and 2 ml of acetic acid, 60 mg of the title compound were prepared.

Nuclear Magnetic Resonance Spectrum (hexadeuterated dimethyl sulphoxide) 8 ppm:
8.33 (1H, singlet);
8.18 (1H, singlet);
7.67 - 7.09 (4H, multiplet);
6.71 (1H, doublet, J = 16.1 Hz);
6.49 (1H, singlet);
6.34 (1H, doublet of triplets, J = 5.9 & 16.1 Hz);
6.07 (1H, doublet of doublets, J = 2.4 & 4.9 Hz);
5.11 (1H, doublet, J = 2.4 Hz);
4.78 (2H, doublet, J = 5.9 Hz);
4.63 (1H, singlet);
4.58 (1H, doublet, J = 4.9 Hz);
2.34 (3H, singlet).

### EXAMPLE 32

### 32(a) 9'-(4-Acetoxy-3-methoxycinnamyl) 8'-benzhydryl griseolate

A procedure similar to that described in Example 18(b) was repeated, except that 855 mg of 4-acetoxy-3-methoxycinnamyl bromide were used instead of the 3-acetoxybenzyl bromide, and that 763 mg of 8'-benzhydryl griseolate, 372 mg of 1,5-diazabicyclo[4.3.0]-5-nonene and 10 ml of dimethylformamide were used, to prepare 460 mg of the title compound, melting at 116-118°C.

Nuclear Magnetic Resonance Spectrum (CDCℓ₃) 8 ppm:
8.24 (1H, singlet);
7.79 (1H, singlet);
7.33 - 6.84 (14H, multiplet);
6.46 (1H, doublet, J = 15.8 Hz);
6.38 (1H, singlet);
6.14 (1H, doublet of doublets, J = 2.2 & 5.2 Hz);
5.92 (1H, doublet of triplets, J = 6.0 & 15.8 Hz);
5.16 (1H, doublet, J = 2.2 Hz);
4.91 (1H, singlet);
4.66 - 4.55 (2H, multiplet);
4.65 (1H, doublet, J = 5.2 Hz);
3.81 (3H, singlet);
2.31 (3H, singlet).

### 32(b) 9'-(4-Acetoxy-3-methoxycinnamyl) griseolate

Following a procedure similar to that described in Example 18(c), but using 320 mg of 9'-(4-acetoxy-3-meth- oxycinnamyl) 8'-benzhydryl griseolate [prepared as described in step 31 (a) above], 212 mg of boron trifluoride etherate and 5 ml of acetic acid, 60 mg of the title compound were prepared.

Nuclear Magnetic Resonance Spectrum (hexadeuterated dimethyl sulphoxide) 8 ppm:
8.34 (1H, singlet);
8.18 (1H, singlet);
7.21 - 7.04 (2 peaks, each 3H, together singlet);
6.70 (1H, doublet, J = 15.6 Hz);
6.49 (1H, singlet);
6.35 (1H, doublet of triplets, J = 6.0 & 15.6 Hz);
6.05 (1H, doublet of doublets, J = 2.4 & 5.3 Hz);
5.11 (1H, doublet, J = 2.4 Hz);
4.76 (2H, doublet, J = ₅.₉ Hz);
4.65 (1H, singlet);
4.59 (1H, doublet, J = 5.3 Hz);
3.36 (3H, singlet);
2.24 (3H, singlet).

### EXAMPLE 33

### 33(a) 9'-(4-Acetylthiobenzyl) 8'-benzhydryl griseolate

A procedure similar to that described in Example 18(b) was repeated, except that 245 mg of 4-acetylthiobenzyl bromide were used instead of the 3-acetoxybenzyl bromide, and that 381 mg of 8'-benzhydryl griseolate, 124 mg of 1,5-diazabicyclo[4.3.0]-5-nonene and 3 ml of dimethylformamide were used, to prepare 250 mg of the title compound, melting at 114 - 116°C.

Nuclear Magnetic Resonance Spectrum (CDCℓ₃) 8 ppm:
8.28 (1H, singlet);
7.80 (1H, singlet);
7.63 - 7.17 (14H, multiplet);
6.83 (1H, singlet);
6.38 (1H, singlet);
6.18 (1H, doublet of doublets, J = 2.5 & 4.8 Hz);
5.24 (1H, doublet, J = 2.5 Hz);
4.91 (1H, singlet);
4.64 (1H, doublet, J = 4.8 Hz);
2.42 (3H, singlet).

### 33(b) 9'-(4-Acetylthiobenzyl) griseolate

Following a procedure similar to that described in Example 18(c), but using 220 mg of 9'-(4-acetylthiobenzyl) 8'-benzhydryl griseolate [prepared as described in step 33(a) above], 100 mg of boron trifluoride etherate and 3 ml of acetic acid, 120 mg of the title compound were prepared.

Nuclear Magnetic Resonance Spectrum (hexadeuterated dimethyl sulphoxide) 8 ppm:
8.33 (1H, singlet);
8.20 (1H, singlet);
7.49-7.35 (4H, multiplet);
6.49 (1H, singlet);
6.06 (1H, doublet of doublets, J = 2.4 & 4.8 Hz);
5.23 (1 H, doublet, J = 13.2 Hz);
5.13 (1H, doublet, J = 13.2 Hz);
5.11 (1H, doublet, J = 2.4 Hz);
4.69 (1H, singlet);
4.60 (1H, doublet, J = 4.8 Hz);
2.43 (3H, singlet).

### EXAMPLE 34

### 8'-(4-Acetoxybenzyl) griseolate

450 mg of trifluoromethanesulphonic acid were added, whilst ice-cooling, to a suspension of 380 mg of griseolic acid and 500 mg of molecular sieves 4A in 10 ml of acetone. The mixture was then stirred for 2 hours at room temperature. At the end of this time, 220 mg of calcium carbonate were added to the mixture, and the mixture was stirred for 1.5 hours at room temperature. The insoluble materials were then filtered off, and the solvent was evaporated from the filtrate under reduced pressure. The whole of the residue thus obtained from this first step was dissolved in 5 ml of dimethylformamide, and 370 mg of 1,5-diazabicyclo[4.3.0]-5-nonene and 340 mg of 4-acetoxybenzyl bromide were added to the resulting solution. The mixture was then stirred overnight at room temperature, after which it was extracted with ethyl acetate. The extracts were washed with water and with a saturated aqueous solution of sodium chloride in that order, and were then dried over anhydrous magnesium sulphate. The solvent was then removed by evaporation under reduced pressure, and the residue was dissolved in a mixture of 7 ml of methanol and 3 ml of water. The resulting solution was stirred whilst heating for 2 hours, and then the solvent was removed by evaporation under reduced pressure. The residue was extracted with ethyl acetate. The extracts were washed with water and dried. The solvent was evaporated off under reduced pressure to give crystals. These crystals were washed thoroughly with diethyl ether to give 120 mg of the title compound.

Nuclear Magnetic Resonance Spectrum (hexadeuterated dimethyl sulphoxide) 8 ppm:
8.33 (1H, singlet);
8.15 (1H, singlet);
7.43 (2H, doublet, J = 8.3 Hz);
7.11 (2H, doublet, J = 8.3 Hz);
6.47 (1H, singlet);
6.04 (1H, doublet of doublets, J = ₂.0 & 4.4 Hz);
5.20 (1H, doublet, J = 13.0 Hz);
5.13 (1H, doublet, J = 13.0 Hz);
5.08 (1H, doublet, J = 2.0 Hz);
4.52 (1H, doublet, J = 4.4 Hz);
4.35 (1 H, singlet);
2.26 (3H, singlet).

### EXAMPLE 35

### 8'-(4-Acetoxy-3-methoxybenzyl) griseolate

A procedure similar to that described in Example 34 was repeated, except that, in the second step, 360 mg of 4-acetoxy-3-methoxybenzyl bromide were employed instead of the 4-acetoxybenzyl bromide, to obtain 80 mg of the title compound.

Nuclear Magnetic Resonance Spectrum (hexadeuterated dimethyl sulphoxide) δ ppm:
8.34 (1H, singlet);
8.15 (1H, singlet);
7.13-6.94 (3H, multiplet);
6.51 (1H, singlet);
6.11 (1H, doublet of doublets, J = 2.5 & 4.9 Hz);
5.17 (3H, broad singlet);
4.59 (1H, doublet, J = 4.9 Hz);
4.56 (1H, singlet);
3.72 (3H, singlet);
2.24 (3H, singlet).

### EXAMPLE 36

### 9'-(4-Acetoxybenzyl) 7'-deoxygriseolate

229 mg of 4-acetoxybenzyl bromide were added to a solution of 363 mg of 7'-deoxygriseolic acid and 124 mg of 1,5-diazabicyclo[4.3.0]-5-nonene dissolved in 5 ml of dimethylformamide, and the mixture was stirred at room temperature overnight. It was then extracted with ethyl acetate. The organic extract was washed with a saturated aqueous solution of sodium chloride. The solution was then extracted with a saturated aqueous solution of sodium bicarbonate. The extract was adjusted to a pH value of 3.0 by the addition of dilute hydrochloric acid, and the resulting precipitate was collected by filtration. After drying, there were obtained 51 mg of the title compound.

Nuclear Magnetic Resonance Spectrum (hexadeuterated dimethyl sulphoxide) 8 ppm:
8.32 (1H, singlet);
8.14 (1H, singlet);
7.42 (2H, doublet, J = 12.9 Hz);
7.09 (2H, doublet, J = 12.9 Hz);
6.46 (1H, singlet);
6.07 (1H, doublet of doublets, J = 2.₉ & 4.9 Hz);
5.17 (1H, doublet, J = 2.9 Hz);
5.08 (1H, doublet, J = 2.4 Hz);
4.56 (1H, doublet, J = 4.9 Hz);
3.17 (1H, doublet, J = 16.6 Hz);
2.80 (1H, doublet, J = 16.6 Hz);
2.26 (3H, singlet).

### EXAMPLE 37

### 9'-(4-Acetoxy-3-methoxybenzyl) 7'-deoxygriseolate

A procedure similar to that described in Example 36 was repeated, except that 259 mg of 4-acetoxy-3-methoxybenzyl bromide were employed instead of the 4-acetoxybenzyl bromide, to obtain 42 mg of the title compound.

Nuclear Magnetic Resonance Spectrum (hexadeuterated dimethyl sulphoxide) 8 ppm:
8.32 (1H, singlet);
8.12 (1H, singlet);
7.38 - 6.96 (3H, multiplet);
6.45 (1H, singlet);
6.06 (1H, doublet of doublets, J = 2.5 & 5.0 Hz);
5.17 (3H, singlet);
5.10 (1H, doublet, J = 2.₅ Hz);
4.56 (1H, doublet, J = 5.0 Hz);
3.70 (3H, singlet);
3.18 (1H, doublet, J = 16.6 Hz);
2.82 (1H, doublet, J = 16.6 Hz);
2.24 (3H, singlet).

### PREPARATION 1

### Ophthalmic solution

The following ingredients were mixed by conventional means to prepare an ophthalmic solution having a pH value of 7.0:

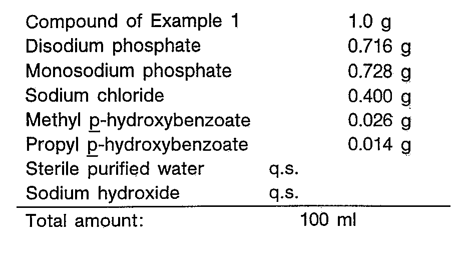

### PREPARATION 2

### Ophthalmic solution

The following ingredients were mixed by conventional means to prepare an ophthalmic solution having a pH value of 7.0:

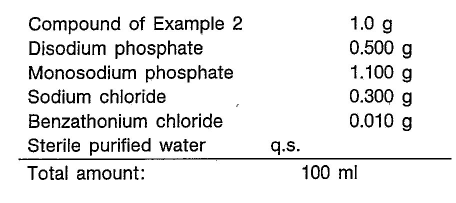

### PREPARATION 3

### Ophthalmic solution

The following ingredients were mixed by conventional means to prepare an ophthalmic solution having a pH value of 7.0:

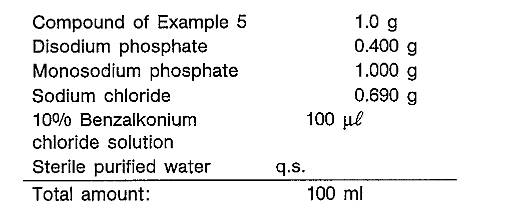

### PREPARATION 4

### Ophthalmic solution

The following ingredients were mixed by conventional means to prepare an ophthalmic solution having a pH value of 7.0:

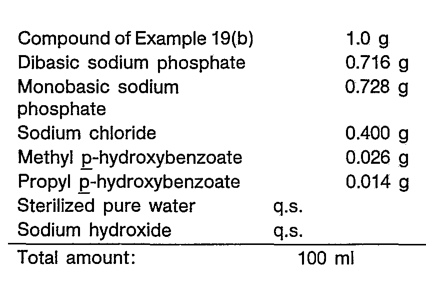

### PREPARATION 5

### Ophthalmic solution

The following ingredients were mixed by conventional means to prepare an ophthalmic solution having a pH value of 7.0:

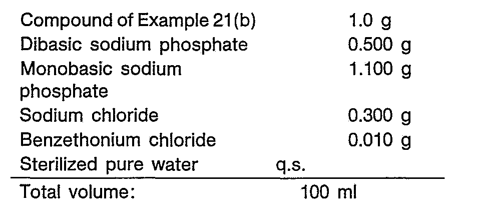

### PREPARATION 6

### Ophthalmic solution

The following ingredients were mixed by conventional means to prepare an ophthalmic solution having a pH value of 7.0:

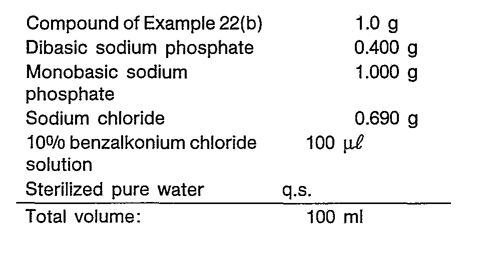

### BIOLOGICAL EFFECTS

### Test animals

In the following Experiments 1 to 6, the test animals used were male New Zealand white rabbits, each weighing about 2.5 kg, raised in a room where the room temperature, relative humidity and lighting time were controlled to be 23° C, 60% and from 07:00 to 19:00, respectively. The animals were chosen from those where no abnormalities could be detected in their eyes by observation with the naked eye. They were given water freely but feed was restricted.

### Experiment 1

### Intraocular pressure reduction in normal rabbits

The reduction in intraocular pressure (IOP) caused by the compounds of the present invention was investigated using groups of three male New Zealand white rabbits for each test. The compounds of the present invention were employed as solutions dissolved in a 50 mM phosphate buffer (pH 7.0) containing 0.4% w/v of sodium chloride which were adjusted to concentrations of about 1% w/v of the compound under test.

Both eyes of each rabbit were anesthetized with eye drops containing an ophthalmic anesthetic (0.4% Benoxil, available from Santen Pharmaceutical Co., Ltd.), and then the IOP in each eye was measured by means of a PTG (Pneumatonograph: Alcon). The test substance was then instilled into either the left eye or the right eye (the eye to be tested) in an amount of 50 µℓ (in the case of 0.5% timolol, a known compound used for comparison, the amount was 30 µℓ). Sixty minutes after this instillation, the same amount of the same test compound was instilled into the same eye of each test animal. On the other hand, no instillation was effected into the other eye of each animal, which was used as a control eye. The IOP values of both eyes were measured 30,60,90,120,150 and 180 minutes, after the initial instillation of the test substance. The IOP reduction at each time of measurement caused by each test substance was calculated from the following equation: Intraocular pressure reduction (Ap, mmHg) = (I_{c} - It) - (I_{c}° - Iₜ°),
in which:
I_{c} the IOP of the control eye;
It is the IOP of the test eye;
I_{c}° the IOP of the control eye immediately before the initial instillation; and
It° is the IOP of the test eye immediately before the initial instillation.

The average value of the IOP reduction at each time of measurement was calculated to obtain the intraocular reduction (Ap, mmHg) of the test substance. The results are reported as a ratio of the Δp of the test substance to that of the known substance, 0.5% timolol, which is, therefore, taken as 1. The results are given in Table 13.

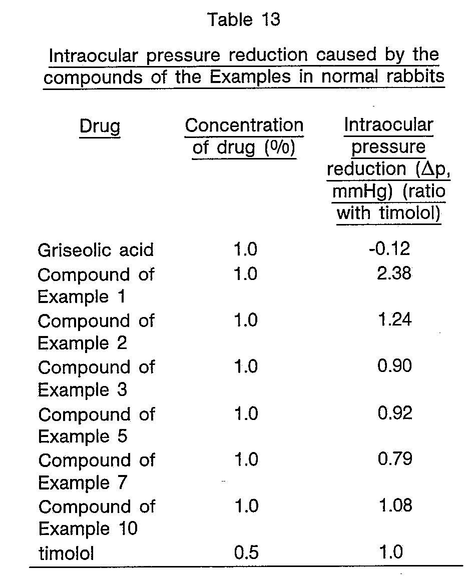

As can be seen from the above Table, the novel griseolic acid monoester derivatives of the present invention have an excellent ability to reduce IOP and, moreover, they have effectively no toxicity. Accordingly, they are expected to be of considerable value in the treatment and prophylaxis of glaucoma.

In Experiments 2,3,4 and 6, the compounds under test were all formed into preparations as follows:

### Sample preparation:

The compound under test was dissolved in 100 mM of a sodium phosphate buffer solution (pH 7.2) containing a small amount of a 0.1 N aqueous solution of sodium hydroxide. In the case of timolol maleate, a 0.5% w/v eye lotion (Timoptol 0.5%, Santen Pharm. Co.) was used as such.

### Experiment 2

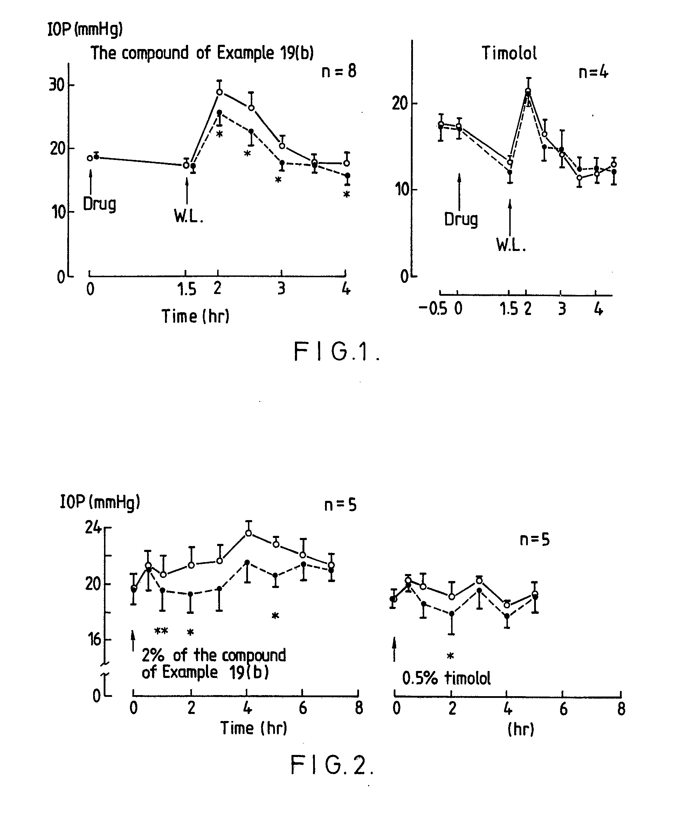

### Effect on water load-induced elevation of intraocular pressure (water load test) [cf. Fig. 1]

Each rabbit was anesthetized with urethane (1.0 g/kg, i.v.), and the IOP values of both eyes were measured. Immediately thereafter, 50 µℓ of the sample solution was applied to one eye (test eye). To the other eye (control eye), 50 µℓ of the vehicle alone was applied. Ninety minutes after this application, the values of IOP were measured and then the water load test was performed as follows. Sixty ml/kg of tap water was given compulsorily per os to the anesthetized rabbit, and then the values of IOP for both eyes were measured with the passage of time.

In this Experiment, the test compound was the compound of Example 19(b), i.e. 9'-(4-acetoxybenzyl) griseolate.

At 90 minutes after the application of a 2.0% sample solution, the values of IOP were 17.1 ± 1.1 mmHg (2280 ± 147 Pa) for the control eye and 16.8 ± 1.0 mmHg (2240 ± 133 Pa) for the test eye. Following the administration of the tap water to the rabbit, the IOP of the control eye increased transiently. In detail, IOP values of the control eye were 28.7 ± 1.8 mmHg (3826 ± 240 Pa), 26.3 ± 2.4 mmHg (3506 ± 320 Pa), 20.3 ± 1.6 mmHg (2706 ± 213 Pa),17.4 ± 1.5 mmHg (2320 ± 200 Pa) and 17.4 ± 1.9 mmHg (2320 ± 253 Pa) at 0.5, 1.0, 1.5, 2.0 and 2.5 hours after water loading, respectively. On the other hand, those of the applied eye (test eye) were 25.6 ± 2.2 mmHg (3413 ± 293 Pa), 22.6 ± 2.3 mmHg (3013 ± 307 Pa), 17.5 ± 1.2 mmHg (2333 ± 160 Pa), 17.1 ± 1.1 mmHg (2280 ± 147 Pa) and 15.6 ± 1.5 mmHg (2080 ± 200 Pa) at the same times as above after water loading, respectively. Statistically significant differences (p < 0.05) were noted between the values of IOP of the two eyes, in the values at 0.5,1.0,1.5 and 2.5 hours after water loading. The effect of 0.5% timolol maleate was examined in a similar way, but no significant difference could be observed between the control eye and the test eye.

The results are shown graphically in Figure I of the accompanying drawings.

### Experiment 3

### Normal intraocular pressure lowering effect [cf. Fig. 2]

The corneal surface was anesthetized by the application of oxybuprocain hydrochloride (Benoxil 0.4% solution, Santen Pharm. Co.), and the IOP was measured by means of an Alcon Applanation Pneumatograph (Japan Alcon) in rabbits. The measurements were carried out 2 or 3 times for each eye, and the mean value was calculated and is regarded as the value of IOP. After determination of the IOP, 25 µℓ of a sample solution was applied to one eye (test eye) three times every two minutes, and the same volume of the vehicle alone was applied to the other eye (control eye). The values of IOP of both eyes were measured with the passage of time. In this Experiment, the test compound was the compound of Example 19(b).

Just before the application, the values of IOP of the control and test eyes were very nearly equal to each other, i.e. 19.7 ± 1.1 mmHg (2626 ± 147 Pa) and 19.6 ± 1.0 mmHg (2613 ± 133 Pa) respectively. One hour after application of a 2.0% sample solution of a compound of the present invention, the values of IOP of the control and test eyes were 20.7 ± 1.4 mmHg (2760 ± 187 Pa) and 19.5 ± 1.4 mmHg (2600 ± 187 Pa) respectively; 2 hours after application they were 21.4 ± 1:3 mmHg (2853 ± 173 Pa) and 19.3 ± 1.3 mmHg (2573 ± 173 Pa) respectively; and 5 hours after application they were 22.8 ± 0.5 mmHg (3040 ± 67 Pa) and 20.6 ± 0.7 mmHg (2746 ± 93 Pa) respectively. In any case, the value of IOP in the test eye was significantly lower than that of the control eye statistically (p < 0.05), and a normal IOP lowering effect was observed. On the other hand, the IOP values of the control and test eyes just before application of 0.5% timolol maleate were both 18.9 ± 0.5 mmHg (2520 ± 67 Pa). A significant lowering (p 0.05) of IOP by application of timolol maleate to the eye was only observed 2 hours after application, and no significant effect was observed at other times. That is, the IOP lowering effect of the application of timolol maleate was transient.

The results are shown graphically in Figure 2 of the accompanying drawings.

### Experiment 4

### Effect on rise of intraocular pressure during darkness

Intraocular pressure was measured at 10:00 (light phase) and at 23:00 hours (dark phase). After measurement of the IOP at 23:00, 25 µℓ of a 2% sample solution of the test compound was applied to one eye (test eye) three times every two minutes, and the same volume of the vehicle alone was applied to the other eye (control eye). After 3 hours, the values of IOP for both eyes were measured.

At 10:00 (light phase), the values for the control and test eyes were 14.3 ± 0.7 mmHg (1907 ± 93 Pa) and 14.3 ± 0.8 mmHg (1907 ± 107 Pa), respectively. At 23:00 (dark phase), the values for the control and test eyes were 21.4 ± 1.0 mmHg (2853 ± 133 Pa) and 21.8 ± 0.9 mmHg (2906 ± 120 Pa), respectively, showing an increase in IOP of about 7 mmHg (933 Pa). Just after measurement of the IOP at 23:00, a 2.0% sample solution was applied, and then the IOP was measured for both eyes at 02:00. The values for the control and test eyes were 21.9 ± 0.8 mmHg (2920 ± 107 Pa) and 20.8 ± 0.8 mmHg (2773 ± 107 Pa) respectively between which a statistically significant difference (p < 0.05) was noted.

### Experiment 5

### Determination of CAMP accumulation in Balb/3T3 cells

Balb/3T3 cells were incubated in Dulbecco's modified Eagle medium (glucose-rich) containing 100/o v/v bovine fetal serum. After the cell culture had become confluent, a cell suspension was prepared as follows: The cells were treated with 0.05% trypsin-0.01% ethylenediamine tetraacetate (EDTA), washed with a buffer solution (pH = 7.4) containing 140 mM of sodium chloride, 3 mM of magnesium chloride and 10 mM of HEPES [4-(2-hydroxyethyl)-1-piperazineethane-sulphonic acid], and suspended in the same kind of buffer solution as mentioned above so as to give a suspension containing 2 to 3 x 10⁶ cells/ml. The sample was then dissolved in a buffer solution prepared by the further addition of 4 mM of calcium chloride to the buffer solution mentioned above. If the sample was difficult to dissolve in water, it was dissolved first in dimethyl sulphoxide (DMSO) and then the resulting solution was diluted with the buffer (the final concentration of DMSO was determined to be 0.1%). The cell suspension was preincubated at 30° C for 5 minutes, and 100 µℓ of this suspension were added to 100 µℓ of the sample solution to initiate enzymatic reaction. After incubation at 30° C for 10 minutes, 20 µℓ of 1 N aqueous hydrochloric acid was added to stop the reaction. The reaction mixture was then centrifuged at 3000 rpm for 3 minutes to obtain 100 µℓ of the supernatant. Cyclic 3',5'-adenosine monophosphate (cAMP) contained in the supernatant was quantitatively analyzed by radioimmunoassay using a Yamasa cAMP radioimmunoassay kit YSI-7701. The CAMP accumulating effect is reported as the ratio between the value measured using a test compound and that measured in the control. The results are reported in the following Table 14.

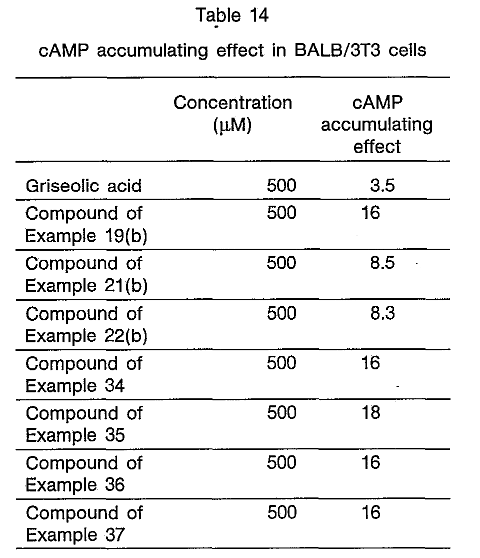

### Experiment 6

### IOP Lowering Effect:

The cornea of the rabbit was anesthetized by the topical application of one or two drops of 0.4% oxybuprocaine hydrochloride. The IOP was measured two or three times for each eye and the mean value was calculated and is reported as the IOP value. After the IOP of the rabbit eye had been measured, 25 µℓ of the sample solution was applied to center of the cornea of one eye three times every 2 minutes. The other eye was treated with the vehicle alone in a similar way. The IOP of both eyes was then measured.

### Statistical Analysis:

IOP values obtained from both eyes were plotted against times after application and the area enclosed by the two IOP lines (with and without the active compound) was calculated. The IOP lowering effect of each sample was expressed as a ratio of the area described above to that of 0.5⁰/o timolol. The results are reported in the following Table 15.

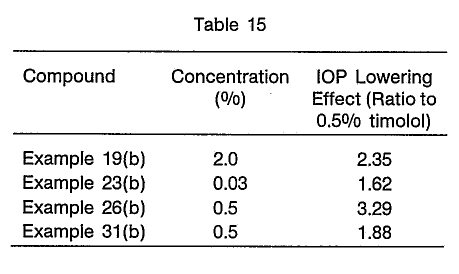

### Experiment 7

### Acute toxicity in the rat

Animals: Male F344 rats were used, each group consisting of 3 animals.

Drug preparation and administration: the compound of Example 19(b) was dissolved in distilled water and administered subcutaneously to the rats in a volume of 0.5 ml per 100 g of body weight. Observation of the rats was carried out over a period of one week.

### Test results:

The results are summarized in the following Table 16.

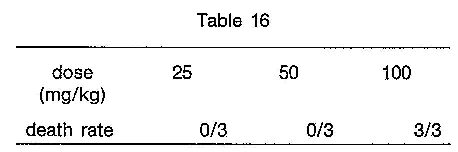

### Experiment 8

### Four-day toxicity in the rat

Animals: Male F344 rats were used, each group consisting of 5 animals.

Drug preparation and administration: the compound of Example 19(b) was dissolved in distilled water and administered subcutaneously to the rats in a volume of 0.4 ml per 100 g of body weight at 09:00 for 4 days. Twenty-four hours after the final dose the rats were anesthetized with diethyl ether and their blood and organs were collected, weighed and used for pathological examination.

Results: These are shown in the following Table 17.

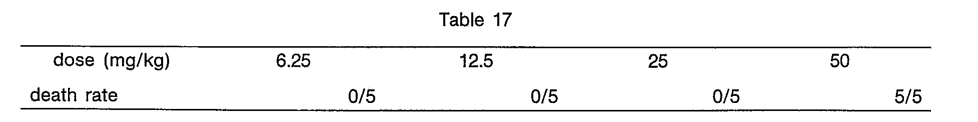

These results demonstrate that the compounds are effectively non-toxic at therapeutic doses.

### _{.} Experiment 9

### Measurement of aqueous humor protein concentration.

Animals: Eight male albino rabbits, each weighing about 2.5 kg, were used.

Drug preparation and administration: the compound of Example 19(b) was dissolved in a 0.1 M sodium phosphate buffer (pH 7.2) and administered to one eye in a volume of 25 µℓ three times every 2 minutes. The other eye of the same rabbit was treated with the vehicle alone in the same way. Four hours after the application, the rabbit was killed by administration of an overdose of sodium pentobarbiturate. Immediately thereafter, the aqueous humor of each eye was collected in a syringe through a needle penetrating the cornea and the protein concentration was measured using a protein aaasay kit (BIO-RAD, U.S.A.).

### Result:

The aqueous humor protein concentrations were 0.27 ± 0.03 µg/ml and 0.27 ± 0.04 µg/ml for the eye treated with the vehicle and that treated with 2% of the compound of Example 19(b), respectively. Thus, there was no change in the aqueous humor protein concentration due to the topical administration of the compound of Example 19(b).

### Experiment 10

### Biochemical examination of serum from a rabbit to which the compound of Example 19(b) was topically applied for a week.

Animals: Male albino rabbits, each weighing about 2.5 kg, were used in a group of 5 animals.

Drug preparation and administration: the compound of Example 19(b) was dissolved in a 0.1 M sodium phosphate buffer (pH 7.2) and administered topically to one eye of the rabbit twice a day at 10:00 and 16:00 for one week, although the administration at 16:00 on the final day was not carried out. The other group was left intact as a control. Four hours after the final administration at 09:00, blood was collected from the marginal ear vein of the rabbit in a syringe, and was centrifuged to obtain the serum.

Biochemical analysis of the serum: the rabbit serum was biochemically analyzed for trigly.ceride (TG), total cholesterol (T-CHO), alkaline phosphatase (ALP), total protein (TP), phospholipid (PL), urea nitrogen (UN), glucose (GLU), transaminase (GOT and GPT), albumin (ALB), total calcium (CA), inorganic phosphorus (IP), creatinine (CRE), and ratio of albumin to globulin (A/G). The analysis was carried out by using an autoanalyzer (HITACHI, Japan).

### Result:

As summarized in Table 18, statistically (student's t-test) no significant differences were observed between the control eye and the experimental eye for all the items examined in this experiment.

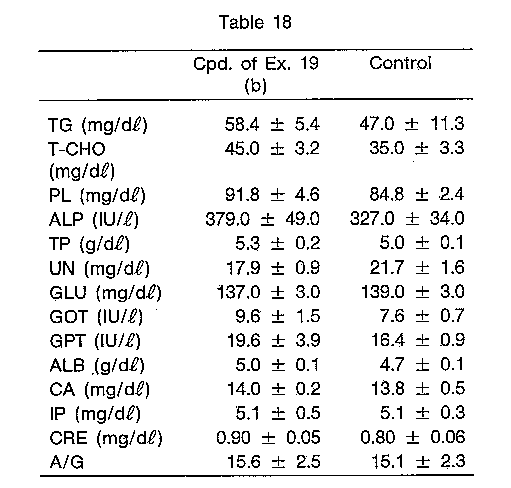

### Experiment 11

The irritation to the eyes caused by solutions of the compound of Example 19(b) was determined by the Draize method [J.H. Draize et af., J. Pharmacol. Exp. Ther., 82,377 (1944)] and evaluated by the method of Kay and Calandra [J.H. Kay and J.C. Calandra, J. Soc. Cosmet. Chem., 13, 281 (1962)].

The test solution employed was a simple solution of 2% by weight of the test compound in 100 mM of a sodium phosphate buffer (pH 7.2) for injections and adjusted to a pH value of from 7 to 8, as required.

A male New Zealand white rabbit of body weight about 2.5 kg was placed in a pillory to fix its head. 0.05 ml of the test solution was instilled into its right eye three times a day (at 09:00, 13:00 and 17:00) for seven days. The symptoms experienced by the anterior portion of the right eye were observed every day at 18:00 over a period of seven days, and the irritation observed was scored. No significant stimulus was observed.

## Claims

1. Compounds of formula (I) ; 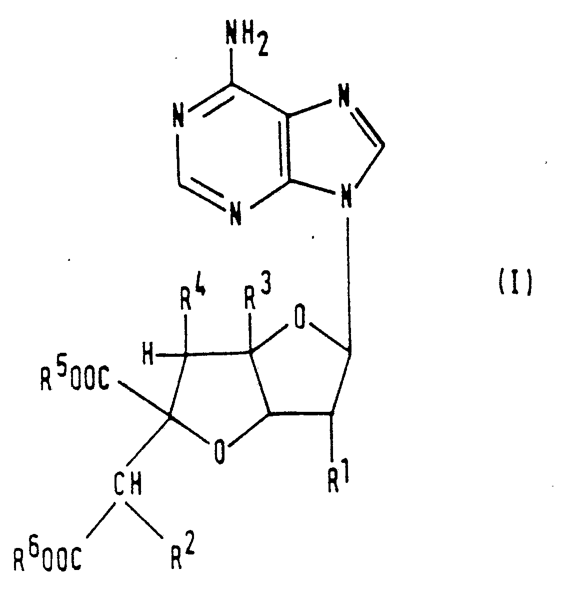 in which:
R¹ and R² are the same or different and each represents a hydrogen atom, a hydroxy group or a protected hydroxy group;
R³ and R⁴ each represents a hydrogen atom, or together represent an extra carbon-carbon bond between the two carbon atoms to which they are attached; and
one of R⁵ and R⁶ represents a hydrogen atom and the other represents a carboxy-protecting group removable in the biochemical environment of the human eye;
and pharmaceutically acceptable salts thereof.

2. Compounds according to Claim 1, in which: one of R⁵ and R⁶ represents a hydrogen atom; and the other represents:
(i) a group of formula (II); 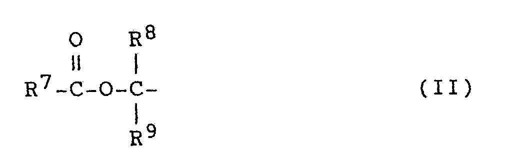 in which: R⁷ represents a straight or branched chain alkyl group having from 1 to 10 carbon atoms, an aryl group having from 6 to 14 ring carbon atoms or a cycloalkyl group having from 3 to 10 carbon atoms, the aryl group being unsubstituted or having at least one of substituents (a), defined below; R⁸ represents a hydrogen atom, a straight or branched chain alkyl group having from 1 to 10 carbon atoms or a cycloalkyl group having from 3 to 10 carbon atoms; and R⁹ represents a hydrogen atom or a straight or branched chain alkyl group having from 1 to 10 carbon atoms;
(ii) a group of formula (III): 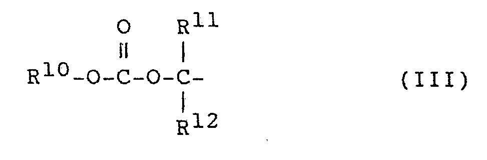 in which: R¹⁰ represents a straight or branched chain alkyl group having from 1 to 10 carbon atoms, an aryl group having from 6 to 14 ring carbon atoms or a cycloalkyl group having from 3 to 10 carbon atoms, the aryl group being unsubstituted or having at least one of substituents (a), defined below; R¹¹ represents a hydrogen atom, a straight or branched chain alkyl group having from 1 to 10 carbon atoms or a cycloalkyl group having from 3 to 10 carbon atoms; and R¹² represents a hydrogen atom or a straight or branched chain alkyl group having from 1 to 10 carbon atoms;
(iii) a group of formula (IV): 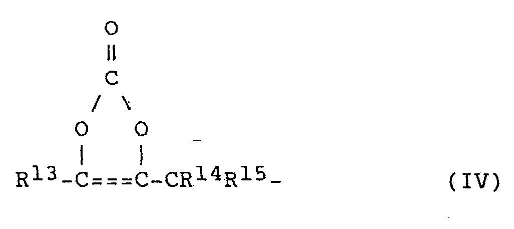 in which: R¹³ represents a straight or branched chain alkyl group having from 1 to 10 carbon atoms, a cycloalkyl group having from 3 to 10 carbon atoms or an aryl group having from 6 to 10 carbon atoms, which aryl group is unsubstituted or has at least one of substituents (a), defined below; R¹⁴ and R¹⁵ are the same or different and each represents a hydrogen atom or a straight or branched chain alkyl group having from 1 to 10 carbon atoms;
(iv) a group of formula (V): 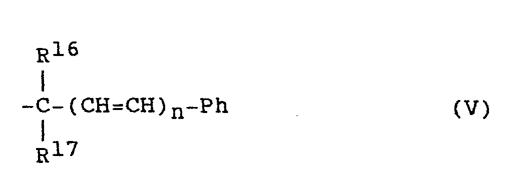 in which R¹⁶ and R¹⁷ are the same or different and each represents a hydrogen atom or a Ci - C₆ alkyl group; Ph represents a phenyl group which is unsubstituted or has at least one substituent selected from: C₁ - C₁₈ aliphatic acyloxy groups, Ci - C₁₈ aliphatic acylthio groups, substituted Ci - C₁₈ aliphatic acyloxy and Ci - C₁₈ aliphatic acylthio groups, aromatic acyloxy groups, aromatic acylthio groups, Ci - C₁₀ alkyl groups, Ci - C6 alkoxy groups, halogen atoms, hydroxy groups, mercapto groups, carboxy groups, amino groups and nitro groups, in which the substituent(s) on said substituted aliphatic acyloxy and acylthio groups are selected from halogen atoms and C₃ - C₁₀ cycloalkyl groups, said aromatic acyloxy and acylthio groups being unsubstituted or having at least one of substituents (a), defined below; and n represents 0,1 or 2;
(v) a group of formula (VI): 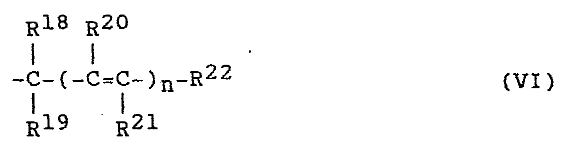 in which R¹⁸, R19, R20 and R21, are the same or different and each represents a hydrogen atom or a C₁ - C₆ alkyl group; R²² represents a carboxylic acyloxy group; and n is as defined above;
(vi) a group of formula (VII) : 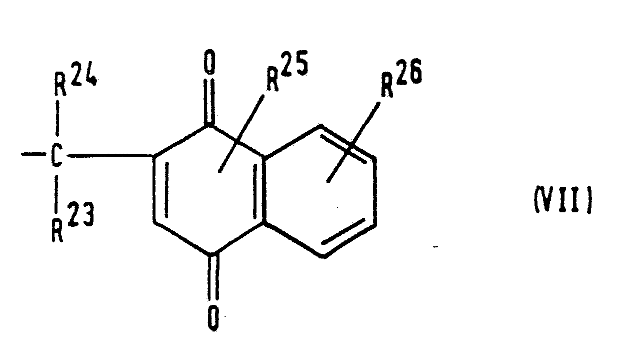 in which R²³ and R²⁴ are the same or different and each represents a hydrogen atom or a C₁ - C₆ alkyl group; and R²⁵ and R²⁶ are the same or different and each represents a C₁ - C₆ alkyl group, a C₁ - C₆ alkoxy group or a halogen atom; or
(vii) a group of formula (VIII): 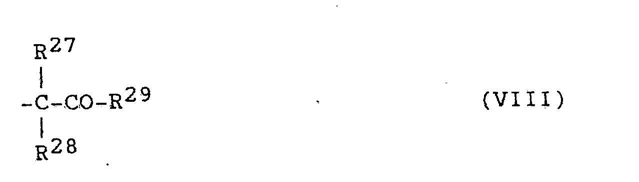 in which: R²⁷ and R²8 are the same or different and each represents a hydrogen atom or a C₁ - C₆ alkyl group; and R²⁹ represents a hydrogen atom, a C₁ - C₆ alkyl group or an aryl group having from 6 to 14 ring atoms and which is unsubstituted or has at least one of substituents (a), defined below; substituents (a): Ci - C₆ alkyl groups, C₁ - C₆ alkoxy groups, the nitro group, halogen atoms, the cyano group, aryl groups, provided that, where any such aryl group is a substituent on an aryl group, it is not itself further substituted by another aryl group, C₁ - Cs alkylenedioxy groups, the hydroxy group, the amino group, mono- and di- alkylamino groups, in which the or each alkyl group is C₁ - C₆, the carbamoyl group, mono- and di- alkylcarbamoyl groups, in which the or each alkyl group is C₁ - C₆, C₁- C₁₈ aliphatic acyl groups, halogenated Ci - C₆ alkyl groups, the carboxy group, alkoxycarbonyl groups, in which the alkoxy group is Ci - C₆, halogenated alkoxycarbonyl groups or aralkyloxycarbonyl groups.

3. Compounds according to Claim 2, in which one of R⁵ and R⁶ represents a group of formula (IIa) : 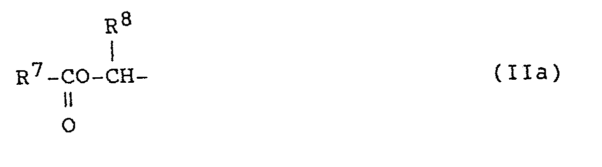 in which:
R⁷ represents a straight or branched chain alkyl group having from 1 to 10 carbon atoms, an aryl group having from 6 to 14 ring carbon atoms or a cycloalkyl group having from 3 to 10 carbon atoms, the aryl group being unsubstituted or having at least one of substituents (a) and;
R⁸ represents a hydrogen atom, a straight or branched chain alkyl group having from 1 to 10 carbon atoms or a cycloalkyl group having from 3 to 10 carbon atoms;
substituents (a) being as defined in Claim 2.

4. Compounds according to Claim 3, in which one of R⁵ and R⁶ represents a group of formula (Ila), as defined in Claim 3, where R⁷ represents a straight or branched chain alkyl group having from 1 to 6 carbon atoms or a cycloalkyl group having from 5 to 7 carbon atoms.

5. Compounds according to Claim 3, in which one of R⁵ and R⁶ represents a group of formula (Ila), as defined in Claim 3, where R⁸ represents a hydrogen atom or a straight or branched chain alkyl group having from 1 to 6 carbon atoms.

6. Compounds according to Claim 2, in which one of R⁵ and R⁶ represents a group of formula (IIIa) : 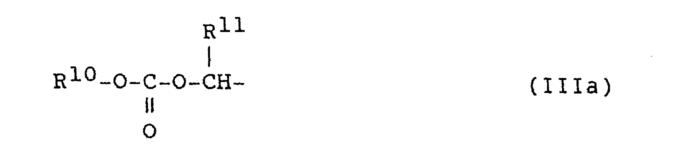 in which:
R¹⁰ represents a straight or branched chain alkyl group having from 1 to 10 carbon atoms, an aryl group having from 6 to 14 ring carbon atoms or a cycloalkyl group having from 3 to 10 carbon atoms, the aryl group being unsubstituted or having at least one of substituents (a) and;
R¹¹ represents a hydrogen atom, a straight or branched chain alkyl group having from 1 to 10 carbon atoms or a cycloalkyl group having from 3 to 10 carbon atoms;
substituents (a) being as defined in Claim 2.

7. Compounds according to Claim 6, in which one of R⁵ and R⁶ represents a group of formula (ilia), as defined in Claim 6, where R¹⁰ represents a straight or branched chain alkyl group having from 1 to 6 carbon atoms.

8. Compounds according to Claim 6, in which one of R⁵ and R⁶ represents a group of formula (IIIa), as defined in Claim 6, where R¹¹ represents a hydrogen atom or a straight or branched chain alkyl group having from 1 to 6 carbon atoms.

9. Compounds according to Claim 2, in which one of R⁵ and R⁶ represents a group of formula (IVa): 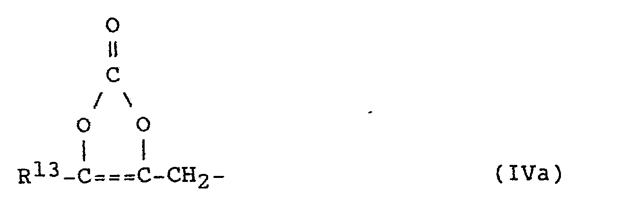 in which:
R¹³ represents a straight or branched chain alkyl group having from 1 to 10 carbon atoms, a cycloalkyl group having from 3 to 10 carbon atoms or an aryl group having from 6 to 10 carbon atoms, which aryl group is unsubstituted or has at least one of substituents (a);
substituents (a) being as defined in Claim 2.

10. Compounds according to Claim 9, in which one of R⁵ and R⁶ represents a group of formula (IVa), as defined in Claim 9, where R¹³ represents a phenyl group, a straight or branched chain alkyl group having from 1 to 6 carbon atoms, or a cycloalkyl group having from 5 to 7 carbon atoms.

11. Compounds according to Claim 9, in which one of R⁵ and R⁶ represents a group of formula (IVa), as defined in Claim 9, where R¹³ represents a straight or branched chain alkyl group having from 1 to 6 carbon atoms.

12. Compounds according to Claim 2, in which one of R⁵ and R⁶ represents a group of formula (Va): 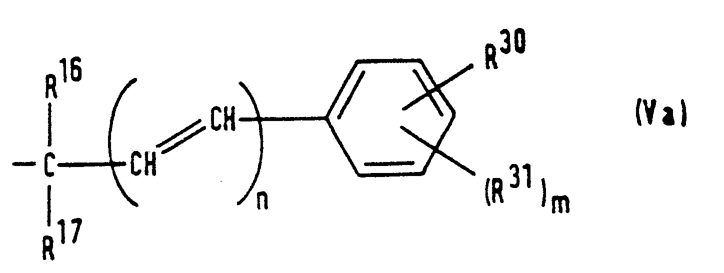 in which:
R¹⁶ and R¹⁷ are the same or different and each represents a hydrogen atom or a Ci - C₆ alkyl group;
n represents 0, 1 or 2;
m represents the cypher 0 or an integer from 1 to 3;
R^{3o} represents a C₁ - C18 aliphatic acyloxy group, a Ci - C₁₈ aliphatic acylthio group, a substituted Ci - C₁₈ aliphatic acyloxy or Ci - C₁₈ aliphatic acylthio group, an aromatic acyloxy group or an aromatic acylthio group, said aromatic acyloxy and acylthio groups being unsubstituted or having at least one of substituents (a);
R³¹ represents a C₁-C₁₈ aliphatic acyloxy group, a C₁-C₁₈ aliphatic acylthio group, a substituted Ci - C₁₈ aliphatic acyloxy or C₁-C₁₈ aliphatic acylthio group, an aromatic acyloxy group, an aromatic acylthio group, a Ci - C₆ alkyl group, a Ci - C₆ alkoxy group, a halogen atom, a hydroxy group, a mercapto group, a carboxy group, an amino group or a nitro group, said aromatic acyloxy and acylthio groups being unsubstituted or having at least one of substituents (a);
the substituent(s) on said substituted aliphatic acyloxy and acylthio groups represented by R³⁰ and R³¹ being selected from halogen atoms and C₃ - C₁₀ cycloalkyl groups;
substituents (a) being as defined in Claim 2.

13. Compounds according to Claim 12, in which one of R⁵ and R⁶ represents a group of formula (Va), as defined in Claim 12, where R¹⁶ represents a hydrogen atom or a C₁ - C₆ alkyl group.

14. Compounds according to Claim 12, in which one of R^{S} and R⁶ represents a group of formula (Va), as defined in Claim 12, where R¹⁷ represents a hydrogen atom or a methyl group.

15. Compounds according to Claim 12, in which one of R⁵ and R⁶ represents a group of formula (Va), as defined in Claim 12, where R³⁰ represents an aliphatic carboxylic acyloxy group containing from 1 to 18 carbon atoms, said group being unsubstituted or having at least one halogen substituent, or an aromatic acyloxy group which is unsubstituted or has at least one of substituents (a).

16. Compounds according to Claim 12, in which one of R⁵ and R⁶ represents a group of formula (Va), as defined in Claim 12 where R³⁰ represents an aliphatic carboxylic acyloxy group containing from 1 to 6 carbon atoms, said group being unsubstituted or having at least one halogen substituent.

17. Compounds according to Claim 12, in which one of R⁵ and R⁶ represents a group of formula (Va), as defined in Claim 12, where R³¹ represents an aliphatic carboxylic acyloxy group containing from 1 to 18 carbon atoms, an alkyl group containing from 1 to 6 carbon atoms, an alkoxy group containing from 1 to 6 carbon atoms or a halogen atom.

18. Compounds according to Claim 12, in which one of R⁵ and R⁶ represents a group of formula (Va), as defined in Claim 12, in which m is 0,1 or 2.

19. Compounds according to Claim 12, in which one of R⁵ and R⁶ represents a group of formula (Va), as defined in Claim 12, in which n is 0 or 1.

20. Compounds according to Claim 12, in which:
one of R⁵ and R⁶ represents a hydrogen atom; and the other of R⁵ and R⁶ represents a group of formula (Va), as defined in Claim 12, in which:
_{R}¹⁶ represents a hydrogen atom or a C₁-C₆ alkyl group,
R¹⁷ represents a hydrogen atom,
R³⁰ represents an aliphatic carboxylic acyloxy group containing from 1 to 18 carbon atoms, said group being unsubstituted or having at least one halogen substituent, or an aromatic acyloxy group which is unsubstituted or has at least one of substituents (a),
R³¹ represents an aliphatic carboxylic acyloxy group containing from 1 to 18 carbon atoms, an alkyl group containing from 1 to 6 carbon atoms, an alkoxy group containing from 1 to 6 carbon atoms or a halogen atom,
m is 0, 1 or 2, and n is 0 or 1.

21. Compounds according to Claim 12, in which:
one of R⁵ and R⁶ represents a hydrogen atom; and
the other of R⁵ and R⁶ represents a group of formula (Va), as defined in Claim 12, in which:
R¹⁶ represents a hydrogen atom or a C₁-C₆ alkyl group,
R¹⁷ represents a hydrogen atom or a methyl group,
R³⁰ represents an aliphatic carboxylic acyloxy group containing from 1 to 6 carbon atoms, said group being unsubstituted or having at least one halogen substituent,
R³¹ represents an aliphatic carboxylic acyloxy group containing from 1 to 6 carbon atoms, an alkyl group containing from 1 to 6 carbon atoms, an alkoxy group containing from 1 to 6 carbon atoms or a halogen atom,
m is 0, 1 or 2, and
n is 0 or 1.

22. Compounds according to Claim 12, in which:
one of R⁵ and R⁶ represents a hydrogen atom; and
the other of R⁵ and R⁶ represents a group of formula (Va), as defined in Claim 12, in which:
R¹⁶ and R¹⁷ each represents a hydrogen atom,
R³⁰ represents an aliphatic carboxylic acyloxy group containing from 1 to 6 carbon atoms, said group being unsubstituted or having at least one halogen substituent,
m is 0, and
n is 0 or 1.

23. Compounds according to Claim 12, in which:
one of R⁵ and R⁶ represents a hydrogen atom; and the other of R⁵ and R⁶ represents a group of formula (Va), as defined in Claim 12, in which:
R¹⁶ and R¹⁷ each represents a hydrogen atom,
R³⁰ and R³¹ each represents an aliphatic carboxylic acyloxy group containing from 1 to 6 carbon atoms, said group being unsubstituted or having at least one halogen substituent, m is 1, and
n is 0 or 1.

24. Compounds according to Claim 12, in which:
one of R⁵ and R⁶ represents a hydrogen atom; and
the other of R⁵ and R⁶ represents a group of formula (Va), as defined in Claim 12, in which:
R¹⁶ and R¹⁷ each represents a hydrogen atom,
R³⁰ represents an aliphatic carboxylic acyloxy group containing from 1 to 6 carbon atoms, said group being unsubstituted or having at least one halogen substituent,
R³¹ represents an alkyl group containing from 1 to 6 carbon atoms or an alkoxy group containing from 1 to 6 carbon atoms,
m is 1 or 2, and
n is 0 or 1.

25. Compounds according to any one of the preceding Claims, in which R¹ and R² are the same or different and each represents a hydrogen atom or a hydroxy group.

26. Compounds according to Claim 1, in which:
R¹ and R² are the same or different and each represents a hydrogen atom or a hydroxy group; and one of R⁵ and R⁶ represents a group of formula (Ila), (ilia) or (IVa): 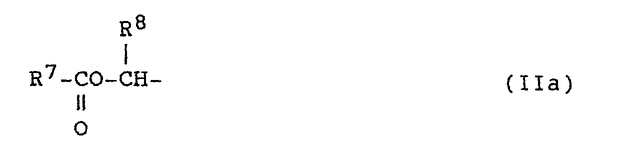 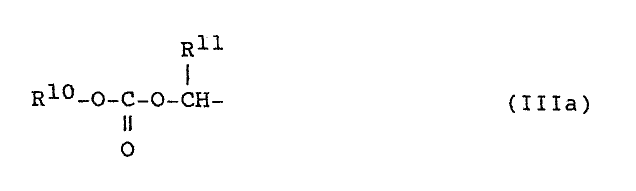 or 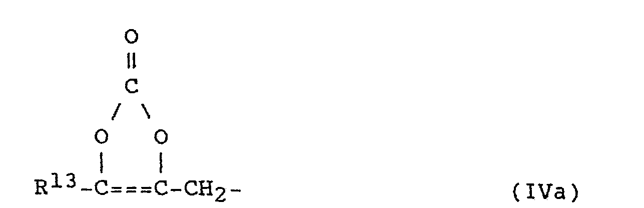 in which:
R⁷ represents a straight or branched chain alkyl group having from 1 to 6 carbon atoms or a cycloalkyl group having from 5 to 7 carbon atoms;
R⁸ represents a hydrogen atom or a straight or branched chain alkyl group having from 1 to 6 carbon atoms;
R¹⁰ represents a straight or branched chain alkyl group having from 1 to 6 carbon atoms;
R¹¹ represents a hydrogen atom or a straight or branched chain alkyl group having from 1 to 6 carbon atoms;
and R¹³ represents a phenyl group, a straight or branched chain alkyl group having from 1 to 6 carbon atoms or a cycloalkyl group having from 5 to 7 carbon atoms.

27. Compounds according to Claim 1, in which:
R¹ and R² are the same or different and each represents a hydrogen atom or a hydroxy group; and one of R⁵ and R⁶ represents a group of formula (Ila), (Illa) or (IVa) : 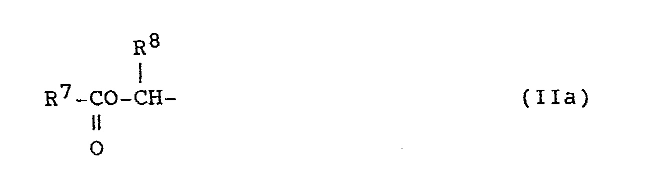 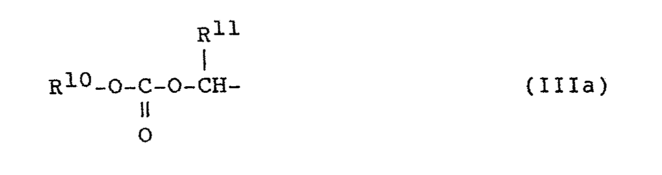 or 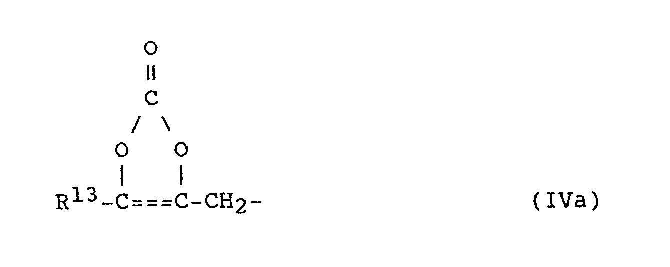 in which:
R⁷ represents a straight or branched chain alkyl group having from 1 to 6 carbon atoms or a cycloalkyl group having from 5 to 7 carbon atoms;
R⁸ represents a hydrogen atom or a straight or branched chain alkyl group having from 1 to 6 carbon atoms;
R¹⁰ represents a straight or branched chain alkyl group having from 1 to 6 carbon atoms;
R¹¹ represents a hydrogen atom or a straight or branched chain alkyl group having from 1 to 6 carbon atoms; and
R¹³ represents a straight or branched chain alkyl group having from 1 to 6 carbon atoms.

28. Compounds according to any one of the preceding Claims, in which R³ and R⁴ together form a single bond.

29. Compounds according to Claim 1, in which:
R¹ and R² are the same or different and each represents a hydrogen atom or a hydroxy group;
R³ and R⁴ together form a single bond; and
one of R⁵ and R⁶ represents a group of formula (Ila), (Ilia) or (IVa) : 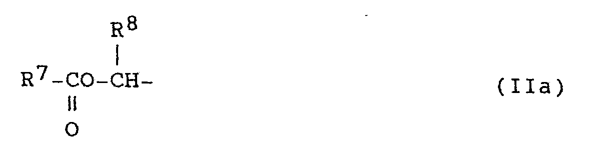 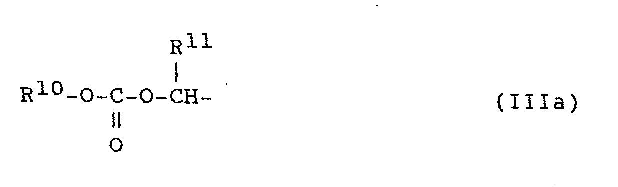 or 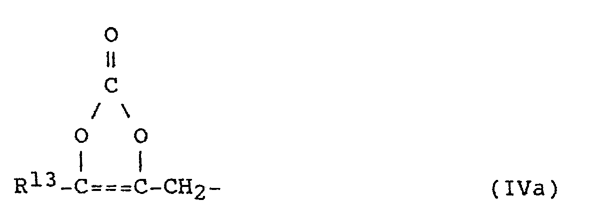 in which:
R⁷ represents a straight or branched chain alkyl group having from 1 to 6 carbon atoms or a cycloalkyl group having from 5 to 7 carbon atoms;
R⁸ represents a hydrogen atom or a straight or branched chain alkyl group having from 1 to 6 carbon atoms;
R¹⁰ represents a straight or branched chain alkyl group having from 1 to 6 carbon atoms;
R¹¹ represents a hydrogen atom or a straight or branched chain alkyl group having from 1 to 6 carbon atoms; and
R¹³ represents a phenyl group, a straight or branched chain alkyl group having from 1 to 6 carbon atoms or a cycloalkyl group having from 5 to 7 carbon atoms.

30. Compounds according to Claim 1, in which:
R¹ and R² are the same or different and each represents a hydrogen atom or a hydroxy group;
R³ and R⁴ together form a single bond; and one of R⁵ and R⁶ represents a group of formula (Ila), (ilia) or (IVa) : 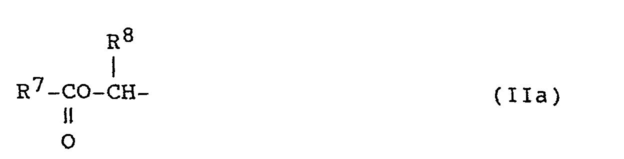 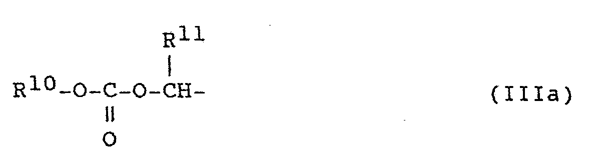 or 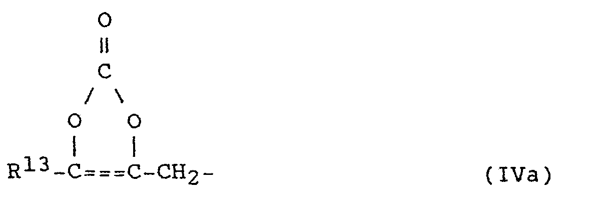 in which:
R⁷ represents a straight or branched chain alkyl group having from 1 to 6 carbon atoms or a cycloalkyl group having from 5 to 7 carbon atoms;
R⁸ represents a hydrogen atom or a straight or branched chain alkyl group having from 1 to 6 carbon atoms;
R¹⁰ represents a straight or branched chain alkyl group having from 1 to 6 carbon atoms;
R¹¹ represents a hydrogen atom or a straight or branched chain alkyl group having from 1 to 6 carbon atoms; and
R¹³ represents a straight or branched chain alkyl group having from 1 to 6 carbon atoms.

31. Compounds according to Claim 12, in which:
R¹ and R² are the same or different and each represents a hydrogen atom or a hydroxy group;
R³ and R⁴ together form a single bond; and one of R⁵ and R⁶ represents a group of formula (Va), as defined in Claim 12, in which:
R¹⁶ represents a hydrogen atom or a Ci - C₆ alkyl group,
R¹⁷ represents a hydrogen atom,
R³⁰ represents an aliphatic carboxylic acyloxy group containing from 1 to 18 carbon atoms, said group being unsubstituted or having at least one halogen substituent, or an aromatic acyloxy group which is unsubstituted or has at least one of substituents (a),
R³¹ represents an aliphatic carboxylic acyloxy group containing from 1 to 18 carbon atoms, an alkyl group containing from 1 to 6 carbon atoms, an alkoxy group containing from 1 to 6 carbon atoms or a halogen atom,
m is 0, 1 or 2, and
nis 0 or 1.

32. Compounds according to Claim 12, in which:
R¹ and R² are the same or different and each represents a hydrogen atom or a hydroxy group;
R3 and R⁴ together form a single bond; and
one of R⁵ and R⁶ represents a group of formula (Va), as defined in Claim 12, in which:
R¹⁶ represents a hydrogen atom or a C₁ - C₆ alkyl group,
R¹⁷ represents a hydrogen atom,
R³⁰ represents an aliphatic carboxylic acyloxy group containing from 1 to 6 carbon atoms, said group being unsubstituted or having at least one halogen substituent,
R³¹ represents an aliphatic carboxylic acyloxy group containing from 1 to 6 carbon atoms, an alkyl group containing from 1 to 6 carbon atoms, an alkoxy group containing from 1 to 6 carbon atoms or a halogen atom,
m is 0 or 1, and
n is 0 or 1.

33. Compounds according to Claim 12, in which:
R and R² are-the same or different and each represents a hydrogen atom or a hydroxy group;
R³ and R⁴ together form a single bond; and
one of R⁵ and R⁶ represents a group of formula (Va), as defined in Claim 12, in which:
R¹⁶ and R¹⁷ each represents a hydrogen atom,
R³⁰ represents an aliphatic carboxylic acyloxy group containing from 1 to 6 carbon atoms, said group being unsubstituted or having at least one halogen substituent,
m is 0, and
n is 0 or 1.

34. Compounds according to Claim 12, in which:
R¹ and R² are the same or different and each represents a hydrogen atom or a hydroxy group;
R³ and R⁴ together form a single bond; and
one of R⁵ and R⁶ represents a group of formula (Va), as defined in Claim 12, in which:
R¹⁶ and R¹⁷ each represents a hydrogen atom,
R³⁰ and R³¹ each represents an aliphatic carboxylic acyloxy group containing from 1 to 6 carbon atoms, said group being unsubstituted or having at least one halogen substituent,
m is 1, and
n is 0 or 1.

35. Compounds according to Claim 12, in which:
R¹ and R² are the same or different and each represents a hydrogen atom or a hydroxy group;
R³ and R⁴ together form a single bond; and
one of R⁵ and R⁶ represents a group of formula (Va), as defined in Claim 12, in which:
R¹⁶ and R¹⁷ each represents a hydrogen atom, R³⁰ represents an aliphatic carboxylic acyloxy group containing from 1 to 6 carbon atoms, said group being unsubstituted or having at least one halogen substituent,
R³¹ represents an alkyl group containing from 1 to 6 carbon atoms or an alkoxy group containing from to 6 carbon atoms,
m is 1 or 2, and
n is 0 or 1.

36. Compounds according to any one of the preceding Claims, in which R⁵ represents a hydrogen atom.

37. Compounds according to any one of Claims 1 to 35, in which R⁶ represents a hydrogen atom.

38. 9'-(1-Butyryloxyethyl) griseolate and pharmaceutically acceptable salts thereof.

39. 9'-(1-Isobutyryloxypropyl) griseolate and pharmaceutically acceptable salts thereof.

40.9'-(1-Isovaleryloxyethyl) griseolate and pharmaceutically acceptable salts thereof.

41. 9'-Pivaloyloxymethyl griseolate and pharmaceutically acceptable salts thereof.

42. 9'-(1-Pivaloyloxyethyl) griseolate and pharmaceutically acceptable salts thereof. 43.9'-Pivaloyloxymethyl 7-desoxygriseolate and pharmaceutically acceptable salts thereof.

44. 9'-Pivaloyloxymethyl 7'-desoxy-4' ,5' -dihydrogriseolate and pharmaceutically acceptable salts thereof.

45. 9'-(1-Pivaloyloxyethyl) 7'-desoxy-4',5'-dihydrogriseolate and pharmaceutically acceptable salts thereof.

46.9'-(1-Isopropoxycarbonyloxyethyl) griseolate and pharmaceutically acceptable salts thereof.

47. 9'-(5-Methyl-2-oxo-1,3-dioxolen-4-yl)methyl griseolate and pharmaceutically acceptable salts thereof.

48. 9'-(4-Acetoxybenzyl) griseolate and pharmaceutically acceptable salts thereof. 49.9'(4-Acetoxy-3-methoxybenzyl) griseolate and pharmaceutically acceptable salts thereof.

50. 9'-(4-Acetylthiobenzyl) griseolate and pharmaceutically acceptable salts thereof.

51. 8'-(4-Acetoxybenzyl) griseolate and pharmaceutically acceptable salts thereof.

52. 8'-(4-Acetylthiobenzyl) griseolate and pharmaceutically acceptable salts thereof.

53. 8'-(4-Acetoxy-3-methoxybenzyl) griseolate and pharmaceutically acceptable salts thereof.

54. 9'-(4-Acetoxybenzyl) 7'-desoxygriseolate and pharmaceutically acceptable salts thereof. 55.9'-(4-Acetoxy-3-methoxybenzyl) 7'-desoxygriseolate and pharmaceutically acceptable salts thereof. 56.8'-(4-Acetoxybenzyl) 7'-desoxygriseolate and pharmaceutically acceptable salts thereof.

57. 8'-(4-Acetoxy-3-methoxybenzyl) 7'-desoxygriseolate and pharmaceutically acceptable salts thereof.

58. 9'-(4-Acetoxybenzyl) 7'-desoxy-4',5'-dihydrogriseolate and pharmaceutically acceptable salts thereof.

59. 9'-(4-Acetoxy-3-methoxybenzyl) T-desoxy-4',5'-dihydrogriseolate and pharmaceutically acceptable salts thereof.

60. 8'-(4-Acetoxybenzyl) 7'-desoxy-4',5'-dihydrogriseolate and pharmaceutically acceptable salts thereof.

61. 8'-(4-Acetoxy-3-methoxybenzyl) 7-desoxy-4',5'-dihydrogriseolate and pharmaceutically acceptable salts thereof.

62. A pharmaceutical composition for application to the eyes, and comprising at least one ophthalmically active compound in admixture with an ophthalmically acceptable carrier or diluent, in which said active compound is at least one compound according to any one of the preceding Claims.

63. A pharmaceutical composition according to Claim 62, formulated for administration by instillation.

64. A process for preparing a compound according to any one of Claims 1 to 61, which process comprises the esterification of a compound of formula (IX): 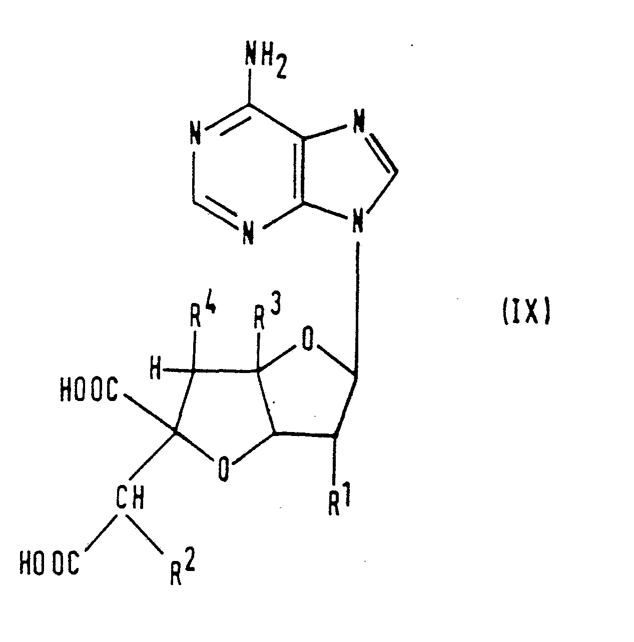 (in which R¹, R², R³ and R⁴ are as defined in Claim 1) or of a corresponding compound in which one of the carboxy groups is protected or of a corresponding compound in which one of the carboxy groups is replaced by an active derivative thereof.

65. A process according to Claim 64, in which the esterifying agent is employed in an approximately equivalent amount with respect to the compound of formula (IX), to prepare predominantly a monoester or mixture of monoesters.

66. The use for the manufacture of a medicament for the treatment of optical disorders in an animal of at least one ophthalmically active compound according to any one of Claims 1 to 61.

67. The use according to Claim 65, in which the medicament is formulated for administration by instillation.

## Claims (Claims for the following Contracting State(s) : ES, GR)

1. A process for preparing a compound of formula (1): 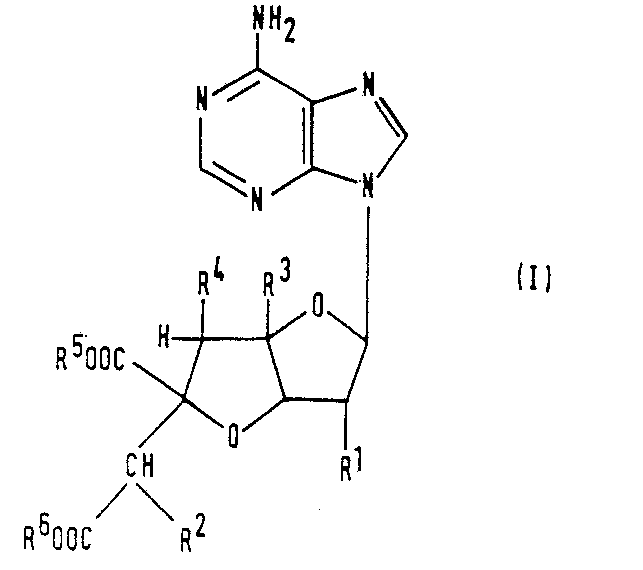 [in which:
R¹ and R² are the same or different and each represents a hydrogen atom, a hydroxy group or a protected hydroxy group;
R³ and R⁴ each represents a hydrogen atom, or together represent an extra carbon-carbon bond between the two carbon atoms to which they are attached; and
one of R⁵ and R⁶ represents a hydrogen atom and the other represents a carboxy-protecting group removable in the biochemical environment of the human eye];
or a pharmaceutically acceptable salt thereof,
by the esterification of a compound of formula (IX) : 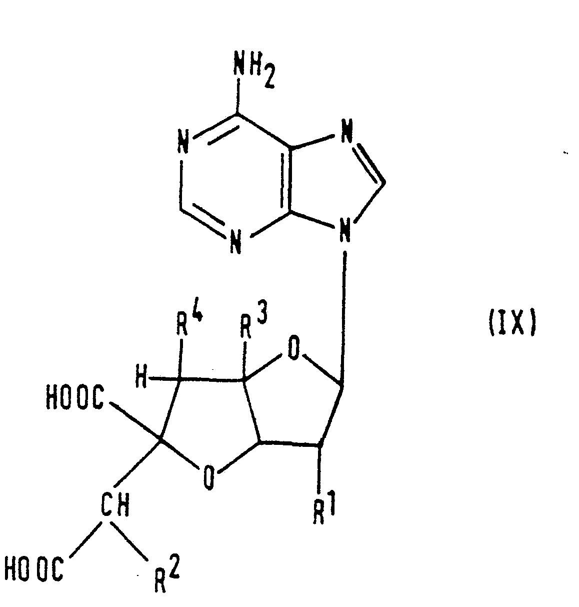 (in which R¹, R², R³ and R⁴ are as defined above) or of a corresponding compound in which one of the carboxy groups is protected or of a corresponding compound in which one of the carboxy groups is replaced by an active derivative thereof.

2. A process according to Claim 1, in which: one of R⁵ and R⁶ represents a hydrogen atom; and the other represents:
(i) a group of formula (II): 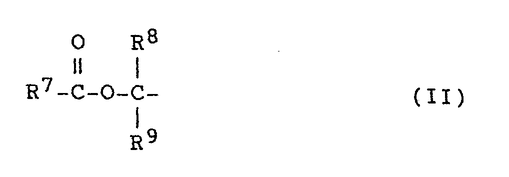 in which: R⁷ represents a straight or branched chain alkyl group having from 1 to 10 carbon atoms, an aryl group having from 6 to 14 ring carbon atoms or a cycloalkyl group having from 3 to 10 carbon atoms, the aryl group being unsubstituted or having at least one of substituents (a), defined below; R⁸ represents a hydrogen atom, a straight or branched chain alkyl group having from 1 to 10 carbon atoms or a cycloalkyl group having from 3 to 10 carbon atoms; and R⁹ represents a hydrogen atom or a straight or branched chain alkyl group having from 1 to 10 carbon atoms;
(ii) a group of formula (III): 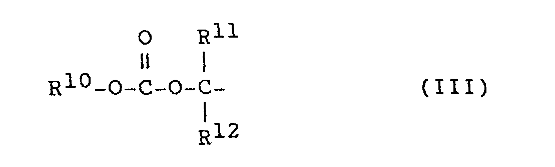 in which: R¹⁰ represents a straight or branched chain alkyl group having from 1 to 10 carbon atoms, an aryl group having from 6 to 14 ring carbon atoms or a cycloalkyl group having from 3 to 10 carbon atoms, the aryl group being unsubstituted or having at least one of substituents (a), defined below; R¹¹ represents a hydrogen atom, a straight or branched chain alkyl group having from 1 to 10 carbon atoms or a cycloalkyl group having from 3 to 10 carbon atoms; and R¹² represents a hydrogen atom or a straight or branched chain alkyl group having from 1 to 10 carbon atoms;
(iii) a group of formula (IV): 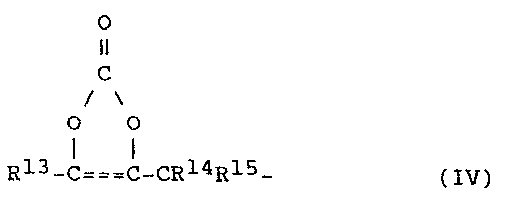 in which: R¹³ represents a straight or branched chain alkyl group having from 1 to 10 carbon atoms, a cycloalkyl group having from 3 to 10 carbon atoms or an aryl group having from 6 to 10 carbon atoms, which aryl group is unsubstituted or has at least one of substituents (a), defined below; R¹⁴ and R¹⁵ are the same or different and each represents a hydrogen atom or a straight or branched chain alkyl group having from 1 to 10 carbon atoms;
(iv) a group of formula (V) : 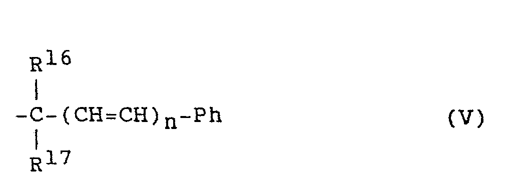 in which R¹⁶ and R¹⁷ are the same or different and each represents a hydrogen atom or a Ci - C6 alkyl group; Ph represents a phenyl group which is unsubstituted or has at least one substituent selected from: C₁ - C¹⁸ aliphatic acyloxy groups, Ci - C¹⁸ aliphatic acylthio groups, substituted C₁ - C₁₈ aliphatic acyloxy and Ci - C₁₈ aliphatic acylthio groups, aromatic acyloxy groups, aromatic acylthio groups, C₁- C₁₀ alkyl groups, Ci - C6 alkoxy groups, halogen atoms, hydroxy groups, mercapto groups, carboxy groups, amino groups and nitro groups, in which the substituent(s) on said substituted aliphatic acyloxy and acylthio groups are selected from halogen atoms and C₃-C₁₀ cycloalkyl groups, said aromatic acyloxy and acylthio groups being unsubstituted or having at least one of substituents (a), defined below; and n represents 0, 1 or 2;
(v) a group of formula (Vi): 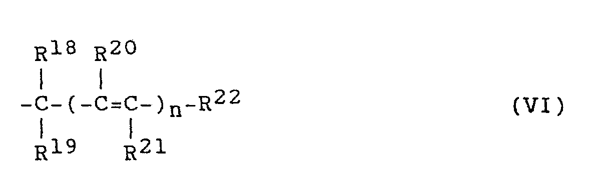 in which R¹⁸, R¹⁹, R²⁰ and R²¹, are the same or different and each represents a hydrogen atom or a Ci - C₆ alkyl group; R²² represents a carboxylic acyloxy group; and n is as defined above;
(vi) a group of formula (VII) : 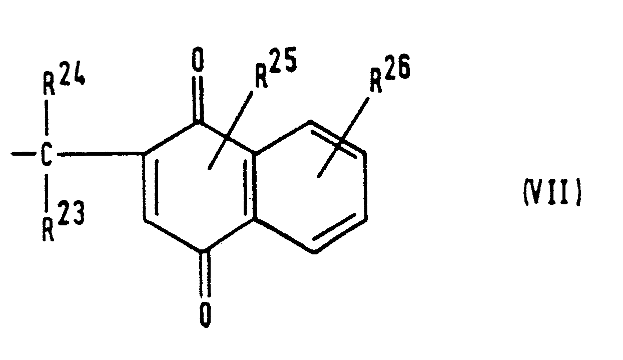 in which R²³ and R²⁴ are the same or different and each represents a hydrogen atom or a Ci - C₆ alkyl group; and R²⁵ and R²⁶ are the same or different and each represents a Ci - C₆ alkyl group, a Ci - C₆ alkoxy group or a halogen atom; or
(vii) a group of formula (VIII) : 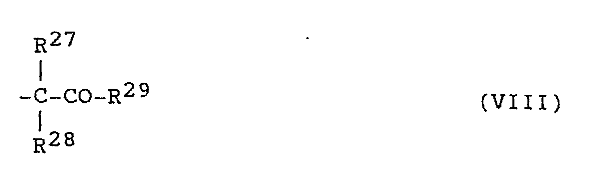 in which: R²⁷ and R²⁸ are the same or different and each represents a hydrogen atom or a C₁ - C₆ alkyl group; and R²⁹ represents a hydrogen atom, a C₁ - C₆ alkyl group or an aryl group having from 6 to 14 ring atoms and which is unsubstituted or has at least one of substituents (a), defined below; substituents (a): Ci - C₆ alkyl groups, C₁ - C₆ alkoxy groups, the nitro group, halogen atoms, the cyano group, aryl groups, provided that, where any such aryl group is a substituent on an aryl group, it is not itself further substituted by another aryl group, Ci - C₃ alkylenedioxy groups, the hydroxy group, the amino group, mono- and di- alkylamino groups, in which the or each alkyl group is C₁ - C₆, the carbamoyl group, mono- and di- alkylcarbamoyl groups, in which the or each alkyl group is C₁ - C₆, C₁ - C₁₈ aliphatic acyl groups, halogenated Ci - C₆ alkyl groups, the carboxy group, alkoxycarbonyl groups, in which the alkoxy group is Ci - C₆, halogenated alkoxycarbonyl groups or aralkyloxycarbonyl groups.

3. A process according to Claim 2, in which one of R⁵ and R⁶ represents a group of formula (Ila) : 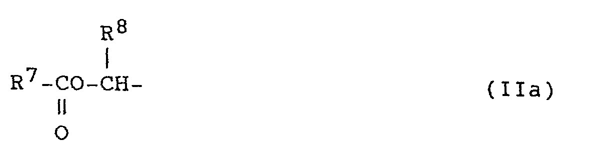 in which:
R⁷ represents a straight or branched chain alkyl group having from 1 to 10 carbon atoms, an aryl group having from 6 to 14 ring carbon atoms or a cycloalkyl group having from 3 to 10 carbon atoms, the aryl group being unsubstituted or having at least one of substituents (a) and;
R⁸ represents a hydrogen atom, a straight or branched chain alkyl group having from 1 to 10 carbon atoms or a cycloalkyl group having from 3 to 10 carbon atoms;
substituents (a) being as defined in Claim 2.

4. A process according to Claim 3, in which one of R⁵ and R⁶ represents a group of formula (Ila), as defined in Claim 3, where R⁷ represents a straight or branched chain alkyl group having from 1 to 6 carbon atoms or a cycloalkyl group having from 5 to 7 carbon atoms.

5. A process according to Claim 3, in which one of R⁵ and R⁶ represents a group of formula (Ila), as defined in Claim 3, where R⁸ represents a hydrogen atom or a straight or branched chain alkyl group having from 1 to 6 carbon atoms.

6. A process according to Claim 2, in which one of R⁵ and R⁶ represents a group of formula (IIIa): 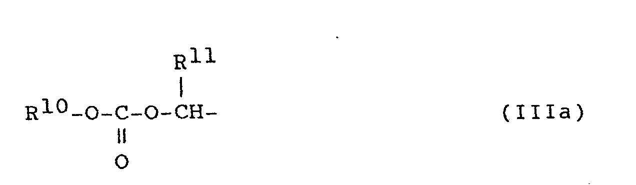 in which:
R¹⁰ represents a straight or branched chain alkyl group having from 1 to 10 carbon atoms, an aryl group having from 6 to 14 ring carbon atoms or a cycloalkyl group having from 3 to 10 carbon atoms, the aryl group being unsubstituted or having at least one of substituents (a) and;
R¹¹ represents a hydrogen atom, a straight or branched chain alkyl group having from 1 to 10 carbon atoms or a cycloalkyl group having from 3 to 10 carbon atoms;
substituents (a) being as defined in Claim 2.

7. A process according to Claim 6, in which one of R⁵ and R⁶ represents a group of formula (ilia), as defined in Claim 6, where R¹⁰ represents a straight or branched chain alkyl group having from 1 to 6 carbon atoms.

8. A process according to Claim 6, in which one of R⁵ and R⁶ represents a group of formula (Illa), as defined in Claim 6, where R¹¹ represents a hydrogen atom or a straight or branched chain alkyl group having from 1 to 6 carbon atoms.

9. A process according to Claim 2, in which one of R⁵ and R⁶ represents a group of formula (lVa): 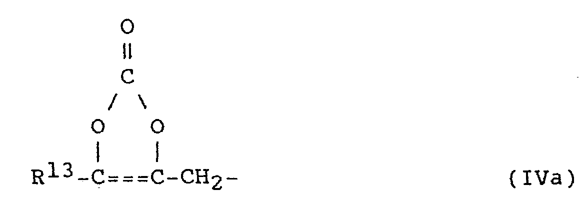 in which:
R¹³ represents a straight or branched chain alkyl group having from 1 to 10 carbon atoms, a cycloalkyl group having from 3 to 10 carbon atoms or an aryl group having from 6 to 10 carbon atoms, which aryl group is unsubstituted or has at least one of substituents (a);
substituents (a) being as defined in Claim 2.

10. A process according to Claim 9, in which one of R⁵ and R⁶ represents a group of formula (lVa), as defined in Claim 9, where R ¹³ represents a phenyl group, a straight or branched chain alkyl group having from 1 to 6 carbon atoms, or a cycloalkyl group having from 5 to 7 carbon atoms.

11. A process according to Claim 9, in which one of R5 and R⁶ represents a group of formula (lVa), as defined in Claim 9, where R¹³ represents a straight or branched chain alkyl group having from 1 to 6 carbon atoms.

12. A process according to Claim 2, in which one of R⁵ and R⁶ represents a group of formula (Va): 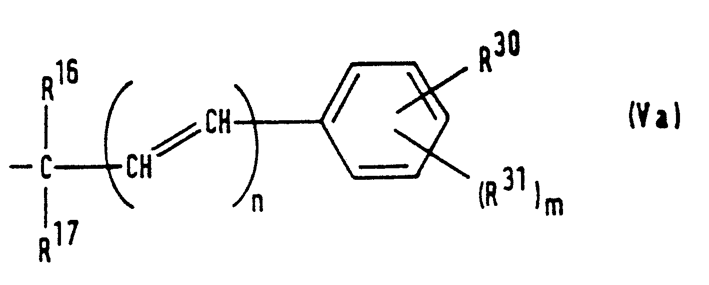 in which:
R¹⁶ and R¹⁷ are the same or different and each represents a hydrogen atom or a Ci - C⁶ alkyl group; n represents 0, 1 or 2;
m represents the cypher 0 or an integer from 1 to 3;
R³⁰ represents a C₁ - C₁₈ aliphatic acyloxy group, a Ci - C₁₈ aliphatic acylthio group, a substituted C₁ - C₁₈ aliphatic acyloxy or C₁ - C₁₈ aliphatic acylthio group, an aromatic acyloxy group or an aromatic acylthio group, said aromatic acyloxy and acylthio groups being unsubstituted or having at least one of substituents (a);
R³¹ represents a C₁-C₁₈ aliphatic acyloxy group, a C₁-C₁₈ aliphatic acylthio group, a substituted Ci C18 aliphatic acyloxy or Ci -C₁₈ aliphatic acylthio group, an aromatic acyloxy group, an aromatic acylthio group, a Ci - C₆ alkyl group, a Ci - C₆ alkoxy group, a halogen atom, a hydroxy group, a mercapto group, a carboxy group, an amino group or a nitro group, said aromatic acyloxy and acylthio groups being unsubstituted or having at least one of substituents (a);
the substituent(s) on said substituted aliphatic acyloxy and acylthio groups represented by R³⁰ and R³¹ being selected from halogen atoms and C₃ - C₁₀ cycloalkyl groups;
substituents (a) being as defined in Claim 2.

13. A process according to Claim 12, in which one of R⁵ and R⁶ represents a group of formula (Va), as defined in Claim 12, where R¹⁶ represents a hydrogen atom or a Ci - C₆ alkyl group.

14. A process according to Claim 12, in which one of R⁵ and R⁶ represents a group of formula (Va), as defined in Claim 12, where R¹⁷ represents a hydrogen atom or a methyl group.

15. A process according to Claim 12, in which one of R⁵ and R⁶ represents a group of formula (Va), as defined in Claim 12, where R³⁰ represents an aliphatic carboxylic acyloxy group containing from 1 to 18 carbon atoms, said group being unsubstituted or having at least one halogen substituent, or an aromatic acyloxy group which is unsubstituted or has at least one of substituents (a).

16. A process according to Claim 12, in which one of R⁵ and R⁶ represents a group of formula (Va), as defined in Claim 12, where R³⁰ represents an aliphatic carboxylic acyloxy group containing from 1 to 6 carbon atoms, said group being unsubstituted or having at least one halogen substituent.

17. A process according to Claim 12, in which one of R⁵ and R⁶ represents a group of formula (Va), as defined in Claim 12, where R³¹ represents an aliphatic carboxylic acyloxy group containing from 1 to 18 carbon atoms, an alkyl group containing from 1 to 6 carbon atoms, an alkoxy group containing from 1 to 6 carbon atoms or a halogen atom.

18. A process according to Claim 12, in which one of R⁵ and R⁶ represents a group of formula (Va), as defined in Claim 12, in which m is 0,1 or 2.

19. A process according to Claim 12, in which one of R⁵ and R⁶ represents a group of formula (Va), as defined in Claim 12, in which n is 0 or 1.

20. A process according to Claim 12, in which:
one of R⁵ and R⁶ represents a hydrogen atom; and
the other of R⁵ and R⁶ represents a group of formula (Va), as defined in Claim 12, in which:
R¹⁶ represents a hydrogen atom or a Ci - C₆ alkyl group,
R¹⁷ represents a hydrogen atom,
R³⁰ represents an aliphatic carboxylic acyloxy group containing from 1 to 18 carbon atoms, said group being unsubstituted or having at least one halogen substituent, or an aromatic acyloxy group which is unsubstituted or has at least one of substituents (a),
R³¹ represents an aliphatic carboxylic acyloxy group containing from 1 to 18 carbon atoms, an alkyl group containing from 1 to 6 carbon atoms, an alkoxy group containing from 1 to 6 carbon atoms or a halogen atom,
m is 0, 1 or 2, and
n̅is 0 or 1.

21. A process according to Claim 12, in which:
one of R⁵ and R⁶ represents a hydrogen atom; and
the other of R⁵ and R⁶ represents a group of formula (Va), as defined in Claim 12, in which:
R¹⁶ represents a hydrogen atom or a Ci - C₆ alkyl group,
R¹⁷ represents a hydrogen atom or a methyl group,
R³⁰ represents an aliphatic carboxylic acyloxy group containing from 1 to 6 carbon atoms, said group being unsubstituted or having at least one halogen substituent,
R³¹ represents an aliphatic carboxylic acyloxy group containing from 1 to 6 carbon atoms, an alkyl group containing from 1 to 6 carbon atoms, an alkoxy group containing from 1 to 6 carbon atoms or a halogen atom,
m is 0, 1 or 2, and
nis0or1.

22. A process according to Claim 12, in which:
one of R⁵ and R⁶ represents a hydrogen atom; and
the other of R⁵ and R⁶ represents a group of formula (Va), as defined in Claim 12, in which:
R¹⁶ and R¹⁷ each represents a hydrogen atom,
R³⁰ represents an aliphatic carboxylic acyloxy group containing from 1 to 6 carbon atoms, said group being unsubstituted or having at least one halogen substituent,
m is 0, and
n̅ is 0 or1.

23. A process according to Claim 12, in which:
one of R⁵ and R⁶ represents a hydrogen atom; and
the other of R⁵ and R⁶ represents a group of formula (Va), as defined in Claim 12, in which:
R¹⁶ and R¹⁷ each represents a hydrogen atom,
R³⁰ and R³¹ each represents an aliphatic carboxylic acyloxy group containing from 1 to 6 carbon atoms, said group being unsubstituted or having at least one halogen substituent,
m is 1, and
n is 0 or 1.

24. A process according to Claim 12, in which:
one of R⁵ and R⁶ represents a hydrogen atom; and
the other of R5 and R⁶ represents a group of formula (Va), as defined in Claim 12, in which:
R¹⁶ and R¹⁷ each represents a hydrogen atom,
R³⁰ represents an aliphatic carboxylic acyloxy group containing from 1 to 6 carbon atoms, said group being unsubstituted or having at least one halogen substituent,
R³¹ represents an alkyl group containing from 1 to 6 carbon atoms or an alkoxy group containing from 1 to 6 carbon atoms,
m is 1 or 2, and
n̅ is 0 or 1.

25. A process according to any one of the preceding Claims, in which R¹ and R² are the same or different and each represents a hydrogen atom or a hydroxy group.

26. A process according to Claim 1, in which:
R¹ and R² are the same or different and each represents a hydrogen atom or a hydroxy group; and one of R⁵ and R6 represents a group of formula (Ila), (Illa) or (lVa) : 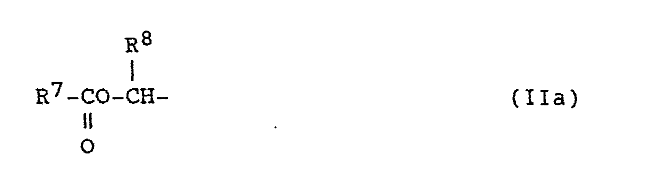 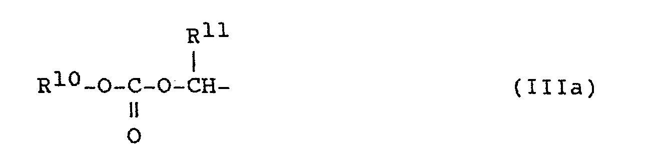 or 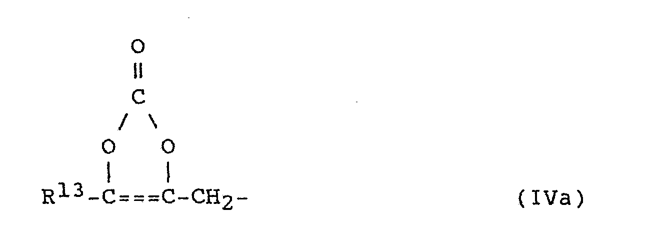 in which:
R⁷ represents a straight or branched chain alkyl group having from 1 to 6 carbon atoms or a cycloalkyl group having from 5 to 7 carbon atoms;
R⁸ represents a hydrogen atom or a straight or branched chain alkyl group having from 1 to 6 carbon atoms;
R¹⁰ represents a straight or branched chain alkyl group having from 1 to 6 carbon atoms;
R¹¹ represents a hydrogen atom or a straight or branched chain alkyl group having from 1 to 6 carbon atoms;
and R¹³ represents a phenyl group, a straight or branched chain alkyl group having from 1 to 6 carbon atoms or a cycloalkyl group having from 5 to 7 carbon atoms.

27. A process according to Claim 1, in which:
R¹ and R² are the same or different and each represents a hydrogen atom or a hydroxy group; and one of R⁵ and R⁶ represents a group or formula (Ila), (IIIa) or (IVa) : 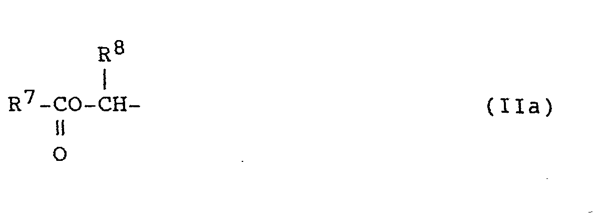 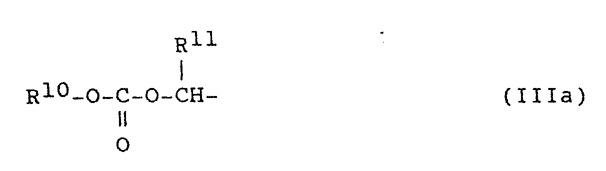 or 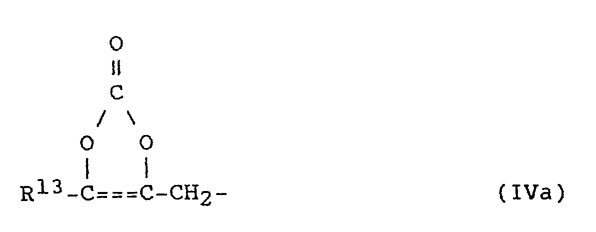 in which:
R⁷ represents a straight or branched chain alkyl group having from 1 to 6 carbon atoms or a cycloalkyl group having from 5 to 7 carbon atoms;
R⁸ represents a hydrogen atom or a straight or branched chain alkyl group having from 1 to 6 carbon atoms;
R¹ represents a straight or branched chain alkyl group having from 1 to 6 carbon atoms;
R¹¹ represents a hydrogen atom or a straight or branched chain alkyl group having from 1 to 6 carbon atoms; and
R¹³ represents a straight or branched chain alkyl group having from 1 to 6 carbon atoms.

28. A process according to any one of the preceding Claims, in which R³ and R⁴ together form a single bond.

29. A process according to Claim 1, in which:
R¹ and R² are the same or different and each represents a hydrogen atom or a hydroxy group;
R³ and R⁴ together form a single bond; and one of R⁵ and R⁶ represents a group of formula (Ila), (Illa) or (IVa) : 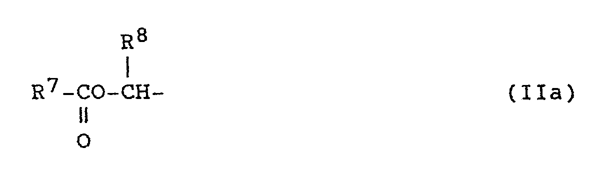 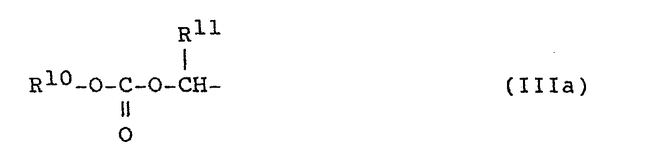 or 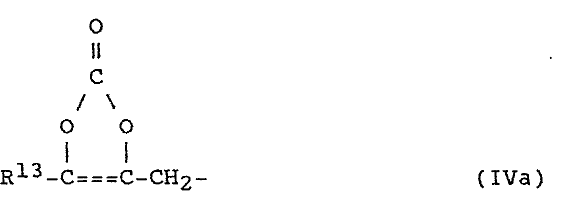 in which:
R⁷ represents a straight or branched chain alkyl group having from 1 to 6 carbon atoms or a cycloalkyl group having from 5 to 7 carbon atoms;
R⁸ represents a hydrogen atom or a straight or branched chain alkyl group having from 1 to 6 carbon atoms;
R¹⁰ represents a straight or branched chain alkyl group having from 1 to 6 carbon atoms;
R¹¹ represents a hydrogen atom or a straight or branched chain alkyl group having from 1 to 6 carbon atoms; and
R¹³ represents a phenyl group, a straight or branched chain alkyl group having from 1 to 6 carbon atoms or a cycloalkyl group having from 5 to 7 carbon atoms.

30. A process according to Claim 1, in which:
R and R² are the same or different and each represents a hydrogen atom or a hydroxy group; R³ and R⁴ together form a single bond; and
one of R⁵ and R⁶ represents a group of formula (Ila), (IIIa) or (IVa): 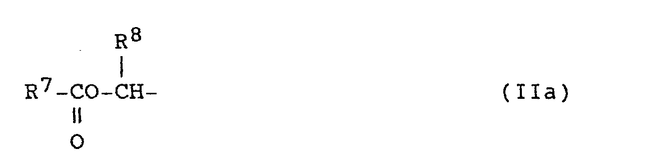 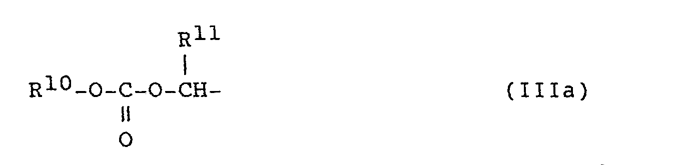 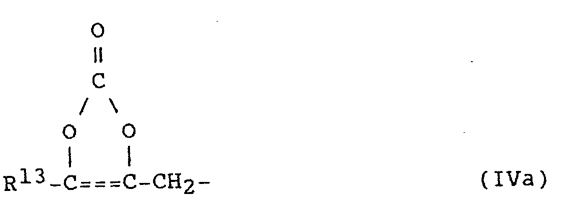 in which:
R⁷ represents a straight or branched chain alkyl group having from 1 to 6 carbon atoms or a cycloalkyl group having from 5 to 7 carbon atoms;
R⁸ represents a hydrogen atom or a straight or branched chain alkyl group having from 1 to 6 carbon atoms;
R¹⁰ represents a straight or branched chain alkyl group having from 1 to 6 carbon atoms;
R¹¹ represents a hydrogen atom or a straight or branched chain alkyl group having from 1 to 6 carbon atoms; and
R¹³ represents a straight or branched chain alkyl group having from 1 to 6 carbon atoms.

31. A process according to Claim 12, in which:
R¹ and R² are the same or different and each represents a hydrogen atom or a hydroxy group; R³ and R⁴ together form a single bond; and
one of R⁵ and R⁶ represents a group of formula (Va), as defined in Claim 12, in which:
R¹⁶ represents a hydrogen atom or a Ci - C₆ alkyl group,
R17 represents a hydrogen atom,
R³⁰ represents an aliphatic carboxylic acyloxy group containing from 1 to 18 carbon atoms, said group being unsubstituted or having at least one halogen substituent, or an aromatic acyloxy group which is unsubstituted or has at least one of substituents (a),
R³¹ represents an aliphatic carboxylic acyloxy group containing from 1 to 18 carbon atoms, an alkyl group containing from 1 to 6 carbon atoms, an alkoxy group containing from 1 to 6 carbon atoms or a halogen atom,
m is 0, 1 or 2, and
n is 0 or 1.

32. A process according to Claim 12, in which:
R¹ and R² are the same or different and each represents a hydrogen atom or a hydroxy group; R³ and R⁴ together form a single bond; and
one of R⁵ and R⁶ represents a group of formula (Va), as defined in Claim 12, in which:
R¹⁶ represents a hydrogen atom or a C₁ - C6 alkyl group,
R¹⁷ represents a hydrogen atom,
R³⁰ represents an aliphatic carboxylic acyloxy group containing from 1 to 6 carbon atoms, said group being unsubstituted or having at least one halogen substituent,
R³¹ represents an aliphatic carboxylic acyloxy group containing from 1 to 6 carbon atoms, an alkyl group containing from 1 to 6 carbon atoms, an alkoxy group containing from 1 to 6 carbon atoms or a halogen atom,
m is 0 or 1, and
n̅ is 0 or 1.

33. A process according to Claim 12, in which:
R¹ and R² are the same or different and each represents a hydrogen atom or a hydroxy group; R³ and R⁴ together form a single bond; and
one of R⁵ and R⁶ represents a group of formula (Va), as defined in Claim 12, in which:
R1⁶ and R¹⁷ each represents a hydrogen atom,
R³⁰ represents an aliphatic carboxylic acyloxy group containing from 1 to 6 carbon atoms, said group being unsubstituted or having at least one halogen substituent,
m is 0, and
n̅ is 0 or 1.

34. A process according to Claim 12, in which:
R¹ and R² are the same or different and each represents a hydrogen atom or a hydroxy group; R³ and R⁴ together form a single bond; and
one of R⁵ and R⁶ represents a group of formula (Va), as defined in Claim 12, in which:
R¹⁶ and R¹⁷ each represents a hydrogen atom,
R³⁰ and R³¹ each represents an aliphatic carboxylic acyloxy group containing from 1 to 6 carbon atoms, said group being unsubstituted or having at least one halogen substituent,
m is 1, and
n̅ is 0 or 1.

35. A process according to Claim 12, in which:
R¹ and R² are the same or different and each represents a hydrogen atom or a hydroxy group; R³ and R⁴ together form a single bond; and
one of R⁵ and R⁶ represents a group of formula (Va), as defined in Claim 12, in which: R¹⁶ and R¹⁷ each represents a hydrogen atom,
R³⁰ represents an aliphatic carboxylic acyloxy group containing from 1 to 6 carbon atoms, said group being unsubstituted or having at least one halogen substituent,
R³¹ represents an alkyl group containing from 1 to 6 carbon atoms, an alkoxy group containing from 1 to 6 carbon atoms,
m is 1 or 2, and
n̅ is 0 or 1.

36. A process according to any of the preceding Claims, in which R⁵ represents a hydrogen atom.

37. A process according to any one of Claims 1 to 35, in which R⁶ represents a hydrogen atom.

38. A process according to Claim 1, in which the starting materials and the reaction conditions are so selected as to prepare:
9'-(1-butyryloxyethyl) griseolate;
9'-(1-isobutyryloxypropyl) griseolate;
9'-(1-isovaleryloxyethyl) griseolate;
9'-pivaloyloxyethyl griseolate;
9'-(1-pivaloyloxyethyl) griseolate;
9'-pivaloyloxyethyl 7-desoxygriseolate;
9'-pivaloyloxyefhyl 7-desoxy-4',5'-dihydrogriseolate;
9'-(1-pivaloyloxyethyl) 7'-desoxy-4',5'-dihydrogriseolate;
9'-(1-isopropoxycarbonyloxyethyl) griseolate;
9'(5-methyl-2-oxo-1,3-dioxolen-4-yl)methyl griseolate;
9'-(4-acetoxybenzyl) griseolate;
9'-(4-acetoxy-3-methoxybenzyl) griseolate;
9'-(4-acetylthiobenzyl) griseolate;
8'-(4-acetoxybenzyl) griseolate;
8'-(4-acetylthiobenzyl) griseolate;
8'-(4-acetoxy-3-methoxybenzyl) griseolate;
9'-(4-acetoxybenzyl) 7'-desoxygriseoiate;
9'-(4-acetoxy-3-methoxybenzyl) 7'-desoxygriseoiate;
8'-(4-acetoxybenzyl) 7'-desoxygriseoiate;
8'-(4-acetoxy-3-methoxybenzyl) 7'-desoxygriseoiate;
9'-(4-acetoxybenzyl) 7'-desoxy-4',5'-dihydrogriseolate;
9'-(4-acetoxy-3-methoxybenzyl) T-desoxy-4',5'-dihydrogriseolate;
8'-(4-acetoxybenzyl) 7'-desoxy-4',5'-dihydrogriseolate;
8'-(4-acetoxy-3-methoxybenzyl) 7'-desoxy-4',5'-dihydrogriseolate;
or a pharmaceutically acceptable salts thereof.

39. A process according to any one of Claims 1 to 38, in which an approximately equivalent amount of esterifying agent is used relative to the compound of formula (IX), to prepare predominantly a monoester of formula (I) or a mixture of monoesters.

40. A process for preparing a pharmaceutical composition for application to the eyes, by mixing at least one ophthalmically active compound with an ophthalmically acceptable carrier or diluent, in which said active compound is at least one compound of formula (I) or a pharmaceutically acceptable salt thereof, as defined in any one of Claims 1 to 38.

41. The use for the manufacture of a medicament for the treatment of optical disorders in an animal of at least one ophthalmically active compound, in which said ophthalmically active compound is as defined in any one of Claims 1 to 38.

42. The use according to Claim 41, in which the medicament is formulated for administration by instillation.

43. The use according to Claim 41 or Claim 42, in which the ophthalmically active compound is:
9'-(1-butyryloxyethyl) griseolate;
9'-(1-isobutyryloxypropyl) griseolate;
9'-(1-isovaleryloxyethyl) griseolate;
9'-pivaloyloxymethyl griseolate;
9'-(1-pivaloyloxyethyl) griseolate;
9'-pivaloyloxymethyl 7'-desoxygriseolate;
9'-pivaloyloxymethyl 7'-desoxy-4',5'-dihydrogriseolate;
9'-(1-pivaloyloxyethyl) 7'-desoxy-4',5'-dihydrogriseolate;
9'-(1-isopropoxycarbonyloxyethyl) griseolate;
9'-(5-methyl-2-oxo-1,3-dioxolen-4-yl)methyl griseolate;
9'-(4-acetoxybenzyl) griseolate;
9'-(4-acetoxy-3-methoxybenzyl) griseolate;
9'-(4-acetylthiobenzyl) griseolate;
8'-(4-acetoxybenzyl) griseolate;
8'-(4-acetylthiobenzyl) griseolate;
8'-(4-acetoxy-3-methoxybenzyl) griseolate;
9'-(4-acetoxybenzyl) 7'-desoxygriseolate;
9'-(4-acetoxy-3-methoxybenzyl) 7'-desoxygriseolate;
8'-(4-acetoxybenzyl) 7'-desoxygriseolate;
8'-(4-acetoxy-3-methoxybenzyl) 7'-desoxygriseolate;
9'-(4-acetoxybenzyl) 7'-desoxy-4',5'-dihydrogriseolate;
9'-(4-acetoxy-3-methoxybenzyl) 7'-desoxy-4',5'-dihydrogriseolate;
8'-(4-acetoxybenzyl) 7'-desoxy-4',5'-dihydrogriseolate;
8'-(4-acetoxy-3-methoxybenzyl) 7'-desoxy-4',5'-dihydrogriseolate;
or a pharmaceutically acceptable salts thereof.
